(19) Europäisches Patentamt
European Patent Office
Office européen des brevets

(11) EP 0 581 939 B1

(12) FASCICULE DE BREVET EUROPEEN

(45) Date de publication et mention de la délivrance du brevet: **02.06.1999 Bulletin 1999/22**

(21) Numéro de dépôt: **93904135.6**

(22) Date de dépôt: **28.01.1993**

(51) Int Cl.[6]: **C07D 209/96**, A61K 31/40, C07D 209/90, C07D 491/10, C07D 471/10, C07D 403/12, C07D 409/12, C07D 209/34

(86) Numéro de dépôt international: **PCT/FR93/00093**

(87) Numéro de publication internationale: **WO 93/15051 (05.08.1993 Gazette 1993/19)**

# (54) DERIVES DU N-SULFONYL-2-OXOINDOLE AYANT UNE AFFINITE POUR LES RECEPTEURS DE LA VASOPRESSINE ET/OU DE L'OCYTOCINE

N-SULFONYL-2-OXOINDOL-DERIVATE MIT EINER AFFINITÄT FÜR DIE VASOPRESSIN UND/ODER OCYTOCIN REZEPTOREN

N-SULPHONYL-2-OXOINDOLE DERIVATIVES HAVING AFFINITY FOR VASOPRESSIN AND/OR OCYTOCIN RECEPTORS

(84) Etats contractants désignés: **AT BE CH DE DK ES FR GB GR IE IT LI LU NL PT SE**

(30) Priorité: **30.01.1992 FR 9201034**

(43) Date de publication de la demande: **09.02.1994 Bulletin 1994/06**

(73) Titulaire: **SANOFI**
**75013 Paris (FR)**

(72) Inventeurs:
- **FOULON, Loic**
  **F-31120 Pinsaguel (FR)**
- **GARCIA, Georges**
  **F-34980 S.-Genis-du-Fesc (FR)**
- **NISATO, Dino**
  **F-34680 Saint-Georges-d'Orques (FR)**
- **ROUX, Richard**
  **F-34570 Vailhaiques (FR)**
- **SERRADEIL-LEGAL, Claudine**
  **F-31750 Escalquens (FR)**
- **VALETTE, Gérard**
  **F-31120 Lacroix-Sasgarde (FR)**
- **WAGNON, Jean**
  **F-34000 Montpellier (FR)**

(74) Mandataire: **Gillard, Marie-Louise et al**
**Cabinet Beau de Loménie**
**158, rue de l'Université**
**75340 Paris Cédex 07 (FR)**

(56) Documents cités:
**EP-A- 0 429 685** **US-A- 3 838 167**

- **CHEMICAL ABSTRACTS, vol. 77, 1972, Columbus, Ohio, US; abstract no. 114172c, NATSUME, MITSUTAKA ET AL. 'Formation of 3-cyano-1,4-dihydroquinolines and their conversion to indole derivatives.' page 429 ;**

Remarques:
Le dossier contient des informations techniques présentées postérieurement au dépôt de la demande et ne figurant pas dans le présent fascicule.

EP 0 581 939 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention a pour objet des dérivés du N-sulfonyl-2-oxoindole, leur préparation et les compositions pharmaceutiques en contenant.

La demande de brevet international WO 91/01306 décrit des dérivés de 2-oxoindole, utiles pour le traitement de la démence sénile. Ces composés répondent à la formule :

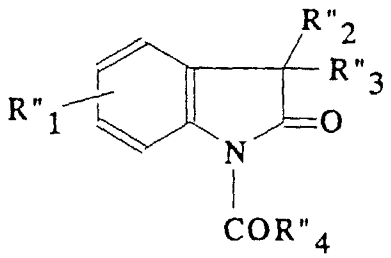

1

dans laquelle

- $R''_1$ représente un hydrogène, un halogène, un alkyle ou un alcoxy ;
- R"2 représente l'hydrogène ou un alkyle inférieur ;
- R"3 représente un alkyle, un cycloalkylméthyle, un benzodioxanylméthyle, ou un benzyle éventuellement substitué ;
- R"4 représente un 1-propylbutyle, un pyridyle ou un phényle éventuellement substitué.

Récemment, plusieurs demandes de brevet ont décrit des familles de composés à structure non-peptidique ayant une activité sur les récepteurs de la vasopressine et/ou de l'ocytocine. On peut citer les demandes européennes EP 382 185 et EP 444 945, la demande internationale WO 91/05 549, et plus particulièrement la demande de brevet japonais J03/127732. Cette dernière décrit des dérivés de l'acide indole-3-propanoique de formule :

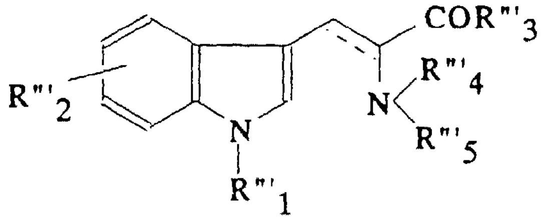

2

dans laquelle

- $R'''_1$ représente l'hydrogène, un alkyle, un alcényle, un phénylalkyle, un tétrahydrofuryle, un alcoxycarbonyle, un alcoxycarbonylalkyle, un carboxyalkyle ou un alcanoyle;
- $R'''_2$ représente l'hydrogène, un hydroxyle, un alcoxy, un alkyle, un phénylalkyle, un phénylalcoxy ou un halogène ;
- $R'''_3$ représente un hydrogène, un alcoxy, un groupe amino libre ou substitué ou un résidu d'aminoacide ;
- $R'''_4$ représente l'hydrogène, un alkyle ou un phénylalkyle;
- $R'''_5$ représente un benzoyle, un phényle, un alkyle, un phénylalcénylcarbonyle, un thiénylcarbonyle, un phénylsulfonyle, un pyridylcarbonyle ou un imidazolyl-carbonyle, les groupes phényle et alkyle du substituant $R'''_5$ pouvant être substitués.

Ces composés sont des antagonistes de la vasopressine.

Le brevet US 4 803 217 revendique des hapalindolinones obtenues par fermentation qui sont des antagonistes de la vasopressine. Ces composés sont représentés par la formule suivante :

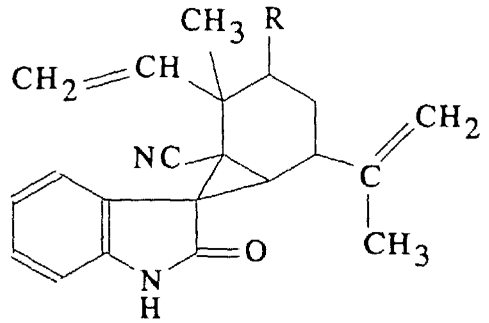

3

dans laquelle R représente H ou Cl.

Les dérivés du N-sulfonyl-2-oxoindole selon la présente invention ont une affinité pour les récepteurs de la vasopressine et de l'ocytocine.

La vasopressine est une hormone connue pour son effet antidiurétique et son effet dans la régulation de la pression artérielle. Elle stimule plusieurs types de récepteurs : $V_1$ ($V_{1a}$, $V_{1b}$), $V_2$ et exerce ainsi des effets cardiovasculaires, hépatiques, antidiurétiques, agrégants et des effets sur le système nerveux central et périphérique. Les antagonistes des récepteurs de la vasopressine peuvent intervenir sur la régulation de la circulation centrale et périphérique, notamment les circulations coronaire, rénale et gastrique, ainsi que sur la régulation hydrique et la libération de l'hormone adrénocorticotrophique (ACTH). Les agonistes non peptidiques de la vasopressine peuvent remplacer avantageusement la vasopressine ou ses analogues dans le traitement du diabète insipide ; ils peuvent également être utilisés dans le traitement de l'énurésie, et dans la régulation de l'hémostase : traitement de l'hemophilie, du syndrome de Von Willebrand, antidote des agrégants plaquettaires, Drug Investigation, 1990, 2 (Suppl. 5), 1-47. les récepteurs de la vasopressine comme ceux de l'ocytocine se trouvent aussi sur le muscle lisse de l'utérus. L'ocytocine a une structure peptidique proche de celle de la vasopressine. Ses récepteurs se trouvent également sur des cellules myoépithéliales de la glande mammaire et dans le système nerveux central (Presse Médicale, 1987, 16 (10), 481-485, J. Lab. Clin. Med., 1989, 114 (6), 617-632 et Pharmacol. Rev., 1991, 43 (1), 73-108). Cette hormone intervient dans la parturition, la lactation et le comportement sexuel.

Ainsi les composés selon l'invention sont utiles notamment dans le traitement des affections du système nerveux central et périphérique, du système cardiovasculaire, de la sphère rénale, de la sphère gastrique et dans les troubles du comportement sexuel, chez l'homme et chez l'animal.

La présente invention a pour objet des composés de formule :

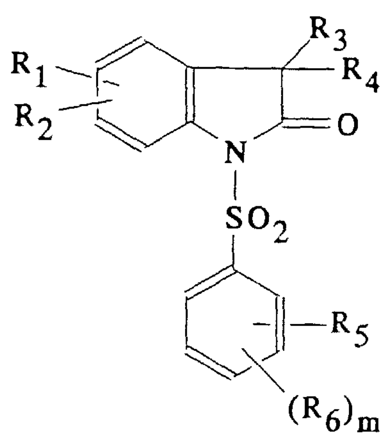

(I)

dans laquelle

- $R_1$ et $R_2$ représentent chacun indépendamment un hydrogène, un hydroxyle, un ω-halogénoalcoxy en $C_1$-$C_4$, un halogène, un alkyle en $C_1$-$C_4$, un trifluorométhyle, un alcoxy en $C_1$-$C_7$, un polyhalogénoalcoxy en $C_1$-$C_4$, un ω-hydroxyalcoxy en $C_2$-$C_4$, un ω-méthoxyalcoxy dans lequel l'alkyle est en $C_2$-$C_4$, un ω-aminoalcoxy en $C_2$-$C_4$ libre ou substitué par un ou deux alkyles en $C_1$-$C_4$ ; un cycloalkyloxy en $C_3$-$C_7$ ; un cycloalkylméthoxy dans lequel le

cycloalkyle est en $C_3$-$C_7$ ; un phénoxy ; un benzyloxy ; un alkylthio en $C_1$-$C_4$ ; un phénylthio ; un nitro ; un amino libre ou substitué par un ou deux alkyles en $C_1$-$C_4$ ; un cyano ; un acyle en $C_1$-$C_4$ ; un acyloxy en $C_1$-$C_4$ ; un alkylsulfonamido en $C_1$-$C_4$ ; un phénylsulfonamido ; un alkylamido en $C_1$-$C_4$ ; un alcoxycarbonylamino en $C_1$-$C_4$ ; un ureido non substitué ou substitué par un phényle ou par un ou deux alkyles en $C_1$-$C_4$ ;

- $R_3$ et $R_4$ représentent chacun indépendamment un alkyle en $C_1$-$C_6$, un cycloalkyle en $C_3$-$C_7$, un phényle, un benzyle, un cycloalkylméthyle dans lequel le cyclolakyle est en $C_3$-$C_7$ ;
  ou
- $R_3$ et $R_4$ ensemble constituent un groupe $-(CH_2)_pX(CH_2)_q-$ ;
  ou
- $R_3$ et $R_4$ ensemble avec le carbone auquel ils sont liés constituent un cycle hydrocarboné en $C_3$-$C_{10}$, saturé ou insaturé, éventuellement condensé, non substitué ou substitué par un ou plusieurs groupes alkyles en $C_1$-$C_7$ ou par un spirocycloalkyle en $C_3$-$C_5$ ;
  ou encore
- $R_1$ et $R_4$ représentent chacun l'une des valeurs indiquées ci-dessus et $R_2$ est situé en position 4 de l'indole et constitue avec $R_3$ un groupement $(CH_2)_3$ ;
- $R_5$ et $R_6$ représentent chacun indépendamment un hydrogène, un halogène, un alkyle en $C_1$-$C_7$, un trifluorométhyle, un cyano, un nitro, un amino libre ou substitué par un ou deux alkyles en $C_1$-$C_7$ ; un hydroxylamino ; un hydroxy ; un carboxy ; un groupe $OR_7$ ; un groupe $SR_7$ ; un acyle en $C_1$-$C_7$ ; un alcoxycarbonyle en $C_1$-$C_7$ ; un phénoxycarbonyle ; un benzyloxycarbonyle ; un carbamoyle substitué par des groupes $R'_6$ et $R''_6$ ; un thiocarbamoyle libre ou substitué par un ou deux alkyles en $C_1$-$C_7$ ; un sulfamoyle ; un alkylsulfamoyle ou dialkylsulfamoyle dans lequel l'alkyle est en $C_1$-$C_7$ ; un groupe $SO_2R'_7$ ; un alkylsulfonamido dans lequel l'alkyle est en $C_1$-$C_7$ ; un groupe $COR'_7$ ; un groupe $NR_8R_9$ ; un groupe CO-NH-CH($R_{10}$)-$COR_{12}$ ; le cas échéant, le groupe phényle constitutif du substituant $R_5$ et/ou $R_6$, peut être non substitué ou substitué une ou plusieurs fois par un alkyle en $C_1$-$C_7$, un trifluorométhyle, un méthoxy, un halogène, un sulfamoyle, un alkylsulfamoyle dans lequel l'alkyle est en $C_1$-$C_4$, un carboxy, un alcoxycarbonyle dans lequel l'alkyle est en $C_1$-$C_7$, un acyloxy en $C_1$-$C_7$, un imidazolyle ;
- $R'_6$ et $R''_6$ représentent chacun indépendamment l'hydrogène, un alkyle en $C_1$-$C_7$ non substitué ou substitué par $R'''_6$, un phényle, un pyridyle, un méthylpyridyle, un pipéridin-4-yle, un méthylpipéridin-4-yle ;
- $R'''_6$ représente un hydroxyle, un cyano, un carboxy libre ou estérifié par un alkyle en $C_1$-$C_7$ ou par un benzyle ; un phényle ; un pyridyle ; un méthylpyridyle; un amino libre ou substitué par un ou deux alkyles en $C_1$-$C_7$;
- $R_7$ représente un alkyle en $C_1$-$C_7$, un phényle, un benzyle, un cycloalkyle en $C_3$-$C_7$, un alcényle en $C_2$-$C_4$, un ω-halogénoalkyle en $C_1$-$C_7$, un polyhalogénoalkyle en $C_1$-$C_7$, un acyle en $C_1$-$C_7$, un ω-carboxyalkyle en $C_1$-$C_7$ libre ou estérifié par un alkyle en $C_1$-$C_4$ ou par un benzyle, un ω-aminoalkyle en $C_2$-$C_7$ dans lequel le groupe amino est libre, substitué par un ou deux alkyles en $C_1$-$C_4$ ou sous forme d'ion ammonium ;
- $R'_7$ représente un groupe pipérazin-1-yl non substitué ou substitué en 4 par un groupe $R''_7$, un groupe pipéridino non substitué ou substitué en 4 par un groupe $R'''_7$, un groupe azétidin-1-yl non substitué ou substitué en 3 par un groupe $R'''_7$, un groupe pyridyle non substitué ou substitué par un méthyle ;
- $R''_7$ représente un alkyle en $C_1$-$C_4$, un phényle, un benzyle, un acyle en $C_1$-$C_4$ ;
- $R'''_7$ représente $R''_7$ ou un amino libre ou portant un groupe protecteur;
- $R_8$ et $R_9$ représentent chacun indépendamment un hydrogène, un alkyle en $C_1$-$C_7$, un phényle, un benzyle, $R_9$ peut de plus représenter un acyle en $C_1$-$C_7$, un thioacyle en $C_1$-$C_7$, un cycloalkylcarbonyle dans lequel le cycloalkyle est en $C_3$-$C_7$, un cycloalkylthiocarbonyle dans lequel le cycloalkyle est en $C_3$-$C_7$, un ω-aminoacyle en $C_1$-$C_4$, un ω-hydroxyacyle en $C_1$-$C_4$, un ω-benzyloxyacyle en $C_1$-$C_4$, un phénoxycarbonyle, un thiénocarbonyle, un pyridylcarbonyle, un méthylpyridylcarbonyle, un alcoxycarbonyle en $C_1$-$C_4$, un benzoyle, un groupe CO-CH($R_{10}$)-$NR_{11}R'_{11}$ ; un groupe $CH(R_{10})CO_2R_{11}$, un groupe $(CH_2)_tCOR_{12}$, un groupe $CO(CH_2)_tCOR_{12}$, un carbamoyle non substitué ou substitué par un phényle ou par un ou deux alkyles en $C_1$-$C_4$ ;
- m est 1 ou, lorsque $R_6$ est un halogène, un alkyle en $C_1$-$C_7$ ou un alcoxy en $C_1$-$C_7$ , m peut également être 2, 3 ou 4 ou bien $(R_6)_m$ peut représenter m subsitutants ayant différentes valeurs choisies parmi halogène, alkyle en $C_1$-$C_7$ ou alcoxy en $C_1$-$C_7$ ;
- p et q représentent chacun un nombre entier, leur somme peut varier de 3 à 6;
- t représente un nombre entier qui peut varier de 1 à 5 ;
- X représente l'oxygène, le soufre ou un groupe $NR_{13}$ ;
- $R_{10}$ représente l'hydrogène, un alkyle en $C_1$-$C_4$ ou un benzyle ;
- $R_{11}$ et $R'_{11}$ représentent chacun indépendamment l'hydrogène ou un alkyle en $C_1$-$C_4$ ;
- $R_{12}$ représente un hydroxy, un alcoxy en $C_1$-$C_4$, un amino non substitué ou substitué par un ou deux alkyles en $C_1$-$C_4$ ;
- $R_{13}$ représente l'hydrogène, un alkyle en $C_1$-$C_4$, un phényle, un benzyle, un acyle en $C_1$-$C_4$, un alcoxycarbonyle en $C_1$-$C_4$, un carbamoyle non substitué ou substitué par un ou 2 alkyles en $C_1$-$C_4$ ;

sous réserve que lorsque $R_3$ représente un méthyle, $R_4$ représente un méthyle ou un éthyle, $R_5$ représente l'hy-

drogène, m est 1 et $R_6$ représente un méthyle en position 4, alors $R_1$ et $R_2$ ne représentent pas simultanément l'hydrogène ; ainsi que leurs sels éventuels.

Des composés 3,3-dialkyl-1-tosyloxindoles sont décrits dans M. Natsume et al. Chem. Pharm. Bull., 1972, 20, 1595.

Lorsqu'un composé selon l'invention présente un ou des carbones asymétriques, l'invention comprend tous les isomères optiques de ce composé.

Les sels des composés de formule (I) selon la présente invention comprennent ceux avec des acides minéraux ou organiques qui permettent une séparation ou une cristallisation convenable des composés de formule (I), tels que l'acide picrique, l'acide oxalique ou un acide optiquement actif, par exemple un acide mandélique ou un acide camphosulfonique, et ceux qui forment des sels physiologiquement acceptables tels que le chlorhydrate, le bromhydrate, le sulfate, l'hydrogénosulfate, le dihydrogénophosphate, le maléate, le fumarate, le 2-naphtalènesulfonate.

Les sels des composés de formule (I) comprennent également les sels avec des bases organiques ou minérales, par exemple les sels des métaux alcalins ou alcalino-terreux comme les sels de sodium, de potassium, de calcium, les sels de sodium et de potassium étant préférés, ou avec une amine, telle que le trométamol, ou bien les sels d'arginine, de lysine, ou de toute amine physiologiquement acceptable.

Selon la présente invention, par halogène on entend un atome choisi parmi le fluor, le chlore, le brome ou l'iode, de préférence le fluor ou le chlore. Par groupe protecteur de l'amine, on entend un groupe tel que le *tert*-butoxycarbonyle, le benzyloxycarbonyle ou le benzyle, par exemple.

Selon la présente invention par cycle hydrocarboné en $C_3$-$C_{10}$ éventuellement condensé, saturé ou insaturé, on entend différents cycles hydrocarbonés de structure monocyclique, bicyclique ou tricyclique, par exemple un cyclobutane, un cyclopentane, un cyclohexane, un cycloheptane, un cyclooctane, un indane, un hexahydroindane, un adamantane, un norbornane, un norbornène, un dihydrophénalène, un tricyclo [5.2.1.0.2,6] décane ou un tricyclo [5.2.1.0.2,6]dec-8-ène.

Les composés de formule (I) dans lesquels $R_1$ est en position 5 de l'indole et $R_2$ est l'hydrogène sont des composés préférés.

Les composés de formule (I) dans lesquels $R_1$ est un atome de chlore ou un groupe éthoxy en position 5 de l'indole et $R_2$ est l'hydrogène sont des composés préférés.

Les composés de formule (I) dans lesquels $R_3$ et $R_4$ ensemble avec le carbone auquel ils sont liés constituent un cycle hydrocarboné en $C_3$-$C_{10}$ sont des composés préférés, particulièrement préférés sont les composés dans lesquels $R_3$ et $R_4$ ensemble avec le carbone auquel ils sont liés constituent un cycloheptane, un adamantane, un tricyclo [5.2.1.0.2,6]dec-8-ène ou un cyclohexane non substitué ou substitué par un spirocycloalkyle en $C_3$-$C_5$ ou par un ou deux groupes alkyles en $C_1$-$C_7$.

Les composés de formule (I) dans lesquels $R_3$ et $R_4$ ensemble avec le carbone auquel ils sont liés constituent un cycle pipéridine-4 ou N-méthylpipéridine-4 sont également préférés.

Les composés de formule (I) dans lesquels $R_5$ et $R_6$ représentent chacun un méthoxy sont des composés préférés. De même les composés dans lesquels $R_5$ en position 2 représente un méthoxy et $R_6$ en position 4 représente un acylamino en $C_1$-$C_7$, un dialkylureido en $C_1$-$C_4$ ou un alcoxycarbonylalkylcarbamoyle dans lequel les groupes alkyles sont en $C_1$-$C_7$ sont des composés préférés.

Dans la description et dans les exemples, les abréviations suivantes sont utilisées.

DCM : dichlorométhane
Ether : éther éthylique
Ether iso : éther isopropylique
Boc : *tert*-butoxycarbonyle
Me, MeO : méthyle, méthoxy
Et : éthyl
Pr, iPr, nPr : propyle, isopropyle, n-propyle
Bu, iBu, tBu : butyle, isobutyle, *tert*-butyle
Ph : phényle
Bz : benzyle
Ac : acétyle
AcOEt : acétate d'éthyle
AcOH : acide acétique
MeOH : méthanol
EtOH : éthanol
DMF : diméthylformamide
THF : tétrahydrofuranne

DMSO : diméthylsulfoxyde
DIPEA : diisopropyléthylamine
TEA : triéthylamine
TFA : acide trifluoroacétique
TMEDA : tétraméthyléthylènediamine
Fc : point de fusion
Solution saline : eau saturée en chlorure de sodium
CCM : chromatographie en couche mince
HPLC : chromatographie liquide à haute pression
Eau chlorhydrique : acide chlorhydrique dilué, environ 1N
TA : température ambiante

La présente invention a également pour objet un procédé de préparation des composés selon l'invention caractérisé en ce que :

on fait agir sur un oxo-2 indole disubstitué en position 3, de formule :

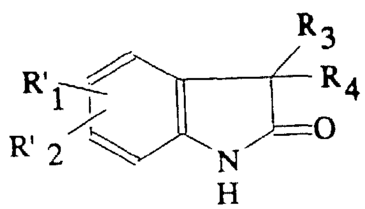

(II)

dans laquelle $R'_1$ et $R'_2$ représentent, respectivement, soit $R_1$ et $R_2$ tels que définis pour (I), soit des groupes précurseurs de $R_1$, $R_2$ , et $R_3$, $R_4$ sont tels que définis ci-dessus pour (I), un halogénure de benzènesulfonyle de formule :

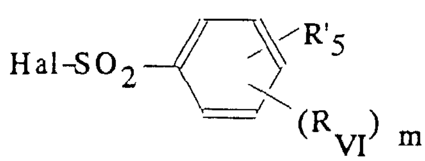

(III)

dans laquelle $R'_5$ et $R_{VI}$ représentent, respectivement, soit $R_5$ et $R_6$ tels que définis ci-dessus pour (I), soit des groupes précurseurs de $R_5$ et $R_6$ ; et,

- soit, lorsque $R'_1 = R_1$, $R'_2 = R_2$, $R'_5 = R_5$ et $R_{VI} = R_6$, on isole le composé de formule (I) ainsi obtenu ;
- soit, lorsque l'un quelconque des groupes $R'_1$, $R'_2$, $R'_5$ et $R_{VI}$ représente respectivement un groupe précurseur de $R_1$, $R_2$, $R_5$ et/ou $R_6$, on soumet le composé obtenu à un traitement ultérieur pour préparer le composé de formule (I) par transformation de l'un quelconque des groupes $R'_1$, $R'_2$, $R'_5$ et $R_{VI}$ en, respectivement, $R_1$, $R_2$, $R_5$ et $R_6$.

La réaction est effectuée dans un solvant anhydre tel que le DMF ou le THF, en présence d'un hydrure métallique tel que l'hydrure de sodium par exemple, ou en présence d'un alcoolate comme le *tert*-butylate de potassium.

Les 2-oxoindoles (II) peuvent être préparés selon différents modes opératoires. Certains de ces composés sont nouveaux et font partie de l'invention.

Des composés (II) dans lesquels $R'_1$ et/ou $R'_2$ représentent un halogène et $R_3$, $R_4$ ensemble avec le carbone auquel ils sont liés constituent un spirocyclobutane, un spirocyclohexane ou un spirocycloheptane sont connus, par exemple dans D.W.Robertson et al., J.Med.Chem., 1987, 30 (5),824-829. De plus, le 5-chloro-3-spirocyclopentaneindol-2-one est décrit dans le brevet US 3 947 451.

Pour la préparation des composés (II), lorsque $R_3$ et $R_4$ représentent ensemble ou séparément un groupement hydrocarboné, on peut utiliser la réaction de Brunner décrite par R.F. Moore et S.G.P. Plant dans J. Chem. Soc., 1951,

3475-3478 et qui conduit à la préparation de composés (II) dans lesquels $CR_3R_4$ représente un cyclopentane ou un cyclohexane.

Cette réaction est effectuée par cyclisation d'un dérivé de phénylhydrazide de formule :

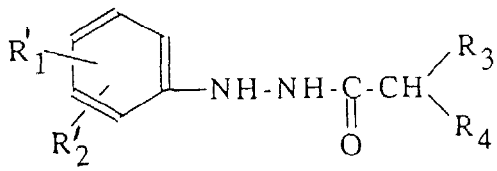

(IV)

dans laquelle $R'_1$ et $R'_2$ sont tels que définis ci-dessus pour (II) et $R_3$, $R_4$ ont les définitions données ci-dessus pour (I), par exemple par chauffage en présence d'oxyde de calcium et de quinoléine.

Le dérivé de phénylhydrazide (IV) est obtenu selon les mêmes auteurs par action d'un dérivé de l'hydrazine de formule :

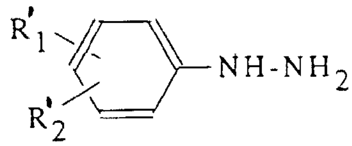

(V)

dans laquelle $R'_1$ et $R'_2$ ont les définitions données ci-dessus pour (II), sur un halogénure d'acide de formule :

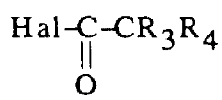

(VI)

dans laquelle $R_3$ et $R_4$ ont les définitions données ci-dessus pour (I).

Selon un mode de réalisation particulier, lorsque $R_3$ et $R_4$ ensemble avec le carbone auquel ils sont liés constituent un cycle hydrocarboné polycondensé, par exemple un norbornane ou un norbornène, on opère d'après la méthode décrite par J. Wolff et al., Tetrahedron, 1986, 42 (15), 4267-4272 : on prépare d'abord un sel de lithium du composé (IV) par action d'un réactif lithié tel que le n-butyllithium, dans un solvant inerte tel que le THF, à basse température, puis on effectue la cyclisation par chauffage dans un solvant tel que le naphtalène ou le prehnitène (1,2,3,4-tétraméthylbenzène).

Les composés (II) dans lesquels $R_1 = R_2 = H$ et $CR_3R_4$ représentent l'adamantane sont décrits dans I. Fleming et al., J.Chem.Soc., Perkin Trans I, 1991, 3, 617-626.. Ainsi, les composés (II) dans lesquels $R_3$ et $R_4$ ensemble avec l'atome de carbone avec lequel ils sont liés constituent un adamantane et $R_1$ et $R_2$ sont différents de l'hydrogène sont nouveaux et font partie de l'invention. Ils peuvent être préparés par la méthode décrite ci-dessus.

Les dérivés de l'hydrazine (V) sont connus ou préparés par des méthodes connues. Il en est de même pour les halogènures d'acide (VI).

On peut également préparer un 2-oxoindole disubstitué en position 3 (II), à partir d'un 2-oxoindole de formule :

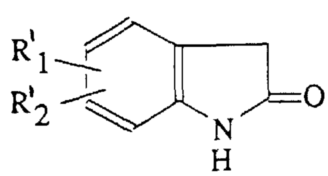

(VII)

dans laquelle $R'_1$ et $R'_2$ ont les valeurs définies ci-dessus pour (II) en utilisant différents procédés.

Par exemple, selon la méthode décrite par A.S. Kende et J.C. Hodges dans Synth. Commun., 1982, 12 (1),

1-10, on réalise l'addition d'un agent alkylant dans un solvant approprié. Ainsi pour préparer un composé (II) dans lequel $R_3 = R_4$, la réaction est effectuée dans le THF à -75°C en présence de TMEDA, par addition d'un alkyl lithium comme le butyllithium puis réaction avec un halogénure de formule $R_3Hal$ ; lorsque $R_3$ et $R_4$ sont différents, la réaction d'alkylation peut être effectuée en 2 étapes, par 2 halogénures d'alkyle différents de formule $R_3Hal$ et $R_4Hal$. Pour préparer un composé (II) dans lequel $R_3$ et $R_4$ ensemble forment un groupe de formule $-(CH_2)_n-$ avec n variant de 2 à 7, on fait agir un composé de formule $Z(CH_2)_nZ$ dans laquelle Z représente un groupe nucleofuge tel qu'un halogène, de préférence le brome ou l'iode, un groupe tosyloxy ou un groupe mésyloxy.

Les composés de formule (II) dans lesquels $R_3$ et $R_4$ représentent chacun indépendamment un alkyle ou un phényle sont connus. Par exemple, le brevet DE 3 300 522 décrit des 5-alcoxy-3,3-diméthylindol-2-ones.

On prépare de la même façon les composés (II) dans lesquels $R_3$ et $R_4$ ensemble avec le carbone auquel ils sont liés constituent un cycle hydrocarboné en $C_4$-$C_8$ substitué par un ou plusieurs groupes alkyles en $C_1$-$C_7$ ou par un spirocycloalkyle en $C_3$-$C_5$. Ces composés sont nouveaux et font partie de l'invention.

Lorsque $R_3$ et $R_4$ ensemble constituent un groupe $-(CH_2)_p X(CH_2)_q-$, dans lequel p, q et X ont les valeurs données ci-dessus pour (I), on peut préparer un 2-oxoindole disubstitué en position 3 de formule (II), à partir d'un 2-oxoindole non substitué en 3 (VII) par action d'un composé de formule :

$$Z-(CH_2)_p-X-(CH_2)_q-Z \qquad (VIII)$$

dans laquelle Z est tel que défini ci-dessus et X, p et q ont les valeurs définies ci-dessus pour (I). La réaction est effectuée en présence d'un alcoolate, par exemple le *tert*-butylate de potassium, dans un solvant anhydre, tel que le THF par exemple.

Lorsque X représente un atome d'azote substitué par un acyle en $C_1$-$C_4$, un alcoxycarbonyle en $C_1$-$C_4$ ou par un alkylcarbamoyle en $C_1$-$C_4$, la substitution sur X peut être réalisée soit sur le dérivé 2-oxoindole (II), soit sur le composé final (I) à partir d'un composé dans lequel l'atome d'azote (X = NH) n'est pas substitué.

Les composés (I) dans lesquels X = NH sont des compsés préférés selon l'invention.

Ainsi lorsque X représente un atome d'azote substitué par un alcoxycarbonyle en $C_1$-$C_4$, on prépare d'abord un composé (II) ou (I) dans lequel X est NH, puis on fait agir le chloroformiate approprié pour obtenir le composé (II) ou (I) souhaité. De la même façon, on fait agir sur un composé (II) ou (I) dans lequel X = NH un isocyanate d'alkyle en $C_1$-$C_4$, pour obtenir un dérivé 2-oxoindole (II) ou un composé (I) dans lequel X représente un atome d'azote substitué par un alkylcarbamoyle. On fait agir sur un composé (II) ou un composé (I) dans lequel X = NH un chlorure d'acide ou un anhydride pour préparer un composé de formule (I) dans lequel X représente un atome d'azote substitué par un acyle.

Les composés (II) dans lesquels $R_3$ et $R_4$ ensemble avec le carbone auquel ils sont liés constituent un cycle pyrrolidine ou N-alkyl pyrrolidine, pipéridine ou N-alkyl pipéridine sont décrits par M.J. Kornet dans J. Med. Chem., 1976, 19 (7), 892-899.

En particulier, la horsfiline de formule :

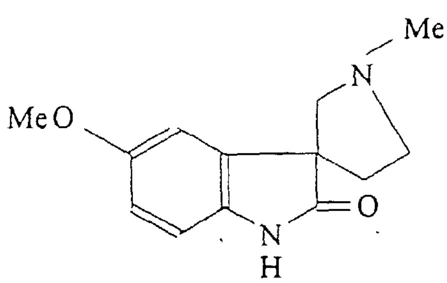

est un alcaloïde décrit dans A. Jossang et al., J. Org. Chem., 1991, 56 (23), 6527-6530.

Les composés (II) dans lesquels $R_3$ et $R_4$ ensemble avec le carbone auquel ils sont liés constituent un groupe $-(CH_2)_pX(CH_2)_q-$ dans lequel p et q sont des nombres entiers dont la somme peut varier de 3 à 6 et X représente l'oxygène, le soufre ou un groupe $NR_{13}$, $R_{13}$ étant un acyle en $C_1$-$C_4$, un benzyle, un alcoxycarbonyle en $C_1$-$C_4$, un carbamoyle non substitué ou substitué par un ou 2 alkyles en $C_1$-$C_4$, sont nouveaux et font partie de l'invention.

Pour préparer un composé de formule (II) dans lequel $R_3$ ou $R_4$ ensemble avec le carbone auquel ils sont liés constituent respectivement un tricyclo [5.2.1.0.2,6]décane ou un tricyclo [5.2.1.0.2,6]dec-8-ène, on fait agir sur un composé de formule (VII), respectivement un composé (VII)' ou un composé (VII)" de formules :

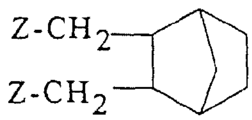

(VII)'

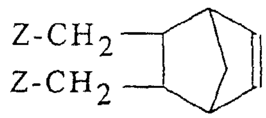

(VII)"

dans lesquelles Z est tel que défini ci-dessus. On utilise des composés (VII)' et (VII)" substitués par un ou plusieurs groupes alkyles en $C_1$-$C_4$ pour préparer des composés (II) dans lesquels lesdits carbocycles sont substitués.

Pour préparer un composé (II) dans lequel $R_3$ et $R_4$ ensemble avec le carbone auquel ils sont liés constituent respectivement un indane ou un hexahydroindane, on fait agir sur un composé (VII), respectivement un composé (VIII) ' ou un composé (VIII)" de formules :

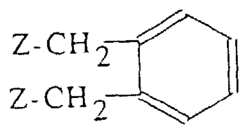

(VIII)'

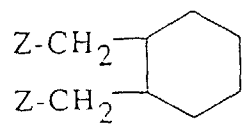

(VIII)"

dans lesquelles Z a la valeur indiquée ci-dessus pour (VIII). On utilise des composés (VIII)' et (VIII)" substitués par un ou plusieurs groupes alkyles en $C_1$-$C_4$ pour préparer des composés (II) dans lesquels l'indane ou l'hexahydroindane sont substitués.

Les composés (II) dans lesquels $R_3$ et $R_4$ ensemble avec le carbone auquel il sont liés constituent un tricyclo [5.2.1.0.2,6]décane, un tricyclo [5.2.1.0.2,6]dec-8-ène, un indane ou un hexahydroindane non substitués ou substitués par un ou plusieurs alkyles en $C_1$-$C_4$ sont nouveaux et font partie de l'invention.

Lorsque $R_3$ et $R_4$ représentent chacun un phényle, on peut utiliser le procédé décrit dans Helv. Chim. Acta, 1946, <u>29</u>, 415-432 pour préparer un composé (II).

Les dérivés de 2-oxoindole (VII) sont connus ou préparés par des méthodes connues. On peut citer par exemple J.V.RajanBabu dans J. Org. Chem., 1986, <u>51</u>, 1704-1712.

Les composés de formule (II) portant certains substituants $R'_1$, $R'_2$ sur leur partie benzénique sont utilisés comme précurseurs pour la préparation de composés de formule (II) portant d'autres substituants $R'_1$, $R'_2$. Par exemple, les composés (II) dans lesquels $R'_1$ et/ou $R'_2$ = H peuvent être nitrés par les réactifs classiques ; ils peuvent également être acylés par action d'un chlorure d'acide de formule RCOCl dans lequel R représente un alkyle en $C_1$-$C_4$, en présence d'un acide de Lewis tel que le chlorure d'aluminium pour préparer un composé (II) dans lequel $R'_1$ et/ou $R'_2$ = COR. A partir d'un composé (II) dans lequel $R'_1$ est un groupe nitro et $R'_2$ est l'hydrogène, on prépare par hydrogénation catalytique le composé (II) dans lequel $R'_1$ est un groupe amino.

Les composés de formule :

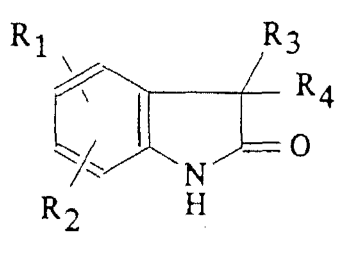

(II)'

dans laquelle :

- $R_1$ et $R_2$ représentent chacun indépendamment un hydrogène, un hydroxyle, un ω-halogénoalcoxy en $C_1$-$C_4$, un

halogène, un alkyle en $C_1$-$C_4$, un trifluorométhyle, un alcoxy en $C_1$-$C_7$, un polyhalogénoalcoxy en $C_1$-$C_4$, un ω-hydroxyalcoxy en $C_2$-$C_4$, un ω-méthoxyalcoxy dans lequel l'alkyle est en $C_2$-$C_4$, un ω-aminoalcoxy en $C_2$-$C_4$ libre ou substitué par un ou deux alkyles en $C_1$-$C_4$ ; un cycloalkyloxy en $C_3$-$C_7$ ; un cycloalkylméthoxy dans lequel le cycloalkyle est en $C_3$-$C_7$ ; un phénoxy ; un benzyloxy; un alkylthio en $C_1$-$C_4$ ; un phénylthio ; un nitro ; un amino libre ou substitué par un ou deux alkyles en $C_1$-$C_4$ ; un cyano ; un acyle en $C_1$-$C_4$ ; un acyloxy en $C_1$-$C_4$ ; un alkylsulfonamido en $C_1$-$C_4$ ; un phénylsulfonamido ; un alkylamido en $C_1$-$C_4$ ; un alcoxycarbonylamino en $C_1$-$C_4$ ; un ureido non substitué ou substitué par un phényle ou par un ou deux alkyles en $C_1$-$C_4$ ;

- $R_3$ et $R_4$ ensemble avec le carbone auquel ils sont liés constituent

  . un adamantane,
  . un indane ou un hexahydroindane non substitués ou substitués par un ou plusieurs groupes alkyles en $C_1$-$C_7$,
  . un tricyclo [5.2.1.0.2,6]décane ou un tricyclo [5.2.1.0.2,6]déc-8-ène non substitués ou substitués par un ou plusieurs groupes alkyles en $C_1$-$C_7$,

  ou

  un cycle hydrocarboné en $C_4$-$C_8$ substitué par un ou plusieurs groupes alkyles en $C_1$-$C_7$ ou par un spirocycloalkyle en $C_3$-$C_5$,

  ou bien $R_3$ et $R_4$ ensemble constituent un groupe $-(CH_2)_p-X(CH_2)_q-$ dans lequel p et q sont des nombres entiers dont la somme peut varier de 3 à 6 et X représente l'oxygène, le soufre ou un groupe $NR_{13}$, $R_{13}$ étant un phényle, un benzyle, un acyle en $C_1$-$C_4$, un alcoxycarbonyle en $C_1$-$C_4$, un carbamoyle non substitué ou substitué par un ou deux alkyles en $C_1$-$C_4$.

avec la limitation que lorsque $CR_3R_4$ représente l'adamantane, $R_1$ et $R_2$ sont différents de l'hydrogène, sont nouveaux et font partie de l'invention.

Les composés de formule :

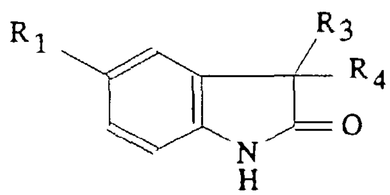

(II)"

dans laquelle

- $R_1$ représente un hydroxyle, un ω-halogénoalcoxy en $C_1$-$C_4$, un halogène, un alkyle en $C_1$-$C_4$, un trifluorométhyle, un alcoxy en $C_1$-$C_7$, un polyhalogénoalcoxy en $C_1$-$C_4$, un ω-hydroxyalcoxy en $C_2$-$C_4$, un ω-méthoxyalcoxy dans lequel l'alkyle est en $C_2$-$C_4$, un ω-aminoalcoxy en $C_2$-$C_4$ libre ou substitué par un ou deux alkyles en $C_1$-$C_4$ ; un cycloalkyloxy en $C_3$-$C_7$ ; un cycloalkylméthoxy dans lequel le cycloalkyle est en $C_3$-$C_7$ ; un phénoxy ; un benzyloxy ; un alkylthio en $C_1$-$C_4$ ; un phénylthio ; un nitro ; un amino libre ou substitué par un ou deux alkyles en $C_1$-$C_4$ ; un cyano ; un acyle en $C_1$-$C_4$ ; un acyloxy en $C_1$-$C_4$ ; un alkylsulfonamido en $C_1$-$C_4$ ; un phénylsulfonamido ; un alkylamido en $C_1$-$C_4$ ; un alcoxycarbonylamino en $C_1$-$C_4$ ; un ureido non substitué ou substitué par un phényle ou par un ou deux alkyles en $C_1$-$C_4$ ;
- $R_3$ et $R_4$ ensemble constituent un groupe $-(CH_2)_pX(CH_2)_q-$ ;

  ou
- $R_3$ et $R_4$ ensemble avec le carbone auquel ils sont liés constituent un cycle hydrocarboné en $C_3$-$C_{10}$, saturé ou insaturé, éventuellement condensé, non substitué ou substitué par un ou plusieurs groupes alkyles en $C_1$-$C_4$ ou par un spirocycloalkyle en $C_3$-$C_5$ ;
- p et q représentent chacun un nombre entier, leur somme peut varier de 3 à 6;
- X représente l'oxygène, le soufre ou un groupe $NR_{13}$ ;
- $R_{13}$ représente l'hydrogène, un alkyle en $C_1$-$C_4$, un phényle, un benzyle, un acyle en $C_1$-$C_4$, un alcoxycarbonyle en $C_1$-$C_4$, un carbamoyle non substitué ou substitué par un ou 2 alkyles en $C_1$-$C_4$ ;

  sous réserve que
- lorsque $R_1$ est méthoxy, $CR_3R_4$ est différent d'une pyrrolidine-3, non substituée ou N-substituée par un alkyle en $C_1$-$C_4$,
- lorsque $R_1$ représente un halogène ou un nitro, $R_3$ et $R_4$ ensemble avec l'atome de carbone auquel ils sont liés soient différents d'un cycle hydrocarboné en $C_3$-$C_{10}$, saturé ou insaturé, et

- lorsque $R_1$ représente un groupe propionyle, $R_3$ et $R_4$ ensemble avec l'atome de carbone auquel ils sont liés ne constituent pas un cyclopropane,

sont nouveaux et font partie de l'invention.

La 2a,3,4,5-tétrahydrobenz[c,d]indol-2 (1 H)-one représentée par la formule

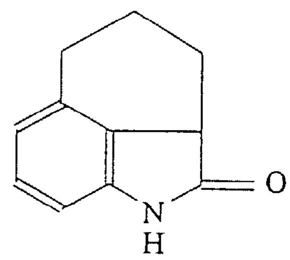

est commerciale, ses dérivés sont connus ou préparés par des méthodes connues.

Les halogénures de benzènesulfonyle (III) sont connus ou préparés par des méthodes connues. Ainsi par exemple, le chlorure de 4-diméthylaminobenzènesulfonyle est préparé selon C.N. Sukenik et al., J. Amer. Chem. Soc., 1977, 99, 851-858. Plus généralement, les halogénures de benzènesulfonyle (III) dans lesquels le substituant $R_5$ est un groupe diméthylamino sont connus ou préparés par des méthodes connues; le chlorure de p-benzyloxybenzènesulfonyle est préparé selon la demande de brevet européen EP 229 566.

Le chlorure d'alcoxybenzènesulfonyle est préparé à partir de l'alcoxybenzènesulfonate de sodium, lui-même préparé par action d'un halogénure d'alkyle sur l'hydroxybenzènesulfonate de sodium.

Le chlorure de 2,4-diméthoxybenzènesulfonyle est préparé selon J. Am. Chem. Soc., 1952, 74, 2008.

On peut préparer les chlorures d'halogénoalcoxybenzènesulfonyle selon le brevet US 2 540 057.

Les halogénures de benzènesulfonyle de formule :

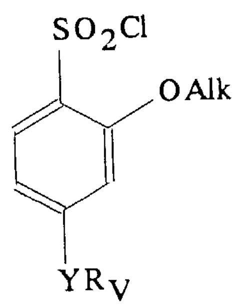

(III)'

dans laquelle

- Alk représente un alkyle en $C_1$-$C_7$ ;
- Y représente O ou S ;
- $R_V$ représente un alkyle en $C_1$-$C_7$, un cycloalkyle en $C_3$-$C_7$, un alcényle en $C_2$-$C_4$, un ω-halogénoalkyle en $C_1$-$C_7$, un polyhalogénoalkyle en $C_1$-$C_7$, un benzyle, un acyle en $C_1$-$C_7$, un ω-carboxyalkyle en $C_1$-$C_7$ estérifié par un alkyle en $C_1$-$C_4$ ou par un benzyle,

sont préparés d'après D. Hofmann et al. dans Liebigs Ann. Chem., 1982, 287-297. On opère sur des composés benzéniques portant les substituants $YR_V$ et OAlk en position -1,3 par action du chlorosulfonate de triméthylsilyle dans un solvant tel que de DCM à TA, On applique ensuite la méthode de R. Passerini et al. dans Gazz. Chim. Ital., 1960, 90, 1277-89 et on neutralise ensuite, par exemple par du carbonate alcalin, puis on fait agir un halogénure tel que $POCl_3$ pour obtenir l'halogénure de benzènesulfonyle souhaité.

Les halogénures de benzènesulfonyle (III) dans lesquels le substituant $R'_5$ représente un alcoxycarbonyle, un phénoxycarbonyle, un benzyloxycarbonyle, un alkylthio, un phénylthio, un benzylthio ou un groupe $SR_7$, $R_7$ étant tel que défini pour (I), sont préparés selon Col. Czechoslov. Chem. Commun., 1984, 49, 1184, à partir d'un dérivé de l'aniline substitué par le même groupement $R'_5$, ledit dérivé de l'aniline est lui-même obtenu à partir du dérivé nitré

correspondant.

Les dérivés de l'acide nitrobenzoïque sont connus ; par une réaction appropriée d'estérification de cet acide, on obtient les esters d'alkyle et de phényle correspondants.

Les dihalogénures de benzène di-sulfonyle (III, $R'_5$ = $SO_2Hal$) sont connus ou préparés par des méthodes connues. Par exemple, le dichlorure de 2,4-diméthoxybenzène-1,5-disulfonyle est décrit dans R.J.W.Cremlyn, J. Chem. Soc. C, 1969, 1344.

Les chlorures d'halogénoalcoxybenzènesulfonyle (III, $R'_5$ = ω-halogénoalcoxy) sont utilisés pour la préparation de composés selon l'invention dans lesquels le substituant $R_5$ est un ω-aminoalcoxy non substitué ou substitué par un ou deux alkyles, selon le schéma suivant:

$$\text{-O-Alk'-Hal} + NHR_8R_9 \rightarrow \text{-O Alk'-}NR_8R_9$$

dans lequel Alk' représente un alkyle en $C_1$-$C_4$.

Pour certaines valeurs des substituants $R_1$, $R_2$, $R_5$ et/ou $R_6$ les composés selon l'invention (I) peuvent être préparés à partir d'un précurseur de formule (I)' substitué par un groupe $R'_1$, $R'_2$, $R'_5$ et/ou $R_{VI}$ appelé groupe précurseur de $R_1$, $R_2$, $R_5$ et/ou $R_6$.

Dans la description qui va suivre, on expose la préparation des composés de formule (I) portant des substituants $R_1$ et/ou $R_5$ ; les mêmes méthodes s'appliquent à la préparation des composés dans lesquels les substituants $R_2$ et/ou $R_6$ ont les valeurs indiquées pour $R_1$ et $R_5$.

Les composés (I) dans lesquels $R_1$ et/ou $R_5$ est un hydroxyle peuvent être obtenus par hydrogénation catalytique, par exemple en présence de palladium sur charbon, d'un composé de formule (I) 'dans lequel $R'_1$ et/ou $R'_5$ est un benzyloxy.

Les composés (I)' dans lesquels $R'_1$ et/ou $R'_5$ représente un hydroxyle permettent de préparer des composés (I) dans lesquels $R_1$ et/ou $R_5$ est un alcoxy par action d'un halogénure d'alkyle en présence d'une base telle qu'un hydrure métallique ou un carbonate alcalin ou alcalino-terreux comme $K_2CO_3$ ou $Cs_2CO_3$ dans un solvant tel que le THF ou le DMF. De même, on prépare les composés de formule (I) dans lesquels $R_1$ et/ou $R_5$ représente un ω-aminoalkyloxy par action d'une ω-chloroalkylamine sur les composés dans lesquels $R'_1$ et/ou $R'_5$ = OH ; de même on prépare les composés dans lesquels $R_1$ et/ou $R_5$ représente un ω-hydroxyalcoxy par action d'un chloroalkylalcool ; dans le cas particulier de la préparation d'un composé (I) dans lequel $R_1$ et/ou $R_5$ = $O(CH_2)_2OH$, on peut également faire agir le carbonate d'éthylène sur un composé (I)' dans lequel $R'_1$ et/ou $R'_5$ = OH.

Les composés de formule (I) dans lesquels $R_1$ et/ou $R_5$ représente un acyloxy sont obtenus par action d'un halogènure d'acide ou d'un anhydride sur un composé (I)' dans lequel $R'_1$ et/ou $R'_5$ est un hydroxyle.

Les composés de formule (I)' dans lesquels $R'_1$ et/ou $R'_5$ représente un amino peuvent subir une alkylation réductrice pour préparer des composés de formule (I) dans lesquels $R_1$ et/ou $R_5$ représente un mono ou un dialkylamino. Lorsque $R'_1$ et/ou $R'_5$ représente un amino, on peut également effectuer une nitrosation, par exemple en présence d'acide nitreux ou de nitrite d'alkyle pour préparer un composé (I) dans lequel $R_1$ et/ou $R_5$ représente un sel de diazonium ; par des réactions connues de l'homme de l'art, on accède alors aux composés (I) selon l'invention dans lesquels $R_1$ et/ou $R_5$ est un cyano, un halogéno ou un thioalkyle en $C_1$-$C_4$. Enfin par des réactions classiques à partir de composés (I)' dans lesquels $R'_1$ et/ou $R'_5$ = $NH_2$, on peut préparer des composés (I) dans lesquels $R_1$ et/ou $R_5$ représente l'un des groupes de formule, RCONH-, ROCONH-, RNHCONH ou $RSO_2NH$-, dans lesquels R représente un alkyle en $C_1$-$C_4$.

Les composés de formule (I)' dans lesquels le substituant $R'_5$ est un phénoxycarbonyle permettent d'obtenir les composés (I) dans lesquels $R_5$ est un phénylcarbamoyle ou un alkylcarbamoyle par action d'une aniline ou d'une alkylamine. Une aniline substituée ou, respectivement, une alkylamine substituée sur l'alkyle permettent d'obtenir des composés de formule (I) dans lesquels $R_5$ est un phénylcarbamoyle ou, respectivement, un alkylcarbamoyle substituée sur l'alkyle.

Les composés de formule (I)' dans lesquels $R'_5$ est un benzyloxycarbonyle permettent d'obtenir par hydrogénation catalytique les composés (I) dans lesquels $R_5$ est un carboxyle. Par action d'un halogénure de thionyle, on obtient les composés de formule (I) dans lesquels $R_5$ est un halogénocarbonyle. A partir de tels composés, on prépare des composés de formule (I) dans lesquels $R_5$ est un carbamoyle N-substitué par action d'une amine substituée.

Les composés de formule (I) dans lesquels $R_5$ est un groupe $COR''_7$ sont préparés à partir de composés (I) ' correspondants dans lesquels $R'_5$ est un phénoxycarbonyle, par action d'une pipérazine ou d'une azétidine substituées.

Un composé (I)' dans lequel $R'_5$ est un groupement nitro permet d'obtenir par hydrogénation catalytique, par exemple en présence d'oxyde de platine, un composé (I) dans lequel $R_5$ est un groupement amino; on peut ensuite préparer d'autres composés dans lesquels le groupe amino est substitué en utilisant des réactions bien connues de l'homme de l'art.

Par exemple lorsque l'on souhaite obtenir un composé (I) selon l'invention dans lequel $R_5$ est un groupe $NR_8R_9$, $R_9$ étant un benzoyle éventuellement substitué, on fait agir sur un composé (I)' dans lequel $R'_5$ est un groupe amino, le chlorure de benzoyle dans lequel le phényle porte le substituant approprié, en présence d'une amine comme la triéthylamine. On peut, par exemple, faire agir le chlorure de 4-chlorosulfonylbenzoyle pour préparer un composés (I)' dans lequel $R'_5$ est un groupe 4-chlorosulfonylbenzamido, puis par action de l'ammoniac ou, respectivement, d'une alkylamine en $C_1$-$C_4$, on obtient un composé (I) dans lequel le substituant $R_5$ est un groupe 4-sulfamoylbenzamido ou, respectivement, un groupe 4-alkylsulfamoylbenzamido.

De la même façon, lorsque l'on souhaite préparer un composé (I) dans lequel $R_5$ est un groupe $NR_8R_9$, $R_9$ étant un acyle en $C_1$-$C_7$, on fait agir sur un composé (I)' dans lequel $R'_5$ est un groupe amino, l'anhydride approprié en présence d'une amine comme la triéthylamine.

Selon un autre exemple de préparation, un composé (I) dans lequel $R_5$ est un groupe alkylsulfonamido est obtenu par action d'un halogénure d'alkylsulfonyle sur un composé (I)' dans lequel $R'_5$ est un groupe amino.

Les composés de formule (I)' dans lesquels $R'_5$ est un groupe amino sont également utiles pour la préparation de composés dans lequel ce groupe amino est substitué par un groupe $(CH_2)_t$-$COR_{12}$. Dans ce cas, on fait agir sur (I)' en présence de chlorure cuivreux, un composé de formule Hal-$(CH_2)_t$-COOAlk dans lequel Hal représente un halogénure, par exemple le brome et Alk représente un alkyle en $C_1$-$C_4$ ; le cas échéant, on transforme l'ester ainsi obtenu en acide ou en amide. On peut utiliser l'action d'une lactone, telle que la butyrolactone ou la valérolactone, sur un composé (I)' dans lequel $R'_5$ est un amino pour préparer le composés (I') dans lequel $R'_5$ =$NHCO(CH_2)_tCO_2H$, avec t = 2 ou 3.

De la même façon, les composés de formule (I) dans lesquels $R_5$ est un groupe amino substitué par un groupe $CH(R_{10})CO_2R_{11}$ sont préparés par action d'un composé de formule Hal-$CH(R_{10})CO_2R_{11}$ sur les composés (I)' correspondants dans lequels le substituant $R'_5$ est un amino.

Par action d'un chloroformiate d'alkyle ou de phényle sur un composé (I)' dont le substituant $R'_5$ est un amino, on prépare un composé (I) dans lequel $R_5$ est un groupe amino substitué par un alcoxycarbonyle ou un phénoxycarbonyle.

Par action de l'ammoniac sur un composé de formule (I)' dans lequel $R'_5$ est un groupe amino substitué par un phénoxycarbonyle, on prépare un composé de formule (I) dans lequel $R_5$ est un ureido ; par action sur un tel composé de formule (I)' d'une aniline ou d'une mono ou dialkylamine en $C_1$-$C_4$, on prépare un composé de formule (I) dans lequel $R_5$ est un N-phénylureido, un N-alkylureido ou N,N-dialkylureido dans lesquels l'alkyle est en $C_1$-$C_4$.

Par action d'une amine appropriée en présence de phosgène sur un composé (I)' dont le substituant $R_5$' est un amino, on prépare un composé (I) dans lequel $R_5$ est un carbamoyle non substitué ou substitué par un ou 2 groupes alkyles.

On peut également préparer un composé (I) dans lequel $R_5$ est un groupe amino substitué par un alkylcarbamoyle ou par un phénylcarbamoyle par action d'un isocyanate d'alkyle ou de phényle sur un composé (I)' dont le substituant $R'_5$ est un amino.

Par ailleurs, un composé (I) dans lequel $R_5$ est un groupe sufamoyle non substitué ou substitué par un alkyle en $C_1$-$C_4$ est préparé par action de l'ammoniac ou d'une alkylamine sur un composé (I)' dans lequel $R'_5$ est un groupe halogénosulfonyle.

Les composés de formule (I)' utiles comme précurseurs pour la préparation de composés de formule (I) sont compris dans la formule (I) et font partie de l'invention.

Parmi les composés de formule (I), les composés de formules (IX), (X), (XI), (XII) et (XIII) ci-dessous utiles pour la préparation d'autres composés de formule (I) représentent des composés préférés selon l'invention.

Ainsi, un objet de la présente invention est constitué par les composés de formule :

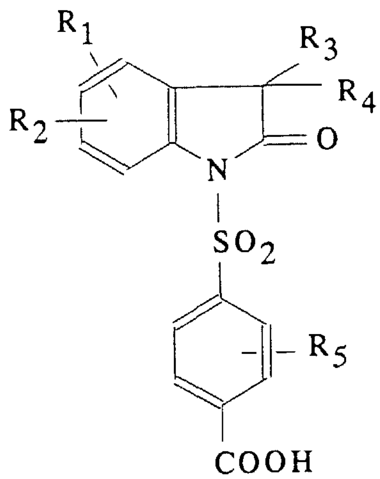

(IX)

dans laquelle $R_1$, $R_2$, $R_3$, $R_4$ et $R_5$ ont les valeurs indiquées ci-dessus pour (I) et leurs dérivés fonctionnels, tels que leurs esters.

Un autre objet de la présente invention est constituée par les composés de formule :

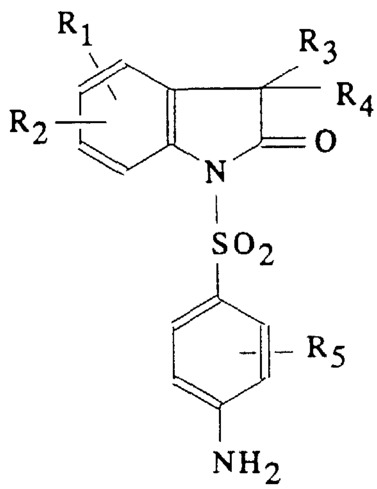

(X)

dans laquelle $R_1$, $R_2$, $R_3$, $R_4$ et $R_5$ ont les valeurs indiquées ci-dessus pour (I) et leurs sels éventuels.

Encore un autre objet de la présente invention est constitué par des composés de formule :

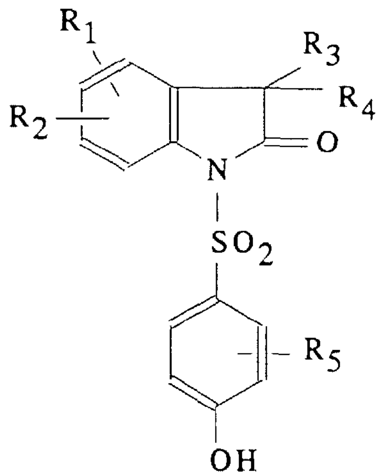

(XI)

dans laquelle $R_1$, $R_2$, $R_3$, $R_4$ et $R_5$ ont les valeurs indiquées ci-dessus pour (I).

Un autre objet de la présente invention est constitué par des composés de formule :

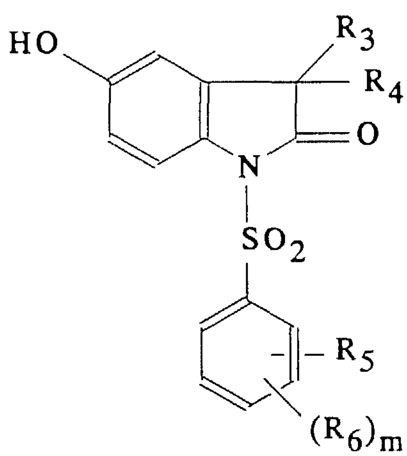

(XII)

dans laquelle $R_3$, $R_4$, $R_5$, $R_6$ et m ont les valeurs indiquées ci-dessus pour

Encore un autre objet de la présente invention est constitué par les composés de formule :

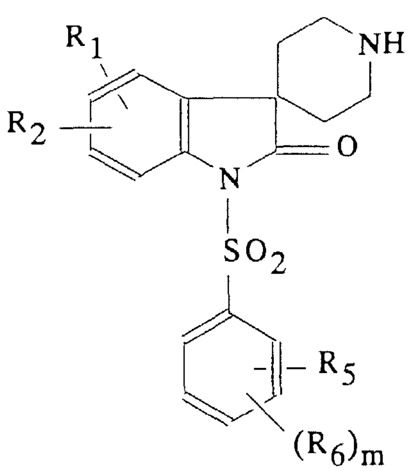

(XIII)

dans laquelle $R_1$, $R_2$, $R_5$, $R_6$ et m ont les valeurs indiquées ci-dessus pour (I).

L'affinité des composés selon l'invention pour les récepteurs de la vasopressine a été déterminée in vitro en utilisant la méthode décrite dans C. J. Lynch et al., J. Biol. Chem., 1985, 260 (5), 2844-2851. Cette méthode consiste à étudier le déplacement de la vasopressine tritiée fixée aux sites $V_1$ de membranes de foie de rats. Les concentrations inhibitrices de 50 % ($CI_{50}$) de la fixation de la vasopressine tritiée des composés selon l'invention sont faibles, allant jusqu'à $10^{-7}$M.

L'affinité des composés (I) selon l'invention pour les récepteurs $V_2$ a été mesurée sur une préparation membranaire de rein de boeuf selon une méthode adaptée de P. Crause et al., Molecular and Cellular Endocrinology, 1982, 28, 529-541 et de F.L. Stassen et al., J. Pharmacol. Exp. Ther., 1982, 223, 50-54. Les composés selon l'invention inhibent la fixation de l'arginine-vasopressine tritiée aux récepteurs de la préparation membranaire. Les $CI_{50}$ des composés selon l'invention sont faibles, allant jusqu'à $10^{-9}$M.

L'activité antagoniste des récepteurs $V_2$ des composés selon l'invention a été montrée par le test de dosage de l'activité adénylate cyclase effectué selon une méthode adaptée de M. Laburthe et al., Molecular Pharmacol., 1986, 29, 23-27. On utilise une préparation membranaire de rein de boeuf et chaque produit est incubé 10 minutes à 37°C, seul ou en présence d'AVP (arginine vasopressine) à la concentration de $3.10^{-8}$M. L'AMP cyclique (adénosine monophosphate cyclique) produite est mesurée par dosage radioimmunologique. On détermine la concentration inhibant de 50 %($CI_{50}$) la stimulation de l'adénylate cyclase induite par $3.10^{-8}$M d'AVP. Les $CI_{50}$ déterminées sont de l'ordre de $10^{-7}$M, allant jusqu'à $10^{-8}$M.

L'activité agoniste ou antagoniste des récepteurs $V_2$ des composés selon l'invention, administrés par voie orale, est évaluée chez le rat (Souche OFA, Sprague-Dawley) en surcharge hydrique, traité à la vasopressine.

De même, l'affinité des composés (I) selon l'invention pour les récepteurs de l'ocytocine a été déterminée in vitro par déplacement d'un analogue radioiodé de l'ocytocine fixé aux récepteurs d'une préparation membranaire de glandes mammaires de rattes gestantes, selon une technique proche de celle décrite par J. Eland et al. dans Eur. J. Pharmacol., 1987, 147, 197-207. Les $CI_{50}$ des composés selon l'invention atteignent $10^{-8}$M.

Les composés selon l'invention sont actifs après administration par différentes voies, notamment par voie orale.

Aucun signe de toxicité n'est observé avec ces composés aux doses pharmacologiquement actives.

Ainsi les composés selon l'invention peuvent être utilisés dans le traitement ou la prévention de différentes affections vasopressine-dépendantes ou ocytocine dépendantes, les affections cardiovasculaires, comme l'hypertension, l'insuffisance cardiaque, ou le vasospasme coronaire, en particulier chez le fumeur, l'ischémie cardiaque, les dérèglements de l'hémostase notamment l'hémophilie, le syndrome de Von Willebrand ; les affections du système nerveux central, les oedèmes cérébraux, la dépression, l'anxiété, les états psychotiques, les troubles de la mémoire par exemple ; les affections du système rénal comme le vasospasme rénal, la nécrose du cortex rénal, l'hyponatriémie, l'hypokaliémie; les affections du système gastrique, l'hépatocirrhose, les ulcères, la pathologie des vomissements, par exemple la nausée, le mal des transports, ou encore le syndrome de la sécrétion inappropriée de l'hormone antidiurétique (SIADH), le diabète insipide et l'énurésie. Les composés selon l'invention peuvent également être utilisés dans le traitement des troubles du comportement sexuel ; chez la femme, les composés selon l'invention peuvent être utilisés pour traiter la dysménorrhée ou le travail prématuré.

La présente invention a également pour objet des compositions pharmaceutiques contenant une dose efficace d'un composé selon l'invention ou d'un sel pharmaceutiquement acceptable et des excipients convenables.

Lesdits excipients sont choisis selon la forme pharmaceutique et le mode d'administration souhaité.

Dans les compositions pharmaceutiques de la présente invention pour l'administration orale, sublinguale, sous-cutanée, intramusculaire, intra-veineuse, topique, intratrachéale, intranasale, transdermique ou rectale, les principes actifs de formule (I) ci-dessus, ou leurs sels éventuels, peuvent être administrés sous formes unitaires d'administration, en mélange avec des supports pharmaceutiques classiques, aux animaux et aux êtres humains pour la prophylaxie ou le traitement des troubles ou des maladies ci-dessus. Les formes unitaires d'administration appropriées comprennent les formes par voie orale telles que les comprimés, les gélules, les poudres, les granules et les solutions ou suspensions orales, les formes d'administration sublinguale, buccale, intratrachéale, intranasale, les formes d'administration sous-cutanée, intramusculaire ou intraveineuse et les formes d'adminitration rectale. Pour l'application topique, on peut utiliser les composés selon l'invention dans des crèmes, pommades ou lotions.

Afin d'obtenir l'effet prophylactique ou thérapeutique désiré, la dose de principe actif peut varier entre 0,01 et 50 mg par kg de poids du corps et par jour.

Chaque dose unitaire peut contenir de 0,5 à 1000 mg, de préférence de 1 à 500 mg, d'ingrédients actifs en combinaison avec un support pharmaceutique. Cette dose unitaire peut être administrée 1 à 5 fois par jour de façon à administrer un dosage journalier de 0,5 à 5000 mg, de préférence de 1 à 2500 mg.

Lorsqu'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose, d'un dérivé cellulosique, ou d'autres matières

appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir ou pour l'administration sous forme de gouttes peut contenir l'ingrédient actif conjointement avec un édulcorant, acalorique de préférence, du méthylparaben et du propylparaben comme antiseptique, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles et injectables qui contiennent des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports ou additifs.

Les compositions de la présente invention peuvent contenir, à côté des produits de formule (I) ci-dessus ou d'un des sels pharmaceutiquement acceptables, d'autres principes actifs qui peuvent être utiles dans le traitement des troubles ou maladies indiquées ci-dessus.

Ainsi, la présente invention a également pour objet des compositions pharmaceutiques contenant plusieurs principes actifs en association dont l'un est un composé selon l'invention.

Ainsi, selon la présente invention, on peut préparer des compositions pharmaceutiques contenant un composé antagoniste des récepteurs $V_1$ associé à un composé agissant sur le système rénine-angiotensine tel qu' un inhibiteur de l'enzyme de conversion, un antagoniste de l'angiotensine II, un inhibiteur de la rénine. On peut également les associer, par exemple, avec un vasodilatateur périphérique ou un inhibiteur calcique. De telles compositions seront utiles en particulier dans le traitement de l'hypertension ou de la défaillance cardiaque.

Préparation des 2-oxoindoles

Préparation 1 :

4,6-Diméthyl-3-spirocyclohexane indol-2-one.

Ce composé est préparé selon Moore et Plant dans J. Chem. Soc., 1951, 3475.

On maintient à reflux sous atmosphère inerte un mélange contenant 15 ml de quinoléine et 10 g d'oxyde de calcium et l'on ajoute en 30 minutes 5 g de 3,5-diméthylphénylhydrazide de l'acide cyclohexane carboxylique (II, $R'_1$, $R'_2$ = $CH_3$, $CR_3R_4$ = cyclohexane). Le milieu réactionnel est refroidi puis versé dans un mélange glace-acide chlorhydrique. On extrait par de l'acétate d'éthyle, lave par de l'acide chlorhydrique normal, par de l'eau jusqu'à neutralité, puis on sèche et concentre sous vide pour obtenir un solide brun. Par trituration dans l'éther iso, on obtient le composé attendu.

F = 223°C

En opérant de la même façon et en faisant varier l'hydrazide de départ, on obtient les dérivés indolone-2 décrits dans le tableau 1 ci-dessous.

Ces composés sont purifiés par chromatographie sur colonne de silice, en éluant par du DCM ou par chromatographie sur colonne d'alumine en éluant par du DCM ou par de l'éther iso.

TABLEAU 1

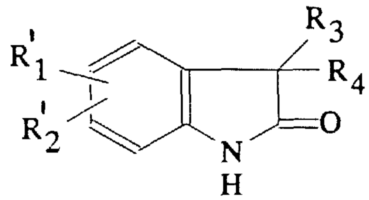

| R'1 | R'2 | CR3R4 | Fc °C |
|---|---|---|---|
| Cl-5 | H | cyclobutane | 191 |
| Cl-5 | H | cyclopentane | 189 |
| Cl-5 | H | cyclohexane | 186 |
| H | H | cyclohexane | 123-124 |
| $CH_3$-5 | H | cyclohexane | 164 |
| $CH_3O$-5 | H | cyclohexane | 226 |
| Cl-6 | H | cyclohexane | 168 |
| $CF_3O$ | H | cyclohexane | 164 |
| $C_6H_5O$-5 | H | cyclohexane | 160 |

Préparation 2 :

La 3-spirocyclohexane indol-2-one décrite dans le tableau 1 ci-dessus peut également être obtenue par alkylation de l'indol-2-one en utilisant le procédé décrit ci-après.

On maintient à -40°C, sous atmosphère d'azote, une solution de 30 g d'indol-2-one dans 900 ml de THF et l'on ajoute 101 g de *tert*-butylate de potassium. On laisse remonter la température à 0°C en 1 heure, puis on refroidit à-60°C et l'on ajoute goutte à goutte une solution de 52 g de 1,5-dibromopentane dans 50 ml de THF. Après 30 minutes à -60°C, on laisse remonter à TA, puis on ajoute 30 ml d'eau et évapore le solvant sous pression réduite. Le résidu est repris par 500 ml de DCM et 200 ml d'eau puis on filtre l'insoluble et sépare la phase organique qui est lavée avec 100 ml d'eau, séchée sur sulfate de magnésium et évaporée sous vide. Le résidu est chromatographié sur silice en éluant par un mélange cyclohexane-éther. On obtient le composé attendu qui est recristallisé dans l'heptane.

m = 34 g

Fc = 123-124°C.

A partir d'autres indol-2-ones et d'autres agents alkylants, on peut appliquer un mode opératoire semblable.

A titre d'exemple, parmi les composés de départ de formule (VII), la 5-chloroindol-2-one est décrite par Bright dans J. Am. Chem. Soc., 1956, 79, 221 et par Rajanbabu dans J. Org. Chem., 1986, 51, 1704. La 4-chloroindol-2-one peut être préparée à partir du 2-chloro-6-nitrotoluène, selon la méthode décrite dans J. Am. Chem. Soc., 1956, 78, 221.

La 5-méthoxyindol-2-one est préparée à partir de la 4-méthoxyaniline selon la méthode décrite dans J. Am. Chem. Soc., 1974, 96, 5512. De la même manière à partir du dérivé aniline approprié, on prépare diverses indol-2-ones:

Préparation 3

5-Ethoxyindol-2-one:

A - 3-Thiométhyl-5-éthoxyindol-2-one.

A une solution refroidie vers -70°C de 12,5 g de chlore dans 400 ml de DCM on ajoute 23,6 g de thiométhyl acétate d'éthyle dans 60 ml de DCM. Après 5 minutes d'agitation à cette même température on additionne une solution 4-éthoxyaniline (48,3 g) dans 120 ml de DCM. On agite une heure vers 70°C, ajoute 39,3 ml de triéthylamine et laisse remonter à température ambiante. On ajoute 200 ml d'eau, décante la phase organique qui est séchée sur sulfate de magnésium et évaporée sous pression réduite. Le résidu est repris dans 500 ml d'isopropanol et 20 ml d'acide chlorhydrique concentré. On agite environ 16 heures à température ambiante, filtre et sépare le précipité. Le filtrat est concentré sous pression réduite pour donner le produit attendu.

B - 5-Ethoxyindol-2-one :

Le solide précédent dans 1500 ml d'éthanol est déthiométhylé en présence de 100 g de nickel de Raney (80 à 100 $m^2$ par g)à reflux durant 3 heures sous atmosphère d'azote. On filtre sur talc, rince avec 1000 ml d'éthanol et concentre le filtrat sous pression réduite. On isole après recristallisation dns le toluène 16 g du produit attendu.

Fc = 156°C

De la même manière, à partir des anilines correspondantes on isole:

| | |
|---|---|
| 5-benzyloxyindol-2-one | Fc = 152°C |
| 5-n-propylindol-2-one | Fc = 136°C |
| 5-éthylindol-2-one | Fc = 152°C |
| 5-(2,2,2-trifluoroéthoxy)indol-2-one | Fc = 145°C |

En opérant selon la technique décrite dans la préparation 2 et en faisant varier le dérivé indol-2-one de départ et le réactif alkylant, on obtient les composés de formule (II) décrits ci-dessous.

TABLEAU 2

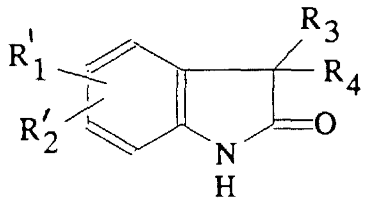

(II)

| $R'_1$ | $R'_2$ | $CR_3R_4$ | Fc °C | Réactif alkylant |
|---|---|---|---|---|
| Cl-5 | H | cyclohexane | 186-189 | $Br(CH_2)_5$ Br |
| Cl-5 | H | cycloheptane | 202 | $Br(CH_2)_6$ Br |
| Cl-5 | H | 4,4-diméthyl cyclohexane | 180 | $TsO(CH_2)_2C(CH_3)_2$- $-(CH_2)_2OTs$ |
| Cl-5 | H | hexahydroindane-2 | 223 | cis-diiodométhyl-1,2 cyclohexane |
| $CH_3O$-5 | H | 4,4-diméthyl cyclohexane | 202 | TsO $(CH_2)_2C(CH_3)_2$- $-(CH_2)_2$-OTs |
| Cl-5 | H | indane-2 | 228 | a,a'-dibromométhyl orthoxylène |
| Cl-5 | H | $C(CH_3)_2$ | 160 | $CH_3I$ |
| Cl-5 | H | $C(CH_2CH_3)_2$ | 156 | $CH_3CH_2I$ |
| Cl-5 | H | C(n Pr)$_2$ | 158 | nPrI |
| Cl-5 | H | C(iBu)$_2$ | 164 | iBuI |
| Cl-5 | H | N-méthyl pipéridine-4 | 260 | $Cl(CH_2)_2N(CH_3)$- $-(CH_2)_2Cl$ |
| Cl-5 | H | tétrahydropyranne-4 | 223 | $I(CH_2)_2O(CH_2)_2I$ |
| Cl-4 | H | cyclohexane | 215 | $Br(CH_2)_5Br$ |
| BzO-5 | H | cyclohexane | 162 | $Br(CH_2)_5Br$ |
| H | H | $C(CH_2C_6H_5)_2$ | 206 | $C_6H_5CH_2Br$ |
| Cl-5 | H | C(n-pentyl)$_2$ | 142 | $CH_3(CH_2)_4Br$ |

| | | | | |
|---|---|---|---|---|
| Cl-5 | H | 2,3-dihydro phénalène-2 | - | 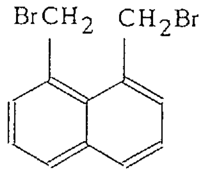 |
| BzO-5 | H | 4,4-diméthyl cyclohexane | 154 | $TsO(CH_2)_2C(CH_3)_2$- -$(CH_2)_2OTs$ |
| Cl-5 | H | 4-spirocyclopentane cyclohexane | 202 | 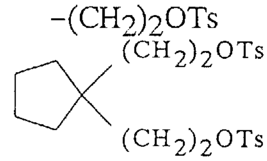 |
| nPr-5 | H | cyclohexane | 151 | $Br(CH_2)_5Br$ |
| EtO-5 | H | N-tBu pipéridine-4 | - | 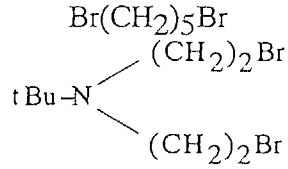 |
| Cl-5 | H | N-Bz pipéridine-4 | 165 | 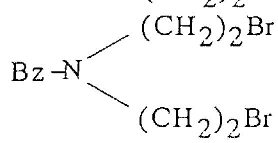 |
| Cl-5 | H | N-phényl pipéridine-4 | 188 | 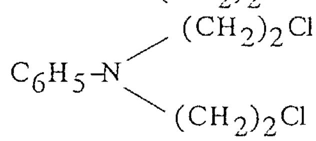 |
| Cl-5 | H | 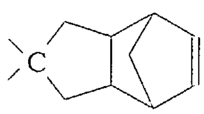 | 300 | 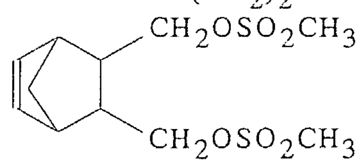 |
| EtO-5 | H | 4,4-diéthyl cyclohexane | 132 | $TsO(CH_2)_2C(C_2H_5)_2$ -$(CH_2)_2OTs$ |
| EtO-5 | H | cyclohexane | 163 | $Br(CH_2)_5Br$ |
| EtO-5 | H | 4,4-diméthyl cyclohexane | 178 | $TsO(CH_2)_2C(CH_3)_2$- -$(CH_2)_2OTs$ |
| EtO-5 | H | cycloheptane | 139 | $Br(CH_2)_6Br$ |
| Et-5 | H | 4,4-diméthyl cyclohexane | 160 | $TsO(CH_2)_2C(CH_3)_2$- -$(CH_2)_2OTs$ |
| $CF_3CH_2O$ -5 | H | 4,4-diméthyl cyclohexane | 164 | idem |
| H | H | 4,4-diméthyl cyclohexane | 169 | idem |

Préparation 4 :

3-Spiroadamantaneindol-2-one.

Ce composé est préparé selon I. Fleming et al., Tetrahedron Letters, 1982, 2053-2056 à partir de 2-bromoaniline et d'adamantane-2-one.

Préparation 5:

5-chloro-3,3-diphénylindol-2-one.

Ce composé est préparé selon la méthode décrite dans Helv. Chim. Acta, 1946, 29, 415-431, par action du benzène sur la 5-chloroisatine en présence de chlorure d'aluminium.
Fc = 281°C.

Préparation 6 :

5-nitro-3-spirocyclohexaneindol-2-one.

Ce composé est préparé selon la méthode décrite dans J. Am. Chem. Soc., 1945, 67, 499 par nitration de la 3-spirocyclohexaneindol-2-one.
Fc = 192°C.

De la même manière, à partir de 3-spiroadamantaneindol-2-one, on prépare la 5-nitro-3-spiroadamantaneindol-2-one.
Fc > 260°C

On prépare également la 5-nitro-3-spiro(4,4-diméthyl)cyclohexaneindol-2-one.
Fc = 195°C

Préparation 7 :

5-amino-3-spirocyclohexaneindol-2-one.

Ce composé est préparé selon la méthode décrite dans J. Chem. Soc., 1951, 3475, par réduction de la 5-nitro-3-spirocyclohexaneindol-2-one, préparée précédemment.
Fc =176°C.

De la même manière, on prépare la 5-amino-3-spiroadamantane.
Fc = 245°C

Préparation 8 :

5-fluoro-3-spirocyclohexaneindol-2-one.

A - tétrafluoroborate de 5-diazonium-3-spirocyclohexaneindol-2-one.

On refroidit à 0°C, une solution contenant 4 g de 5-amino-3-spirocyclohexaneindol-2-one dans 9,2 ml d'acide chlorhydrique 6N et l'on ajoute 2,27 g de nitrite de sodium dans 2,6 ml d'eau puis 2,54 g de tétrafluoroborate de sodium dans 9 ml d'eau. Après 5 minutes sous agitation, on filtre le précipité, lave avec une solution de tétrafluoroborate à 5 %, avec 3 ml de méthanol refroidi vers 0°C, puis avec 5 ml d'éther. Le sel obtenu est séché sous vide à TA en présence d'anhydride phosphorique.

B - 5-fluoro-3-spirocyclohexaneindol-2-one.

1 g du composé obtenu à l'étape A est placé dans 5 ml de xylène et chauffé vers 115°C pendant 2 heures. On refroidit à TA, filtre le précipité, rince au toluène et ajoute au filtrat 0,1 g de charbon actif. Après filtration, le solvant est évaporé sous pression réduite et l'on obtient 0,45 g du composé attendu que l'on recristallise dans du pentane.
Fc = 114°C.

Préparation 9 :

5-cyano-3-spirocyclohexaneindol-2-one.

On dissout à TA 4,78 g de cyanure de potassium et 4,95 g de cyanure cuivreux dans 40 ml de DMSO. On refroidit vers 15°C et l'on ajoute 4,15 g du sel de diazonium obtenu dans la préparation précédente à l'étape A.

Après 30 minutes d'agitation à TA, on ajoute 100 ml d'eau et 100 ml d'éther puis on sépare la phase organique qui est séchée sur sulfate de magnésium et évaporée sous pression réduite. Le résidu est chromatographié sur silice en éluant avec un mélange cyclohexane-éther. On obtient le composé attendu qui est recristallisé dans l'heptane.

m = 1,4 g

Fc = 216°C.

Préparation 10 :

5-chloro-3-spiroadamantaneindol-2-one.

On dissout 1 g de p-chlorophénylhydrazide de l'acide adamantane-2-carboxylique et on ajoute, à -40°C, 2,5 ml de n-butyllithium en solution (1,6 M dans l'hexane). Après 5 minutes d'agitation, on concentre sous vide en maintenant à une température inférieure à 30°C. On ajoute 30 ml de 1,2,3,4-tétraméthybenzène et l'on chauffe à reflux pendant 1 heure. On concentre sous pression réduite, reprend le résidu par de l'acide chlorhydrique normal, extrait à l'éther, lave, sèche et concentre sous vide. L'huile obtenue est chromatographiée sur colonne de silice en éluant par DCM ; on obtient 0,3 g du produit attendu sous forme d'une cire que l'on cristallise dans l'éther iso.

Fc = 249°C.

Préparation 11:

5-chloro-3-cyclohexyl-3-méthylindol-2-one.

On utilise la méthode décrite dans Synth. Commun. 1982, 12 (1), 1-10, pour préparer de façon intermédiaire le 5-chloro-3-cyclohexylindol-2-one puis on obtient le composé attendu par action de l'iodure de méthyle.

Préparation 12

5-Acétyl-3-spirocyclohexaneindol-2-one.

A une solution refroidie à 5°C de 4 g de 3-spirocyclohexaneindol-2-one dans 35 ml de 1,2-dichloroéthane, on ajoute 2,56 g de chlorure d'acétyle puis 8,25 g de chlorure d'aluminium anhydre. On chauffe 2 heures à reflux, évapore le solvant sous pression réduite, hydrolyse le milieu avec 50 g de glace et extrait à l'acétate d'éthyle.

La phase organique est lavée à l'eau, séchée sur sulfate de magnésium puis évaporée sous pression réduite. On chromatographie le résidu sur une colonne de silice en éluant avec un mélange d'heptane et d'éther éthylique et obtient 3,6 g du produit attendu.

Fc = 192°C

En utilisant le mode opératoire décrit, on a préparé les chlorures de benzènesulfonyle décrits dans le tableau ci-après :

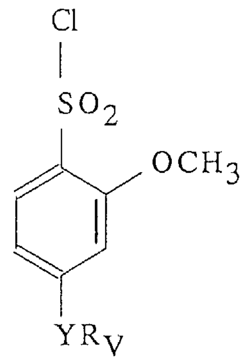

| Y | $R_V$ | F°C |
|---|---|---|
| S | $CH_3$ | 85 |
| O | $CH_2Bz$ | 95 |
| O | $CH_2CO_2Et$ | 89 |
| O | $(CH_2)_3Br$ | 106-108 |

A partir des différents 2-oxoindoles décrits ci-dessus et de chlorures de benzènesulfonyle appropriés on a préparé les composés selon l'invention en utilisant les modes opératoires rapportés dans les exemples qui suivent.

EXEMPLE 1

5-Chloro-1-(2-méthoxy-4-nitrobenzènesulfonyl)-3-spirocyclohexaneindol-2-one.

On agite, sous azote, à TA, pendant 30 minutes, un mélange contenant 0,7 g de 5-chloro-3-spirocyclohexaneindol-2-one et 70 mg d'hydrure de sodium dans 7 ml de THF. On introduit 0,7 g de chlorure de 2-méthoxy-4-nitrobenzènesulfonyle et on maintient l'agitation à TA pendant 20 heures. On concentre sous vide, reprend le résidu par 30 ml d'eau, extrait par de l'acétate d'éthyle, lave à l'eau, puis on sèche et concentre pour obtenir 1,1 g du composé attendu qui cristallise dans l'éther iso.

Fc =188°C.

EXEMPLE 2

1-(4-Amino-2-méthoxybenzènesulfonyl)-5-chloro-3-spirocyclohexaneindol-2-one.

0,8 g du composé obtenu à l'exemple précédent est réduit par l'hydrogène sous pression normale, à TA, pendant 20 heures, dans 10 ml d'acide acétique, en présence de 30 mg d'oxyde de platine. On filtre le milieu réactionnel, concentre, reprend par un mélange eau-acétate d'éthyle, lave à l'eau, sèche et concentre. La mousse jaune obtenue est chromatographiée sur alumine en éluant par DCM. On obtient 0,2 g du produit attendu.

Fc = 173°C

EXEMPLE 3

5-Chloro-1-[4-(2-méthylphénylcarboxamido)-2-méthoxybenzènesulfonyl]-3-spirocyclohexaneindol-2-one.

On laisse sous agitation à TA pendant 48 heures un mélange contenant 0,2 g du composé préparé à l'exemple précédent, 0,5 ml de triéthylamine, 5 ml de DCM et 0,1 g de chlorure d'ortho-toluoyl. On concentre sous vide, reprend par un mélange eau/éther, laisse décanter, lave par une solution saturée d'hydrogénocarbonate de sodium puis par de l'eau, sèche et concentre sous vide pour obtenir 250 mg d'un solide qui est chromatographié sur silice, en éluant par DCM. On obtient 0,1 g du produit attendu.

Fc = 192°C.

EXEMPLE 4

6-Chloro-1-(2,4-diméthoxybenzènesulfonyl)-3-spirocyclohexaneindol-2-one.

On place sous agitation pendant 30 minutes à TA sous azote un mélange contenant 0,15 g de 6-chloro-3-spirocyclohexaneindol-2-one et 15 mg d'hydrure de sodium dans 2 ml de THF ; on introduit 0,15 g de chlorure de 2,4-diméthoxybenzènesulfonyle et on maintient l'agitation à TA pendant 20 heures. On concentre sous vide, reprend le résidu par 30 ml d'eau, extrait par de l'acétate d'éthyle, lave à l'eau, sèche et concentre sous vide. Le produit obtenu est recristallisé dans l'éther iso.

Fc = 147°C.

EXEMPLE 5

Fumarate acide de 5-chloro-1-[4-(3-diméthylaminopropoxy)benzènesulfonyl]-3-spirocyclohexaneindol-2-one.

A) chlorure de 4-(3-bromopropoxy)benzènesulfonyle.

On maintient à reflux pendant 3 heures un mélange contenant 23 g de dihydrate de 4-hydroxybenzènesulfonate de sodium, 7 g de potasse en pastilles (à 85 %), 30 ml d'eau, 50 ml d'éthanol absolu, 40 g de 1,3-dibromopropane et 3,4 g de tétrabutylammonium hydrogénosulfate. Le milieu réactionnel est concentré sous vide, repris par de l'éthanol et concentré une nouvelle fois. Le résidu est repris par du méthanol chaud. On filtre l'insoluble, concentre le filtrat et triture le résidu dans l'éther pour obtenir 22,5 g d'un solide blanc. On ajoute à ce solide 120 ml d'oxychlorure de phosphore et 16 g de pentachlorure de phosphore, on laisse sous agitation 20 heures à TA puis on chauffe 1 heure à reflux. On concentre sous vide, puis le résidu est repris par un mélange éther-eau, décanté, et on lave la phase organique par une solution saturée d'hydrogénocarbonate de sodium. Après séchage et concentration, on obtient le produit attendu sous forme d'une huile jaune.

B) 1-[4-(3-Bromopropoxy)benzènesulfonyl]-5-chloro-3-spirocyclohexaneindol-2-one.

On agite à TA pendant 30 minutes sous azote un mélange contenant 1,2 g de 5-chloro-3-spirocyclohexaneindol-2-one, 0,16 g d'hydrure de sodium dans 6 ml de THF. On ajoute ensuite 1,6 g de chlorure de 4-(3-bromopropoxy) benzènesulfonyle.

Après 20 heures à TA, on concentre sous vide, reprend le résidu par un mélange eau/éther éthylique, décante, lave à l'eau, sèche et concentre la phase organique. L'huile obtenue est purifiée par chromatographie sur silice en éluant par de l'éther iso. Le produit attendu est obtenu sous forme d'huile qui cristallise dans l'éther iso.

m = 1 g

Fc = 123°C.

C) Fumarate acide de 5-chloro-1-[4-(3-diméthylaminopropoxy)benzènesulfonyl]-3-spirocyclohexaneindol-2-one.

On laisse sous agitation à TA pendant 20 heures un mélange contenant 0,5 g du produit obtenu à l'étape ci-dessus, 0,5 g d'iodure de potassium et 20 ml d'une solution de diméthylamine à 33 % dans le méthanol. On concentre le milieu réactionnel, reprend par 10 ml d'eau, et après trituration, on sépare l'insoluble que l'on traite par 10 ml d'acide chlorhydrique 3N. Il se forme une gomme qui est dissoute par 30 ml d'eau tiède, on filtre sur papier puis alcalinise la solution par addition de soude 12N. On extrait l'insoluble à l'éther, lave,sèche puis concentre, on obtient une huile jaune. Celle-ci est dissoute dans 10 ml d'acétone et l'on ajoute à chaud 0,1 g d'acide fumarique.

Le produit attendu précipite à 20°C.

m = 240 mg

Fc = 168°C.

EXEMPLE 6

5-Chloro-1-(2,4-diméthoxybenzènesulfonyl)-3-spiroadamantaneindol-2-one.

On laisse sous agitation pendant 30 minutes à TA, sous atmosphère d'azote un mélange contenant 0,2 g de 5-chloro-3-spiroadamantaneindol-2-one et 20 mg d'hydrure de sodium dans 3 ml de THF. On ajoute 0,18 g de chlorure de diméthoxy-2,4 benzènesulfonyle et on maintient l'agitation à TA pendant 20 heures. On concentre le milieu réactionnel sous vide, reprend le résidu par 30 ml d'eau, extrait par de l'éther, lave à l'eau, sèche et concentre sous vide.

La cire obtenue cristallise dans 15 ml d'éther iso.

m = 240 mg

Fc = 152-154°C.

EXEMPLE 7

5-Chloro-1-(2,4-diméthoxybenzènesulfonyl)-3-spirocycloheptaneindol-2-one.

On porte à -40°C, sous atmosphère inerte, une solution contenant 0,156 g de *tert*-butylate de potassium et 0,33 g de 5-chloro-3-spirocycloheptaneindol-2-one dans 15 ml de THF. On laisse remonter la température vers 10°C en 1 heure puis on refroidit vers -40°C et l'on ajoute goutte à goutte une solution de 0,335 g de chlorure de 2,4-diméthoxybenzènesulfonyle dans 15 ml de THF et on laisse sous agitation à TA pendant 2 heures. Le solvant est évaporé sous pression réduite puis le résidu est repris par 30 ml de DCM et 30 ml d'eau. La phase organique est séparée, lavée par 15 ml d'eau, séchée sur sulfate de magnésium et évaporée sous vide. L'huile obtenue est chromatographiée sur silice en éluant par un mélange cyclohexane-DCM. On obtient le composé attendu qui recristallise dans l'heptane.

m = 0,51 g

Fc = 135°C.

EXEMPLE 8

2,4-Diméthoxy-1-benzènesulfonyl-2a-méthyl-2a,3,4,5-tétrahydrobenz[c, d] indol-2-one. (I : $R_1$ = H, -$R_2$-$R_3$ = $-(CH_2)_3-$, $R_4 = CH_3$, $R_5 = R_6 = OCH_3$). La tétrahydro-2a,3,4,5 benz[c, d] indol-2-one et commerciale. En maintenant à-40°C, et sous atmosphère d'azote, on prépare une solution contenant 0,7 g de ce composé et 1,36 g de *tert*-butylate de potassium dans 40 ml de THF anhydre.

On laisse remonter la température vers 0°C, puis on refroidit à -60°C et l'on ajoute une solution de 0,57 g d'iodure de méthyle dans 20 ml de THF ; on maintient le milieu à -10°C sous agitation pendant 30 minutes, puis on refroidit vers -40°C et l'on ajoute une solution de 0,96 g de chlorure de 2,4-diméthoxybenzènesulfonyle dans 10 ml de THF. Après 16 heures sous agitation à TA, le solvant est évaporé sous pression réduite et l'on reprend le résidu par 30 ml de DCM et 30 ml d'eau ; la phase organique séparée est ensuite séchée sur sulfate de magnésium et évaporée. L'huile obtenue est purifiée par chromatographie sur silice en éluant par un mélange cyclohexane-DCM. On obtient le produit attendu qui est recristallisé dans un mélange cyclohexane/AcOEt (95/5 ;v/v)

Fc = 160°C.

En procédant selon le mode opératoire décrit dans les exemples ci-dessus, à partir des 2-oxoindoles décrites ci-dessus, on a préparé les composés selon l'invention rassemblés dans le tableau 3 ci-après.

TABLEAU 3

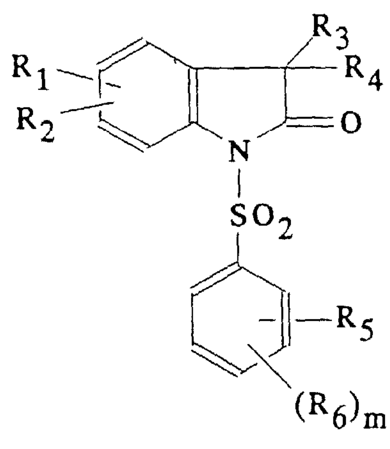

(I)

Sauf indication contraire dans le tableau ci-dessous (*) $R_2$ = H et m = 1.

| Ex. | $R_1$ | $CR_3R_4$ | $R_5$ | $(R_6)m$ | Fc °C |
|---|---|---|---|---|---|
| 9 | Cl-5 | $C(C_6H_5)_2$ | MeO-3 | MeO-4 | 178 |
| 10 | $NO_2$-5 | cyclohexane | MeO-3 | MeO-4 | 157 |
| 11 | Cl-5 | cyclohexane | MeO-4 | H | 112 |
| 12 | Cl-5 | $C(CH_3)_2$ | MeO-3 | MeO-4 | 110 |
| 13 | $NH_2$-5 | cyclohexane | MeO-3 | MeO-4 | 171 |
| 14 | CN-5 | cyclohexane | MeO-2 | MeO-4 | 148 |
| 15 | Cl-5 | cyclohexane | MeO-4 | triMe-2,3,6 | 188 |
| 16 | Cl-5 | $C(Pr)_2$ | MeO-2 | MeO-4 | 186 |
| 17 | Cl-5 | indane-2 | MeO-2 | MeO-4 | 182 |
| 18 | Cl-5 | $C(iBu)_2$ | MeO-2 | MeO-4 | 184 |
| 19 | Cl-5 | N-méthyl pipéridine-4 | MeO-2 | MeO-4 | 142 |
| 20 | Cl-5 | $C(Et)_2$ | MeO-2 | MeO-4 | 190 |
| 21 | F-5 | cyclohexane | MeO-2 | MeO-4 | 149 |
| 22 | Cl-5 | tétrahydro-pyranne-4 | MeO-2 | MeO-4 | 142 |
| 23 | Cl-5 | 4,4-diméthyl cyclohexane | MeO-2 | MeO-4 | 118 |

| | | | | | |
|---|---|---|---|---|---|
| 24 | Cl-5 | hexahydro-indane-2 | MeO-2 | MeO-4 | 89 |
| 25 | Cl-4 | cyclohexane | MeO-2 | MeO-4 | 150 |
| 26 | Cl-5 | cyclohexane | MeO-3 | MeO-4 | 152 |
| 27 | Cl-5 | cyclohexane | Me-4 | H | 150 |
| 28 | H | cyclohexane | MeO-3 | MeO-4 | 107 |
| 29 | Me-5 | cyclohexane | MeO-3 | MeO-4 | 171 |
| 30 | MeO-5 | cyclohexane | MeO-3 | MeO-4 | 124 |
| 31 | Cl-5 | cyclohexane | MeO-2 | MeO-4 | 149 |
| 32 | Cl-5 | cyclohexane | Cl-4 | H | 154 |
| 33 | Cl-5 | cyclobutane | MeO-3 | MeO-4 | 111 |
| 34 | Cl-5 | cyclopentane | MeO-3 | MeO-4 | 106 |
| 35 | Cl-5 | cyclohexane | MeO-4 | Cl-2 | 174 |
| 36 | Cl-5 | cyclohexane | $NO_2$-4 | H | 172 |
| 37 | Cl-5 | cyclohexane | CN-4 | H | 198 |
| 38 | Cl-5 | cyclohexane | MeO-4 | $NO_2$-2 | 147 |
| 39 | Cl-5 | cyclohexane | $CF_3$-4 | H | 139 |
| 40 | Cl-5 | cyclohexane | $CF_3O$-4 | H | 134 |

| | | | | | |
|---|---|---|---|---|---|
| 41 | Cl-5 | cyclohexane | MeO-4 | $NH_2$-2 | 150 |
| 42 * | $CH_3$-4 $R_2$=$CH_3$-6 | cyclohexane | MeO-4 | MeO-2 | 165 |
| 43 | Cl-5 | cyclohexane | Me-3 | BzO-4 | 127 |
| 44 | Cl-5 | cyclohexane | iPr-4 | iPr-2,6 | 172 |
| 45 | Cl-5 | cyclohexane | $CF_3$-2 | H | 154 |
| 46 | Cl-5 | cyclohexane | MeO-2 | 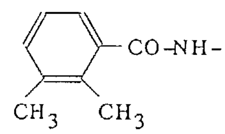 | 215 |
| 47 | Cl-5 | cyclohexane | MeO-4 | 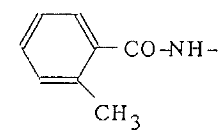 | 193 |
| 48 | Cl-5 | cyclohexane | MeO-2 | $CH_3OCO$-4 | 120 |
| 49 | Cl-5 | 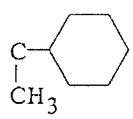 | MeO-2 | MeO-4 | 184 |
| 50 | H | adamantane | MeO-2 | MeO-4 | 172 |
| 51 | MeO-5 | 4,4-diméthyl cyclohexane | MeO-2 | MeO-4 | 152 |
| 52 | Cl-5 | cyclohexane | MeO-2 | $CH_3SO_2NH$-4 | 131 |
| 53 | Cl-5 | cyclohexane | MeO-2 | 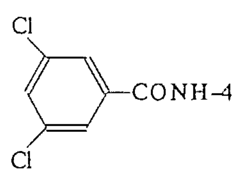 | 240 |

| | | | | | |
|---|---|---|---|---|---|
| 54 | Cl-5 | cyclohexane | Me-2 | F-5 | 153 |
| 55 | Cl-5 | cyclohexane | $CF_3$-$CH_2$O-2 | $CF_3CH_2$O-5 | 175 |
| 56 | Cl-5 | cyclohexane | MeO-2 | 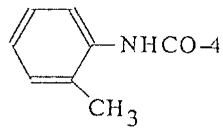 | 218 |
| 57 | Cl-5 | cyclohexane | MeO-2 | 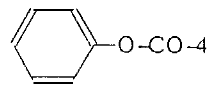 | 165 |
| 58 | Cl-5 | cyclohexane | $NH_2$-$SO_2$-5 | di MeO-2,4 | 270 |
| 59 | BzO-5 | cyclohexane | MeO-2 | MeO-4 | 159 |
| 60 | BzO-5 | 4,4-diméthyl cyclohexane | MeO-2 | MeO-4 | 142 |
| 61 | Cl-5 | cyclohexane | MeO-2 | 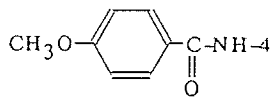 | 192 |
| 62 | Cl-5 | cyclohexane | MeO-2 | 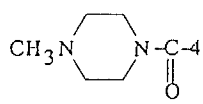 | 158 |
| 64 | H | $C(CH_2C_6H_5)_2$ | MeO-3 | MeO-4 | 146 |
| 65 | $CH_3$CO-5 | cyclohexane | MeO-3 | MeO-4 | 122 |
| 66 | Cl-5 | C(nPentyl)$_2$ | MeO-2 | MeO-4 | 140 |
| 67 | Cl-5 | $C(CH_2C_6H_5)_2$ | MeO-2 | MeO-4 | 185 |
| 68 | $H_2$N-5 | cyclohexane | MeO-2 | MeO-4 | 230 |

| | | | | | |
|---|---|---|---|---|---|
| 69 | Cl-5 | 4-spirocyclopentane cyclohexane | MeO-2 | MeO-4 | 154 |
| 70 | Cl-5 | cyclohexane | MeO-2 | MeO-5 | 116 |
| 71 | nPr-5 | cyclohexane | MeO-2 | MeO-4 | 138 |
| 72 | EtO-5 | N-tBu pipéridine-4 | MeO-2 | MeO-4 | 95 (0,25 $H_2O$) |
| 73 | Cl-5 | N-Bz pipéridine-4 | MeO-2 | MeO-4 | 76 (0,5 $H_2O$) |
| 74 | Cl-5 | N-phényl pipéridine-4 | MeO-2 | MeO-4 | 163 |
| 75 | Cl-5 | cyclohexane | EtO-2 | EtO-4 | 123 |
| 76 | Cl-5 | 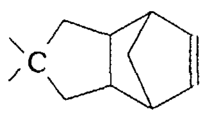 | MeO-2 | MeO-4 | 190 |
| 77 | EtO-5 | 4,4-diéthyl cyclohexane | MeO-2 | MeO-4 | 129 |
| 78 | EtO-5 | cycloheptane | MeO-2 | MeO-4 | 130 |
| 79 | EtO-5 | cyclohexane | MeO-2 | MeO-4 | 134 |
| 80 | EtO-5 | 4,4-diméthyl cyclohexane | MeO-2 | MeO-4 | 160 |
| 81 | Et-5 | 4,4-diméthyl cyclohexane | MeO-2 | MeO-4 | 166 |

| | | | | | |
|---|---|---|---|---|---|
| 82 | EtO-5 | 4,4-diméthyl cyclohexane | MeO-2 | $NO_2$-4 | 110 |
| 83 | EtO-5 | 4,4-diméthyl cyclohexane | MeO-2 | $NH_2$-4 | 230 |
| 84 | $NO_2$-5 | 4,4-diméthyl cyclohexane | MeO-2 | MeO-4 | 102 |
| 85 | $NH_2$-5 | 4,4-diméthyl cyclohexane | MeO-2 | MeO-4 | 180 |
| 86 | $CF_3CH_2O$ -5 | 4,4-diméthyl cyclohexane | MeO-2 | MeO-4 | 169 |

EXEMPLE 87

1-(2,4-Diméthoxybenzènesulfonyl)-3-(4,4-diméthylspirocyclohexane)-5-hydroxyindol-2-one.

On agite à 50°C pendant 1 heure sous atmosphère d'hydrogène 3,51 g du composé préparé à l'exemple 60 avec 0,5 g de palladium à 10 % sur charbon dans 150 ml d'éthanol. On filtre le catalyseur sur talc, rince au DCM et évapore le filtrat sous pression réduite. On obtient 2,8 g du composé attendu qui est recristallisé dans un mélange cyclohexane-AcOEt (90/10, v/v).

Fc = 220°C

EXEMPLE 88

De la même manière, on prépare le 1-(2,4-diméthoxybenzènesulfonyl)-5-hydroxy-3-spirocyclohexaneindol-2-one à partir du dérivé 5-benzyloxy décrit à l'exemple 59.

Fc = 196°C

EXEMPLE 80 bis

1-(2,4-Diméthoxybenzènesulfonyl)-3-(4,4-diméthylspirocyCLohexane)-5-éthoxyindol-2-one.

Ce composé déjà décrit à l'exemple 80 peut être préparé selon une autre méthode à partir du composé homologue en 5-hydroxy. On agite à TA durant 16 heures, sous atmosphère inerte 0,6 g du composé préparé à l'exemple 87 avec 0,19 g de carbonate de potassium anhydre et 0,315 g d'iodure d'éthyle dans 11 ml de DMF. On évapore le solvant sous pression réduite, ajoute 30 ml d'AcOEt et 30 ml d'eau. La phase organique est lavée à l'eau, séchée sur sulfate de magnésium puis concentrée sous pression réduite.

On obtient 0,45 g du produit attendu par cristallisation dans le cyclohexane.

Fc = 160°C

De la même manière, on prépare les composés décrits dans le tableau 4 ci-dessous :

TABLEAU 4

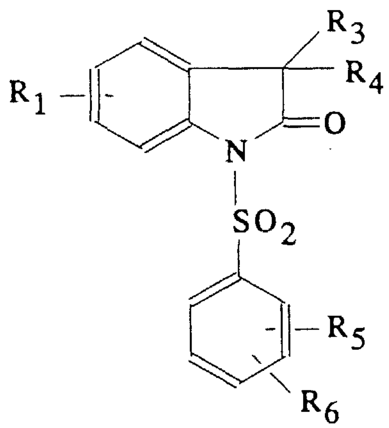

(I)

| Ex. | $R_1$ | $CR_3R_4$ | $R_5$ | $R_6$ | F°C |
|---|---|---|---|---|---|
| 89 | nPrO-5 | cyclohexane | MeO-2 | MeO-4 | 139 |
| 90 | nPrO-5 | 4,4-diméthyl cyclohexane | MeO-2 | MeO-4 | 158 |
| 91 | iPrO-5 | 4,4-diméthyl cyclohexane | MeO-2 | MeO-4 | 154 |
| 92 | 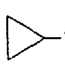$-CH_2-O$ | cyclohexane | MeO-2 | MeO-4 | 155 |

EXEMPLE 93

5-Acétoxy-1-(2,4-diméthoxybenzènesulfonyl)-3-spirocyclohexaneindol-2-one.

On chauffe vers 65°C pendant 15 heures 0,5 g du composé préparé à l'exemple 88, 2,5 ml d'acétate d'isopropényle et 0,165 g de carbonate de potassium dans 2,5 ml de toluène et 0,3 ml de DMF. Après refroidissement, on ajoute 10 ml d'eau, 15 ml d'acétate d'éthyle, décante et lave à l'eau la phase organique qui est séchée sur sulfate de magnésium et concentrée sous pression réduite. Par cristallisation dans un mélange cyclohexane-acétate d'éthyle, on isole 0,51 g du composé attendu comprenant 0,5 mole de cyclohexane.

Fc = 116°C

EXEMPLE 94

1-(2,4-diméthoxybenzènesulfonyl)-5-(2-hydroxyéthoxy)-3-spirocyclohexaneindol-2-one.

On chauffe vers 70°C pendant 40 heures, 0,5 g du composé préparé à l'exemple 88, 0,5 g de carbonate

d'éthylène et 0,272 g de carbonate de potassium anhydre dans 1,25 ml de DMF. Après refroidissement, on ajoute 10 ml d'eau, 15 ml d'acétate d'éthyle, décante et lave à l'eau la phase organique qui est séchée sur sulfate de magnésium et concentrée sous pression réduite. Le résidu huileux est chromatographié sur colonne de silice en éluant avec un mélange cyclohexane-AcOEt (70/30, v/v). On obtient 0,5 g du produit attendu qui est recristallisé dans un mélange heptane-DCM.

Fc =170°C

EXEMPLE 95

5-(2-Diméthylaminoéthoxy)-1-(2,4-diméthoxybenzènesulfonyl)-3-spirocyclohexane-indol-2-one.

On chauffe vers 40°C, pendant 16 heures, sous atmosphère inerte, 0,6 g du composé préparé à l'exemple 88 avec 0,32 g de N,N-diméthyl-(2-chloroéthylamine) et 1,76 g de carbonate de césium dans 7,2 ml d'acétone et 2,4 ml de DMF. On filtre les sels et ajoute 20 ml d'eau et 20 ml d'AcOEt au filtrat. La phase organique est décantée, lavée à l'eau, séchée sur sulfate de magnésium et concentrée sous pression réduite. Le résidu est chromatographié sur silice en éluant avec un mélange DCM-MeOH (9/1, v/v). On obtient 0,6 g du produit attendu que l'on recristallise dans un mélange cyclohexane-éther iso.

Fc = 122°C

EXEMPLE 96

5-Chloro-1-(2,4-diméthoxybenzènesulfonyl)-3-(spiropipéridine-4)indol-2-one.

Cette réaction est effectuée selon J. Org. Chem., 1984, 49, 2795-2799. On ajoute à 0°C, 0,75 g de chloroformiate de 1-chloroéthyle à une solution de 1,31 g du composé décrit à l'exemple 73 et 0,32 g de bis-1,8-diméthylaminonaphtalène dans 22 ml de 1,2-dichloroéthane. On chauffe environ 20 minutes à reflux, concentre sous pression réduite à un volume d'environ 10 ml puis ajoute 22 ml de méthanol. Après chauffage pendant 50 minutes à reflux, on concentre le milieu réactionnel sous pression réduite et chromatographie le résidu sur une colonne de silice en éluant avec un mélange DCM-MeOH (95/5, v/v). On isole 1,16 g du produit attendu qui est recristallisé dans un mélange de cyclohexane et d'acétate d'éthyle.

Fc = 172°C

EXEMPLE 97

3-(N-Acétylspiropipéridine-4)-5-chloro-1-(2,4-diméthoxybenzènesulfonyl)-indol-2-one.

A une solution refroidie vers 0°C de 0,35 g du composé préparé à l'exemple précédent et 0,23 ml de triéthylamine dans 5 ml de DCM, on ajoute 0,086 ml de chlorure d'acétyle. On agite une heure à 20°C, ajoute 5 ml d'eau, décante et lave à l'eau la phase organique, sèche sur sulfate de magnésium, concentre sous pression réduite et chromatographie le résidu sur une colonne de silice en éluant avec un mélange DCM-MeOH (99/1, v/v). On isole 0,29 g du produit attendu sous forme d'hémihydrate.

Fc = 107°C

EXEMPLE 98

5-Chloro-1-(2,4-diméthoxybenzènesulfonyl)-3-(N-méthoxycarbonylspiropipéridine-4)indol-2-one.

Ce composé est préparé à partir de celui obtenu à l'exemple 96 par action du chloroformiate de méthyle.

Fc =147°C

EXEMPLE 99

1-(3,4-diméthoxybenzènesulfonyl)-5-propanamido-3-spirocyclohexaneindol-2-one.

A une solution refroidie vers 0°C de 0,5 g du composé décrit à l'exemple 13 et 0,167 ml de triéthylamine dans 10 ml de DCM, on ajoute une solution de 0,144 g de chlorure de propionyle dans 3 ml de DCM. On agite 2 heures à 20°C puis ajoute 20 ml d'eau, décante, lave à l'eau la phase organique, sèche sur sulfate de magnésium et concentre sous pression réduite. On isole 0,5 g du produit attendu après recristallisation dans un mélange heptane-AcOEt (95/5,

v/v).
Fc = 158°C

EXEMPLE 100

1-(3,4-diméthoxybenzènesulfonyl)-5-(N-méthylureido)-3-spirocyclohexaneindol-2-one.

A une solution refroidie vers 0°C de 0,5 g du composé décrit à l'exemple 13, dans 10 ml de DCM, on ajoute 0,15 g d'isocyanate de méthyle. Après environ 16 heures à TA sous agitation, on ajoute 20 ml d'eau, décante et lave à l'eau la phase organique, sèche sur sulfate de magnésium et concentre sous pression réduite. Après recristallisation dans un mélange d'heptane et d'acétate d'éthyle on isole 0,5 g du produit attendu.
Fc = 214°C

De la même manière, on prépare le composé décrit dans l'exemple suivant :

EXEMPLE 101

1-(3,4-diméthoxybenzènesulfonyl)-5-(N-phénylureido)-3-spirocyclohexaneindol-2-one.

Fc = 124°C

EXEMPLE 102

5-Diméthylamino-1-(2,4-diméthoxybenzènesulfonyl)-3-spirocyclohexaneindol-2-one.

Sous atmosphère d'azote on agite à TA un mélange de 0,5 g du composé décrit à l'exemple 68 avec 0,5 ml d'une solution à 35 % de formaldehyde, 0,12 g de cyanoborohydrure de sodium dans 10 ml d'acétonitrile et ajuste le pH vers 6,5 avec quelques gouttes d'acide acétique. Après 48 heures à 20°C on évapore le solvant sous pression réduite, ajoute 20 ml de solution aqueuse de soude environ 2N et 20 ml de DCM. On décante la phase organique, lave à l'eau, sèche sur sulfate de magnésium et évapore le solvant sous pression réduite. Le résidu est chromatographié sur une colonne de silice en éluant avec un mélange cyclohexane-acétate d'éthyle (80/20, v/v). On isole 0,27 g du produit attendu.
Fc = 167°C

EXEMPLE 103

1-(2,4-Diméthoxybenzènesulfonyl)-5-éthylthio-3-spirocyclohexaneindol-2-one.

Ce composé est préparé selon J. Chem. Soc., Chem. Commun., 1980, 16, 756. Sous atmosphère inerte on chauffe vers 80°C un mélange de 2,95 g de disulfure de diéthyle et 0,386 g de nitrite d'isopentyle et ajoute 0,8 g du composé préparé à l'exemple 68. Le milieu est agité une heure à 80°C puis concentré sous pression réduite. Le résidu est chromatographié sur une colonne de silice en éluant avec un mélange DCM-cyclohexane (80/20, v/v). On isole après cristallisation dans le cyclohexane le produit attendu.
Fc = 123°C

EXEMPLE 104

5-Chloro-1-[4-(diméthylaminométhylcarboxamido)-2-méthoxybenzènesulfonyl]-3-spirocyclohexaneindol-2-one.

A) 5-Chloro-1-[4-(chlorométhylcarboxamido)-2-méthoxybenzènesulfonyl]-3-spirocyclohexaneindol-2-one

0,2 g du composé préparé à l'exemple 2 est placé dans 4 ml de DCM et 0,5 g de TEA à TA et l'on ajoute 0,1 g de chlorure de chloroacétyle. Après 20 heures d'agitation à TA, on concentre sous vide. On extrait à l'acétate d'éthyle, lave à l'eau, par une solution de carbonate de sodium, puis le résidu est chromatographié sur silice en éluant par un mélange de DCM et AcOEt. On obtient 0,15 g du produit attendu.

B) 5-Chloro-1-[4-(diméthylaminométhylcarboxamido)-2-méthoxybenzènesulfonyl]-3-spirocyclohexaneindol-2-one.

Le composé obtenu à l'étape précédente (150 mg) est agité à TA pendant 20 heures dans 20 ml de diméthylamine à 33 % dans l'éthanol. On extrait par AcOEt, lave par de la soude N puis par de l'eau. Le résidu est chromatographié sur silice en éluant par AcOEt. On obtient 0,025 g du produit attendu.

Fc = 173°C

EXEMPLE 105

1-[4-(4-Sulfamoylphénylcarboxamido)-2-méthoxybenzènesulfonyl]-5-chloro-3-spirocyclohexaneindol-2-one.

Le chlorure de 4-chlorosulfonylbenzoyle est préparé selon Chem. Ber., 1941, 271.

On place 0,2 g du composé préparé à l'exemple 2, en présence de 0,5 g de TEA dans 5 ml de DCM ; on ajoute 0,13 g de chlorure de 4-chlorosulfonylbenzoyle et l'on agite pendant 20 heures à TA. On concentre sous vide, reprend par du THF et ajoute 10 ml d'ammoniaque. On laisse à nouveau 20 heures sous agitation à TA et concentre sous vide. Le résidu est extrait à l'éther, lavé à l'eau, séché au sulfate de sodium puis on chromatographie sur silice en éluant par AcOEt. On obtient le produit attendu.

Fc = 238-242°C, après recristallisation dans AcOEt.

EXEMPLE 106

1-[4-(3-Sulfamoylphénylcarboxamido)-2-méthoxybenzènesulfonyl]-5-chloro-3-spirocyclohexaneindol-2-one.

A) Chlorure de 3-chlorosulfonylbenzoyle.

Ce composé est préparé selon le brevet US 3 290 370. On chauffe à 60°C, 11 g d'acide chlorosulfonique auquel on ajoute goutte à goutte 8 g de phénylchloroforme. Après 2 heures de chauffage à 130°C, on distille pour obtenir 1 g du produit attendu.

Eb = 120-125°C, sous 0,5 mm Hg.

B) 1-[4-(3-Sulfamoylphénylcarboxamido)-2-méthoxybenzènesulfonyl]-5-chloro-3-spirocyclohexaneindol-2-one.

Dans 10 ml de DCM, on place 210 mg du composé préparé à l'exemple 2 avec 220 mg du composé obtenu à l'étape précédente et 200 mg de TEA, on laisse sous agitation une nuit puis on évapore les solvants sous vide. Le résidu est repris dans 20 ml de THF et 20 ml d'ammoniaque et on laisse sous agitation pendant 6 heures à TA. On chasse les solvants sous vide puis on reprend le résidu par AcOEt et de l'eau. On extrait par AcOEt, lave à l'eau puis on effectue une chromatographie sur silice en éluant par un mélange AcOEt-cyclohexane (50/50, v/v) pour obtenir le produit attendu.

Fc = 176°C

EXEMPLE 107

1-[4-(2-carboxyphénylcarboxamido)-2-méthoxybenzènesulfonyl]-5-chloro-3-spirocyclohexaneindol-2-one.

La préparation est effectuée selon le J. Heterocycl. Chem., 1974, 997-1000. On agite à 60°C pendant 3 heures un mélange contenant 0,2 g du composé préparé à l'exemple 2 avec 0,5 ml de TEA et 160 mg d'anhydride phtalique. On concentre sous vide et traite par de l'acide chlorhydrique normal. Le précipité formé est essoré et traité par une solution de carbonate de sodium à 10 % ; il se forme à nouveau un précipité, on décante la phase aqueuse et on traite le précipité par AcOH à 10 %. On essore le précipité puis on le lave par AcOH à 10 % puis de l'éther isopropylique et l'on recristallise dans l'éther iso pour obtenir le produit attendu.

m = 0,150 g

Fc = 157-158°C

EXEMPLE 108

1-[4-(Benzyloxyméthylcarboxamido)-2-méthoxybenzènesulfonyl]-5-chloro-3-spirocyclohexaneindol-2-one.

Ce composé est préparé selon le mode opératoire décrit à l'exemple 3 par action du chlorure de benzyloxyacétyle sur le composé préparé à l'exemple 2.

Fc = 143°C, après recristallisation dans éther iso.

EXEMPLE 109

5-Chloro-1-[4-(hydroxyméthylcarboxamido)-2-méthoxybenzènesulfonyl]-3-spirocyclohexaneindol-2-one.

Ce composé est obtenu par hydrogénation sous pression d'une colonne d'eau du composé préparé à l'exemple précédent en présence de Palladium sur charbon à 5 % dans un mélange EtOH/AcOEt..

Fc = 202°C

EXEMPLE 110

5-Chloro-1-[4-(imidazol-1-ylphénylcarboxamido)-2-méthoxybenzènesulfonyl]-3-spirocyclopentaneindol-2-one.

A) Ester éthylique de l'acide 4-(imidazol-1-yl)benzoique.

On porte à reflux pendant 15 minutes un mélange contenant 35 g de chlorure de 4-fluorobenzoyle dans 50 ml d'éthanol 100. 35 g de l'ester éthylique de l'acide 4-fluorobenzoique obtenu sont mélangés avec 22 g d'imidazole et 61 g de carbonate de potassium dans 35 ml de DMSO. Le mélange est chauffé pendant 18 heures à 120-130°C puis on ajoute 500 ml d'eau glacée. Il se forme un précipité et le produit attendu cristallise dans l'éther iso.

Fc = 98°C

B) Chlorure d'imidazol-1-yl benzoyle.

On porte à reflux pendant 2 heures 5 g de l'ester obtenu à l'étape A dans 20 ml d'eau et 20 ml de lessive de soude. Le milieu réactionnel est lavé à l'éther puis acidifié (pH 2) par de l'acide chlorhydrique concentré. Le précipité formé est essoré puis lavé à l'éther iso. On porte à reflux 5 g de l'acide obtenu dans 35 ml de chlorure de thionyle. On filtre le précipité formé puis on le lave à l'éther iso pour obtenir le chlorure d'acide attendu.

Fc = 243°C

C) 5-Chloro-1-[4-(imidazol-1-ylphénylcarboxamido)-2-méthoxybenzènesulfonyl]-3-spirocyclopentaneindol-2-one.

On laisse sous agitation à TA pendant 1 h un mélange contenant 210mg du composé préparé à l'exemple 2 et 200 mg du chlorure d'acide préparé à l'étape B dans 10 ml de DCM et 1,5 ml de TEA puis on porte à reflux pendant 3 heures. Le milieu réactionnel est extrait au DCM puis lavé à l'eau et par une solution aqueuse de soude. Après évaporation des solvants, le résidu est chromatographié sur silice en éluant par un mélange DCM-méthanol. Le produit attendu est recristallisé de l'éther iso.

m = 0,010 g

Fc = 145°C

EXEMPLE 111

5-Chloro-1-[2-méthoxy-4-(phénoxycarboxamido)benzènesulfonyl]-3-spirocyclohexaneindol-2-one.

Ce composé est préparé par action du chloroformiate de phényle sur le composé préparé à l'exemple 2.

Fc = 209°C après recristallisation dans l'éther iso.

EXEMPLE 112

5-Chloro-1-[4-(N-méthylureido)-2-méthoxybenzénesulfonyl]-3-spirocyclohexaneindol-2-one.

On mélange 140 mg du composé obtenu à l'exemple précédent avec 5 ml d'éthanol, 5 ml de DCM et 5 ml de méthylamine à 33 % dans l'éthanol. Après une heure à TA, on chasse les solvants puis on chromatographie sur silice en éluant par un mélange DCM-MeOH. Le produit obtenu est recristallisé dans l'éther iso.

Fc = 254°C

EXEMPLE 113

5-Chloro-1-(2-méthoxy-4-ureidobenzènesulfonyl)-3-spirocyclohexaneindol-2-one.

On agite pendant 1 heure à TA un mélange contenant 200 mg du composé préparé à l'exemple 111 avec 5 ml d'ammoniaque à 20 %, 5 ml d'éthanol et 5 ml de DCM. Après filtration du milieu réactionnel et évaporation des solvants, on fait cristalliser le produit attendu dans l'éther iso.

Fc = 228°C

EXEMPLE 114

5-chloro-1-[4-(N-o-tolylureido)-2-méthoxybenzènesulfonyl]-3-spirocyclohexaneindol-2-one.

On porte à reflux pendant 18 heures un mélange contenant 250 mg du composé préparé à l'exemple 2, 10 ml de xylène et 80 mg d'isocyanate d'orthotoluyle. Il se forme un précipité blanc que l'on filtre. On extrait à l'éther le milieu réactionnel, lave à l'eau puis on effectue une chromatographie sur silice en éluant par un mélange DCM-MeOH. Le produit attendu cristallise dans l'éther iso.

Fc = 182°C

EXEMPLE 115

4-(5-méthoxy-2-oxo-3-spirocyclohexaneindol-1-yl)sulfonyl-3-méthoxybenzoate de benzyle.

On verse par petites fractions 60 mg d'hydrure de sodium sur un mélange contenant 500 mg de 3-spirocyclohexane-5-méthoxyindol-2-one dans 50 ml de THF. Après 30 minutes à TA, on ajoute 800 mg de 3-méthoxy-4-chlorosulfonylbenzoate de benzyle et on laisse sous agitation 2 heures à TA. On concentre le milieu, reprend par AcOEt, lave à l'eau, sèche sur sulfate de sodium et concentre. Le résidu est chromatographié sur silice en éluant par DCM.

RMN: (à 250 MHz dans le DMSO)

1,2-1,8 ppm : 10 H : cyclohexyle
3,6 ppm et 3,8 ppm : 2x3H ; 2x$OCH_3$
5,4 ppm : 2 H : $CO_2$-C<u>H</u>$_2$-$C_6H_5$
6,8-8,2 ppm : 11 H : aromatiques

EXEMPLE 116

Acide 4-(3-spirocyclohexane-5-méthoxy-2-oxoindol-1-yl)sulfonyl-3-méthoxy benzoïque.

600 mg du composé préparé à l'exemple précédent sont placés dans 50 ml de AcOEt et hydrogénés à TA et pression atmosphérique en présence de 140 mg de Palladium sur Charbon. On obtient 310 mg de l'acide attendu qui est recristallisé du mélange hexane-éthanol (70/30, v/v).

Fc = 210°C

EXEMPLE 117

5-chloro-1-[4-(N-(éthoxycarbonylméthyl)carbamoyl)-2-méthoxybenzènesulfonyl]-3-spirocyclohexaneindol-2-one.

450 mg de chlorhydrate de glycinate d'éthyle dans 20 mg de méthylate de sodium sont placés dans du méthanol. On ajoute 200 mg du composé décrit à l'exemple 57 dans 50 ml de DCM et on laisse sous agitation à TA pendant 48 heures. On extrait au DCM, lave à l'eau, sèche et concentre puis on chromatographie sur silice en éluant par DCM/MeOH (99,5/0,5, v/v).

Fc = 164°C

EXEMPLE 118

1-(4-carbamoyl-2-méthoxybenzènesulfonyl)-5-chloro-3-spirocyclohexaneindol-2-one.

On mélange 300 mg du composé décrit à l'exemple 57 avec 5 ml d'ammoniaque à 30 %, 10 ml d'éthanol et 10 ml de DCM. Après 1 heure à TA, on concentre, extrait au DCM, lave à l'eau, sèche et concentre puis on chromatographie sur silice en éluant par DCM-MeOH (99/1, v/v). On obtient 109 mg du produit attendu.
Fc = 160°C

EXEMPLE 119

5-Chloro-1-[2-méthoxy-4-(N-(2-méthoxycarbonyléthyl)carbamoyl)benzènesulfonyl]-3-spirocyclohexaneindol-2-one.

On porte à reflux pendant 30 minutes un mélange comprenant 320 mg du composé décrit à l'exemple 57, 2 g d'aminobispropionate de méthyle dans 30 ml de tétraméthylbenzène. On extrait par AcOEt, lave par une solution d'acide chlorhydrique 1N, sèche sur sulfate de sodium et concentre. Le résidu est chromatographié sur silice en éluant par DCM-MeOH (99/1, v/v). On obtient 100 mg du produit attendu.
Fc =147°C

EXEMPLE 120

1[4-(3-(N-Boc)aminoazetidin-1-ylcarbonyl)-2-méthoxybenzènesulfonyl]-5-chloro-3-spirocyclohexaneindol-2-one.

On laisse sous agitation à TA pendant 1 heure un mélange contenant 300 mg du composé préparé à l'exemple 57, 900 mg de 3-(N-Boc)aminoazétidine, 1 ml de triéthylamine, 10 ml de DCM et 10 ml de méthanol. On concentre, extrait à l'acétate d'éthyle, lave par une solution d'acide chlorhydrique 1N, sèche sur sulfate de sodium et concentre. Après chromatographie sur silice en éluant par DCM-MeOH (99/1,v/v), on obtient le produit attendu.
Fc = 136°C

EXEMPLE 121

1-[4-(3-aminoazétidin-1-ylcarbonyl)-2-méthoxybenzènesulfonyl)-5-chloro-3-spirocyclohexaneindol-2-one.

On agite pendant 30 minutes à TA un mélange contenant 160 mg du composé préparé à l'exemple précédent et 3 ml de TFA dans 10 ml de DCM. On concentre le milieu réactionnel, cristallise dans l'éther iso, filtre et sèche. Le produit obtenu est dissous dans 10 ml d'eau, puis 10 ml de soude 1N ; on extrait au DCM, lave à l'eau, sèche sur sulfate de sodium et concentre. Après chromatographie sur silice en éluant par DCM-MeOH (96/4, v/v), on obtient le produit attendu.
Fc = 145°C

EXEMPLE 122

Chlorhydrate de 5-éthoxy-1-[4-(3-diméthylaminopropoxy)-3-méthoxybenzène sulfonyl]-3-spirocyclohexaneindol-2-one.

A) 5-éthoxy-1-[4-(3-bromopropoxy)-3-méthoxybenzènesulfonyl]-3-spiroxyclohexane indol-2-one.

On laisse sous agitation à 20°C pendant 15 minutes un mélange contenant 0,5 g de 5-éthoxy-3-spirocyclohexaneindol-2-one, 5 ml de THF, 0,07 g d'hydrure de. sodium puis on ajoute 1,65 g de chlorure de 4-(3-bromopropoxy)-3-méthoxybenzènesulfonyl et on laisse sous agitation 20 heures à TA. On concentre sous vide, extrait à l'éther, lave à l'eau puis par une solution de carbonate de sodium à 10 %. Le produit attendu cristallise dans le pentane puis est recristallisé dans l'éther iso.
Fc = 114-118°C

B) Chlorhydrate de 5-éthoxy-1-[4-(3-diméthylaminopropoxy)-3-méthoxybenzène sulfonyl]-3-spirocyclohexaneindol-2-one.

Le composé obtenu à l'étape précédente est mélangé aved 7,5 g de diméthylamine à 33 % dans l'éthanol et placé dans 10 ml de THF. Après 3 heures d'agitation, on concentre sous vide, reprend par 10 ml d'eau, extrait à l'éther. La phase éthérée est traitée par 20 ml d'acide chlorhydrique 2N puis on ajoute du carbonate de potassium solide pour alcaliniser la solution à pH 9. L'huile qui précipite est extraite par DCM. Le produit attendu cristallise dans l'éther.
Fc = 135-138°C

EXEMPLE 123

1-[(4-aminosulfonamido-2-méthoxybenzènesulfonyl]-5-chloro-3-spirocyclohexane indol-2-one.

0,3 g du composé préparé à l'exemple 2 sont placés dans 4 ml de DCM en présence de 0,5 g de TEA, on ajoute 0,3 g de chlorure d'aminosulfonyle préparé selon Chem. Ber., 1958, 91, 1339-1341. Après 2 jours à TA sous agitation, le milieu est concentré sous vide, extrait à l'éther, lavé à l'eau. Après séchage, le résidu est chromatographié sur silice en éluant par DCM puis AcOEt. On obtient le produit attendu qui cristallise dans l'éther.

Fc = 205-208°C

EXEMPLE 124 et 125

1-(4-Diméthylamino-2-méthoxybenzènesulfonyl)-5-méthoxy-3-spirocyclohexane indol-2-one.

et 1-(4-Méthylamino-2-méthoxybenzènesulfonyl)-5-méthoxy-3-spirocyclohexane indol-2-one.

On mélange 500 mg de 1-(4-amino-2-méthoxybenzènesulfonyl)-5-méthoxy-3-spirocyclohexane indol-2-one avec 1 ml de formaldehyde à 37 % dans l'eau, 10 ml d'acétonitrile et 430 mg de cyanoborohydrure de sodium puis on ajoute 0,12 ml d'acide acétique. La température du milieu s'élève, on refroidit dans un bain de glace. Deux produits de polarité différente se forment successivement. On ajoute dans le milieu 1 ml de formaldehyde en solution aqueuse, 300 mg de cyanoborohydrure de sodium et 0,12 ml d'acide acétique. On laisse sous agitation pendant 1 heure et demie, coule sur de l'eau glacée puis extrait par AcOEt. On lave à l'eau, sèche et concentre. On obtient 2 produits qui sont séparés par chromatographie sur silice en éluant par DCM-AcOEt (98.2, v/v).

Fc = 210°C (ex 124)

Fc = 170°C (ex 125)

TABLEAU 5

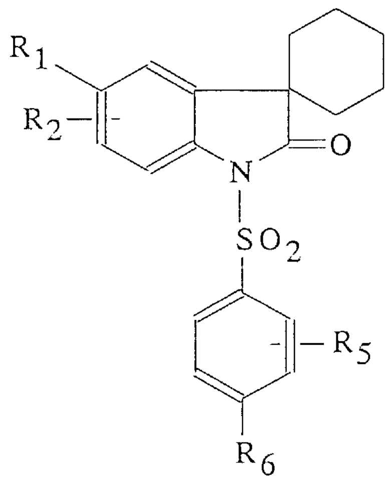

(I)

sauf indication contraire le substituant $R_6$ est en position 4 et m = 1.

| Ex | $R_1$ | $R_2$ | $R_5$ | $R_6$ | Fc°C |
|---|---|---|---|---|---|
| 126 | Cl- | H | MeO-2 | 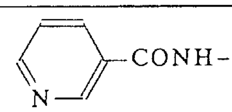 | 210 |
| 127 | Cl- | H | MeO-2 | 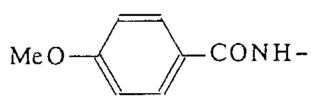 | 192 |
| 128 | Cl- | H | MeO-2 | 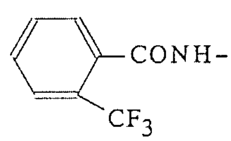 | 188 |
| 129 | Cl- | H | MeO-2 | 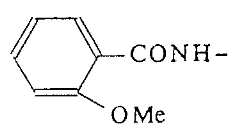 | 146 |

| | | | | | |
|---|---|---|---|---|---|
| 130 | Cl– | H | MeO–2 | 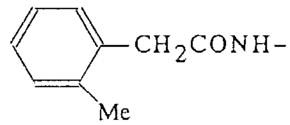 | 190 |
| 131 | Cl– | H | MeO–2 | 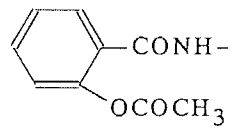 | 147 |
| 132 | Cl– | H | MeO–2 | $CH_3CONH-$ | 230 |
| 133 | Cl– | H | MeO–2 | 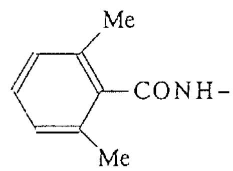 | 205 |
| 134 | Cl– | H | MeO–2 | $HO_2C(CH_2)_2-CONH-$ | 205 |
| 135 | Cl– | H | H | 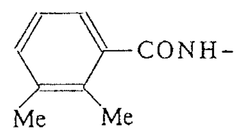 | 180 |
| 136 | Cl– | H | MeO–2 | 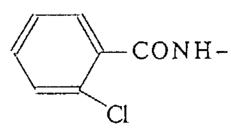 | 189 |
| 137 | Cl– | H | MeO–2 | 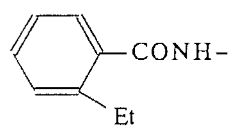 | 176 |
| 138 | MeO– | H | MeO–2 | 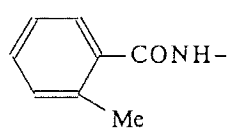 | 245 |

| | | | | | |
|---|---|---|---|---|---|
| 139 | MeO– | H | MeO–2 | 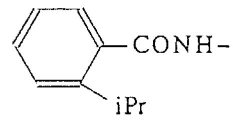 | 194 |
| 140 | Cl– | H | MeO–2 | 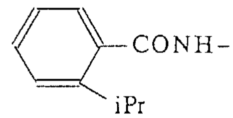 | 141 |
| 141 | Cl– | H | MeO–2 | 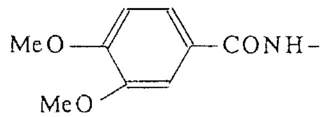 | 140 |
| 142 | Cl– | H | MeO–2 | 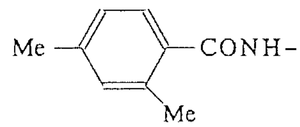 | 225 |
| 143 | MeO– | H | MeO–2 | $MeOCH_2CONH–$ | 161 |
| 144 | MeO– | H | MeO–2 | $tBuCH_2CONH–$ | 209 |
| 145 | EtO– | H | MeO–2 | 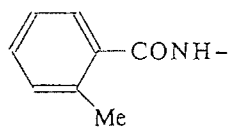 | 223 |
| 146 | EtO– | H | MeO–2 | 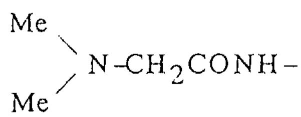 | 136 |
| 147 | Cl– | H | MeO–2 | 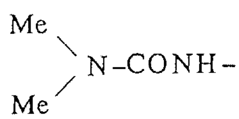 | 226 |
| 148 | $CH_3O$ | H | MeO–2 | 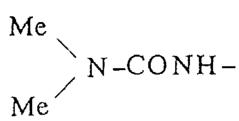 | 190 |

| | | | | | |
|---|---|---|---|---|---|
| 149 | EtO- | H | MeO-2 | 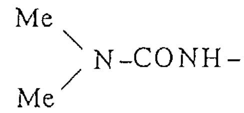 | 192 |
| 150 | EtO- | H | MeO-2 | 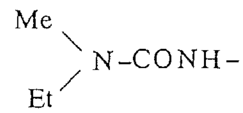 | 160 |
| 151 | EtO- | H | MeO-2 | 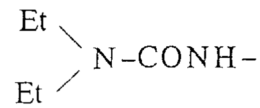 | 168 |
| 152 | EtO- | H | MeO-2 | 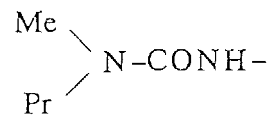 | 137 |
| 153 | Cl- | H | MeO-2 | 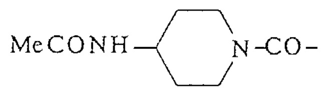 | 157 |
| 154 | Cl- | H | MeO-2 | 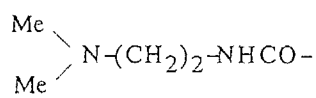 | 163 |
| 155 | Cl- | H | MeO-2 | 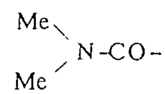 | 192 |
| 156 | Cl- | H | MeO-2 | 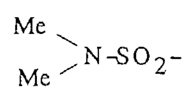 | 231 |
| 157 | Cl- | H | MeO-2 | H | 106 |
| 158 | Cl- | H | MeO-2 | 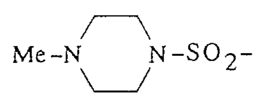 | 226 |
| 159 | Cl- | H | MeO-2 | 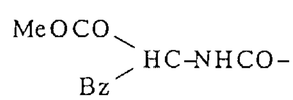 | 117 |

| | | | | | |
|---|---|---|---|---|---|
| 160 | MeO- | H | MeO-2 | $O_2N$- | 188 |
| 161 | Cl- | H | MeO-2 | BzOCO- | RMN |
| 162 | Cl- | H | MeO-2 | 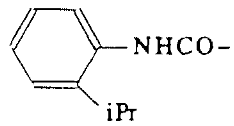 | 215 |
| 163 | MeO- | H | MeO-2 | $NH_2$- | 188 |
| 164 | MeO- | H | MeO-2 | MeO- | 172 |
| 165 | MeO- | H | MeO-2 | 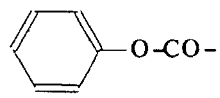 | 162 |
| 166 | MeO- | H | MeO-2 | 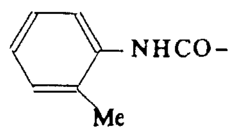 | 198 |
| 167 | EtO- | H | MeO-2 | $H_2N$- | 177 |
| 168 | MeO- | MeO-6 | MeO-2 | MeO- | 183 |
| 169 | EtO- | H | MeO-2 | 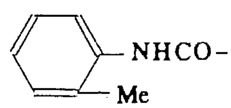 | 150 |
| 170 | EtO- | H | MeO-2 | BzOCO- | 135 |
| 171 | EtO- | H | MeO-2 | HOOC- | RMN |
| 172 | EtO- | H | MeO-2 | MeNHCO- | 239 |
| 173 | EtO- | H | MeO-2 | MeO-5 | 131 |

| | | | | | |
|---|---|---|---|---|---|
| 174 | EtO- | H | MeO-3 | MeO- | 127 |
| 175 | EtO- | H | MeO-2 | 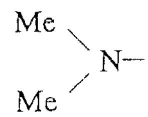 | 167 |
| 176 | EtO- | H | MeO-3 | di-MeO-4,5 | 130 |
| 177 | EtO- | H | MeO-2 | 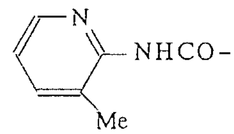 | 195 |
| 178 | EtO- | H | MeO-2 | 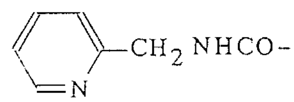 | 168 |
| 179 | EtO- | H | MeO-2 | $O_2N$- | 160 |
| 180 | EtO- | H | Me-2 | MeO- | 176 |
| 181 | EtO- | H | MeO-3 | $CH_2$=CH-$CH_2$O- | 130 |
| 182 | $CF_3$O- | H | MeO-2 | MeO- | 127 |
| 183 | EtO- | H | MeO-2 | 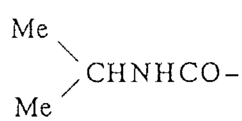 | 171 |
| 184 | EtO- | H | MeO-2 | EtOCOC$H_2$NHCO- | 203 |
| 185 | EtO- | H | MeO-2 | 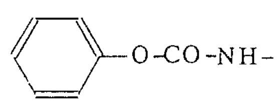 | 181 |
| 186 | EtO- | H | MeO-2 | di-MeO-4,5 | 136 |

| | | | | | |
|---|---|---|---|---|---|
| 187 | EtO– | H | Me–2 | MeO–4, Cl–5 | 129 |
| 188 | EtO– | H | MeO–2 | 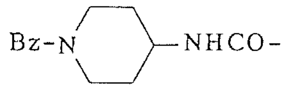 | 188 |
| 189 | EtO | H | MeO–2 | $HO(CH_2)_2$–NHCO– | 157 |
| 190 | EtO– | H | MeOCO–2 | H | 117 |
| 191 | EtO– | H | MeO–2 | 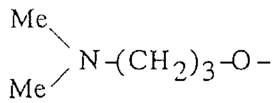 , HCl | 212 |
| 192 | EtO– | H | MeO–2 | 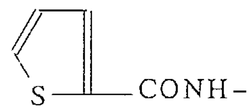 | 181 |
| 193 | EtO– | H | MeO– | 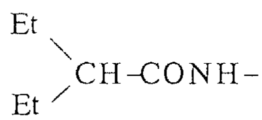 | 206 |
| 194 | EtO– | H | MeO–2 | $BzOCOCH_2NHCO$– | RMN |
| 195 | EtO– | H | MeO–2 | 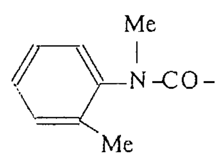 | 144 |
| 196 | EtO– | H | MeO–2 | 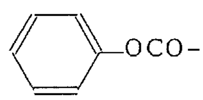 | 152 |
| 197 | EtO– | H | MeO–2 | 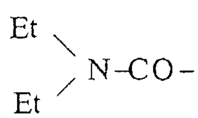 | 148 |

| | | | | | |
|---|---|---|---|---|---|
| 198 | EtO– | H | MeO–2 | 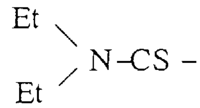 | 128 |
| 199 | EtO– | H | MeO–2 | $CN-CH_2NH-CO-$ | 232 |
| 200 | EtO– | H | MeO–2 | 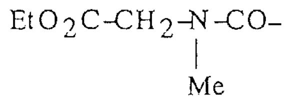 | RMN |
| 201 | EtO– | H | MeO–2 | $HO_2C-CH_2NH-CO-$ | 137 |
| 202 | Cl– | H | MeO–2 | $(Et)_2N-CO-NH-$ | 194 |
| 203 | EtO– | H | MeO–2 | 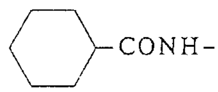 | 214 |
| 204 | EtO– | H | MeO–2 | $H_2N(CH_2)_3O-$ | 136–140 |
| 205 | EtO– | H | MeO–2 | $(CH_3)_3\overset{+}{N}(CH_2)_3O-$ $I-$ | 145–150 |
| 206 | $C_6H_5O$ – | H | MeO–2 | MeO–4 | 130 |
| 207 | EtO– | H | MeO–2 | 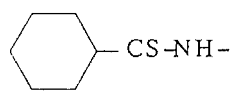 | 210 |
| 208 | EtO– | H | MeO–2 | 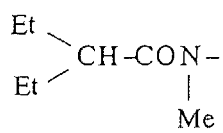 | 138 |
| 209 | EtO– | H | MeO–2 | $EtO-CO-CH_2O-$ | 160 |

Les spectres de RMN sont enregistrés dans le DMSO à 200 MHz.

**RMN** de l'exemple 161 :.

1,3-1,8 ppm : 10 H : cyclohexyle
3,5 ppm : 3 H : $OCH_3$
5,3 ppm : 2 H : O-C<u>H</u>$_2$-$C_6H_5$-

7,2-8,2 ppm : 11 H : aromatiques.

**RMN** de l'exemple 171 :

1,15 ppm : 3 H : $CH_3$
1,19-2 ppm : 10 H : cyclohexyle
3,6 ppm : 3 H : $OCH_3$
4 ppm : 2 H : $OC\underline{H}_2$-$CH_3$
6,7-8,2 ppm : 6 H : aromatiques

**RMN** de l'exemple 200 :

1-2,2 ppm : 16 H : cyclohexyle + $2CH_3$
3 ppm : 3 H : N $C\underline{H}_3$
4-4,4 ppm : 6 H : aromatiques
6,8-8,2 ppm : 6 H aromatiques

On observe un dédoublement des signaux lié à l'isomérie d'amide.

EXEMPLE 210

1 -(4-benzyloxy-2-méthoxybenzènesulfonyl)-5-éthoxy-3-spirocyclohexane indol-2-one.

A) 4-benzyloxy-2-méthoxybenzènesulfonate de potassium.

Cette préparation est effectuée selon K. Hofmann et al., Liebigs. Ann. Chem., 1982, 282-297.

On mélange à 5°C 10,5 g de 4-benzyloxy-2-méthoxybenzène dans 30 ml de DCM et on ajoute en 15 minutes, à une température comprise entre 5 et 10°C, 8 ml de chlorosulfonate de triméthylsilyle dans 30 ml de DCM ; après 15 minutes d'agitation, on ajoute 50 g de glace. On lave à l'éther éthylique, traite par du carbonate acide de potassium puis concentre sous vide. Après séchage, on reprend par 150 ml de méthanol. On filtre l'insoluble à ébullition puis le composé attendu cristallise à 5°C.

Fc > 300°C.

La structure du composé est confirmée par l'analyse du spectre RMN.

B) Chlorure de 4-benzyloxy-2-méthoxybenzènesulfonyle.

On mélange 2,8 g du composé préparé à l'exemple précédent dans 30 ml de $POCl_3$ et on porte à reflux pendant 3 heures. On concentre sous vide, traite par 20 g de glace, extrait à l'éther éthylique, lave par 30 ml de soude N puis par de l'eau. On concentre le milieu puis on triture l'huile obtenue dans 30 ml d'éther iso. Le produit attendu (0,7 g) cristallise .

Fc = 95°C.

C) 1-(4-benzyloxy-2-méthoxybenzènesulfonyl)-5-éthoxy-3-spirocyclo hexaneindol-2-one.

Ce composé est préparé selon le mode opératoire habituel. Il cristallise dans l'éther iso.

Fc = 135°C.

La structure du composé est vérifiée par l'analyse du spectre RMN en 2 dimensions. (effet NOESY : Nuclear Overhauser Effect Spectroscopy).

Par débenzylation, on prépare ensuite le composé de l'exemple suivant.

EXEMPLE 211

5-éthoxy-1-(4-hydroxy-2-méthoxybenzènesulfonyl)-3-spirocyclohexane indol-2-one.

Fc = 209°C.

## Revendications

1. Un composé de formule:

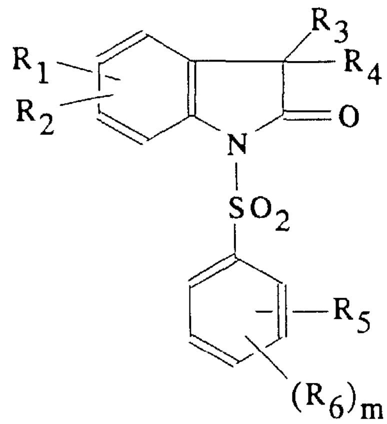

(I)

dans laquelle

- $R_1$ et $R_2$ représentent chacun indépendamment un hydrogène, un hydroxyle, un ω-halogénoalcoxy en $C_1$-$C_4$, un halogène, un alkyle en $C_1$-$C_4$, un trifluorométhyle, un alcoxy en $C_1$-$C_7$, un polyhalogénoalcoxy en $C_1$-$C_4$, un ω-hydroxyalcoxy en $C_2$-$C_4$, un ω-méthoxyalcoxy dans lequel l'alkyle est en $C_2$-$C_4$, un ω-aminoalcoxy en $C_2$-$C_4$ libre ou substitué par un ou deux alkyles en $C_1$-$C_4$ ; un cycloalkyloxy en $C_3$-$C_7$ ; un cycloalkylméthoxy dans lequel le cycloalkyle est en $C_3$-$C_7$ ; un phénoxy ; un benzyloxy ; un alkylthio en $C_1$-$C_4$ ; un phénylthio ; un nitro ; un amino libre ou substitué par un ou deux alkyles en $C_1$-$C_4$ ; un cyano ; un acyle en $C_1$-$C_4$ ; un acyloxy en $C_1$-$C_4$ ; un alkylsulfonamido en $C_1$-$C_4$ ; un phénylsulfonamido ; un alkylamido en $C_1$-$C_4$ ; un alcoxycarbonylamino en $C_1$-$C_4$ ; un ureido non substitué ou substitué par un phényle ou par un ou deux alkyles en $C_1$-$C_4$ ;
- $R_3$ et $R_4$ représentent chacun indépendamment un alkyle en $C_1$-$C_6$, un cycloalkyle en $C_3$-$C_7$, un phényle, un benzyle, un cycloalkylméthyle dans lequel le cycloalkyle est en $C_3$-$C_7$ ;
  ou
- $R_3$ et $R_4$ ensemble constituent un groupe $-(CH_2)_pX(CH_2)_q-$ ;
  ou
- $R_3$ et $R_4$ ensemble avec le carbone auquel ils sont liés constituent un cycle hydrocarboné en $C_3$-$C_{10}$, saturé ou insaturé, éventuellement condensé, non substitué ou substitué par un ou plusieurs groupes alkyles en $C_1$-$C_7$ ou par un spirocycloalkyle en $C_3$-$C_5$ ;
  ou encore
- $R_1$ et $R_4$ représentent chacun l'une des valeurs indiquées ci-dessus et $R_2$ est situé en position 4 de l'indole et constitue avec $R_3$ un groupement $(CH_2)_3$ ;
- $R_5$ et $R_6$ représentent chacun indépendamment un hydrogène, un halogène, un alkyle en $C_1$-$C_7$, un trifluorométhyle, un cyano, un nitro, un amino libre ou substitué par un ou deux alkyles en $C_1$-$C_7$ ; un hydroxylamino ; un hydroxy ; un carboxy ; un groupe $OR_7$ ; un groupe $SR_7$ ; un acyle en $C_1$-$C_7$ ; un alcoxycarbonyle en $C_1$-$C_7$ ; un phénoxycarbonyle ; un benzyloxycarbonyle ; un carbamoyle substitué par des groupes $R'_6$ et $R''_6$ ; un thiocarbamoyle libre ou substitué par un ou deux alkyles en $C_1$-$C_7$ ; un sulfamoyle ; un alkylsulfamoyle ou dialkylsulfamoyle dans lequel l'alkyle est en $C_1$-$C_7$ ; un groupe $SO_2R'_7$ ; un alkylsulfonamido dans lequel l'alkyle est en $C_1$-$C_7$ ; un groupe $COR'_7$ ; un groupe $NR_8R_9$ ; un groupe $CO-NH-CH(R_{10})-COR_{12}$; le cas échéant, le groupe phényle constitutif du substituant $R_5$ et/ou $R_6$, peut être non substitué ou substitué une ou plusieurs fois par un alkyle en $C_1$-$C_7$, un trifluorométhyle, un méthoxy, un halogène, un sulfamoyle, un alkylsulfamoyle dans lequel l'alkyle est en $C_1$-$C_7$, un carboxy, un alcoxycarbonyle dans lequel l'alkyle est en $C_1$-$C_7$, un acyloxy en $C_1$-$C_7$, un imidazolyle ;
- $R'_6$ et $R''_6$ représentent chacun indépendamment l'hydrogène, un alkyle en $C_1$-$C_7$ non substitué ou substitué par $R'''_6$, un phényle, un pyridyle, un méthylpyridyle, un pipéridin-4-yle, un méthylpipéridin-4-yle ;
- $R'''_6$ représente un hydroxyle, un cyano, un carboxy libre ou estérifié par un alkyle en $C_1$-$C_7$ ou par un benzyle ; un phényle ; un pyridyle ; un méthylpyridyle; un amino libre ou substitué par un ou deux alkyles en $C_1$-$C_7$;
- $R_7$ représente un alkyle en $C_1$-$C_7$, un phényle, un benzyle, un cycloalkyle en $C_3$-$C_7$, un alcényle en $C_2$-$C_4$, un

ω-halogénoalkyle en $C_1$-$C_7$, un polyhalogénoalkyle en $C_1$-$C_7$, un acyle en $C_1$-$C_7$, un ω-carboxyalkyle en $C_1$-$C_7$ libre ou estérifié par un alkyle en $C_1$-$C_4$ ou par un benzyle, un ω-aminoalkyle en $C_2$-$C_7$ dans lequel le groupe amino est libre, substitué par un ou deux alkyles en $C_1$-$C_4$ ou sous forme d'ion ammonium ;

- $R'_7$ représente un groupe pipérazin-1-yl non substitué ou substitué en 4 par un groupe $R''_7$, un groupe pipéridino non substitué ou substitué en 4 par un groupe $R'''_7$, un groupe azétidin-1-yl non substitué ou substitué en 3 par un groupe $R'''_7$, un groupe pyridyle non substitué ou substitué par un méthyle ;
- $R''_7$ représente un alkyle en $C_1$-$C_4$, un phényle, un benzyle, un acyle en $C_1$-$C_4$ ;
- $R'''_7$ représente $R''_7$ ou un amino libre ou portant un groupe protecteur ; -$R_8$ et $R_9$ représentent chacun indépendamment un hydrogène, un alkyle en $C_1$-$C_7$, un phényle, un benzyle, $R_9$ peut de plus représenter un acyle en $C_1$-$C_7$, un thioacyle en $C_1$-$C_7$, un cycloalkylcarbonyle dans lequel le cycloalkyle est en $C_3$-$C_7$, un cycloalkylthiocarbonyle dans lequel le cycloalkyle est en $C_3$-$C_7$, un ω-aminoacyle en $C_1$-$C_4$, un ω-hydroxyacyle en $C_1$-$C_4$, un ω-benzyloxyacyle en $C_1$-$C_4$, un phénoxycarbonyle, un thiénocarbonyle, un pyridylcarbonyle, un méthylpyridylcarbonyle, un alcoxycarbonyle en $C_1$-$C_4$, un benzoyle, un groupe $CO$-$CH(R_{10})$-$NR_{11}R'_{11}$; un groupe $CH(R_{10})CO_2R_{11}$, un groupe $(CH_2)_tCOR_{12}$, un groupe $CO(CH_2)_tCOR_{12}$, un carbamoyle non substitué ou substitué par un phényle ou par un ou deux alkyles en $C_1$-$C_4$ ;
- m est 1 ou, lorsque $R_6$ est un halogène, un alkyle en $C_1$-$C_7$ ou un alcoxy en $C_1$-$C_7$, m peut également être 2, 3 ou 4 ou bien $(R_6)_m$ peut représenter m substituants ayant différentes valeurs choisies parmi halogène, alkyle en $C_1$-$C_7$ ou alcoxy en $C_1$-$C_7$ ;
- p et q représentent chacun un nombre entier, leur somme peut varier de 3 à 6 ;
- t représente un nombre entier qui peut varier de 1 à 5 ;
- X représente l'oxygène, le soufre ou un groupe $NR_{13}$ ;
- $R_{10}$ représente l'hydrogène, un alkyle en $C_1$-$C_4$ ou un benzyle ;
- $R_{11}$ et $R'_{11}$ représentent chacun indépendamment l'hydrogène ou un alkyle en $C_1$-$C_4$ ;
- $R_{12}$ représente un hydroxy, un alcoxy en $C_1$-$C_4$, un amino non substitué ou substitué par un ou deux alkyles en $C_1$-$C_4$ ;
- $R_{13}$ représente l'hydrogéne, un alkyle en $C_1$-$C_4$, un phényle, un benzyle, un acyle en $C_1$-$C_4$, un alcoxycarbonyle en $C_1$-$C_4$, un carbamoyle non substitué ou substitué par un ou 2 alkyles en $C_1$-$C_4$ ;

sous réserve que lorsque $R_3$ représente un méthyle, $R_4$ représente un méthyle ou un éthyle, $R_5$ représente l'hydrogène, m est 1 et $R_6$ représente un méthyle en position 4, alors $R_1$ et $R_2$ ne représentent pas simultanément l'hydrogène ; ainsi que ses sels éventuels.

2. Un composé selon la revendication 1 de formule (I) dans lequel $R_1$ est un atome de chlore ou un groupe éthoxy en position 5 de l'indole et $R_2$ est l'hydrogène.

3. Un composé selon la revendication 1 de formule (I) dans lequel $R_3$ et $R_4$ ensemble avec le carbone auquel ils sont liés constituent un cycle hydrocarboné en $C_3$-$C_{10}$.

4. Un composé selon la revendication 1 de formule (I) dans lequel $R_3$ et $R_4$ ensemble avec le carbone auquel ils sont liés constituent un cyclohexane non substitué ou substitué par un ou deux groupes alkyles en $C_1$-$C_7$ ou par un spirocycloalkyle en $C_3$-$C_5$, un cycloheptane, un adamantane ou un tricyclo [5.2.1.0.2,6]dec-8-ène.

5. Un composé de formule (I) dans lequel $R_3$ et $R_4$ ensemble avec le carbone auquel ils sont liés constituent un cycle pipéridine-4 ou N-méthylpipéridine-4.

6. Un composé selon la revendication 1 de formule (I) dans lequel $R_5$ et $R_6$ représentent chacun un méthoxy.

7. Un composé selon la revendication 1 de formule (I) dans lequel $R_5$ en position 2 représente un méthoxy et $R_6$ en position 4 représente un acylamino en $C_1$-$C_7$, un dialkylureido en $C_1$-$C_4$, un alcoxycarbonylalkylcarbamoyle dans lequel les groupes alkyles sont en $C_1$-$C_7$.

8. Un composé selon la revendication 1 de formule (I) dans lequel $R_1$ est en position 5 et $R_2$ est l'hydrogène.

9. Un composé selon la revendication 1 de formule :

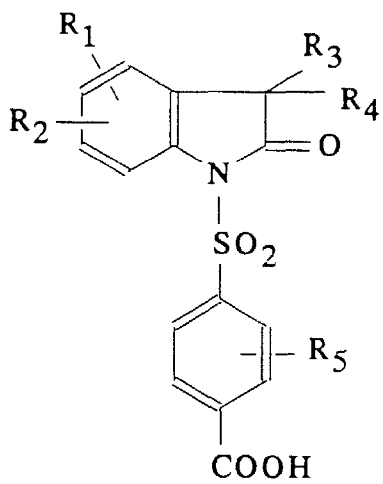

(IX)

dans laquelle $R_1$, $R_2$, $R_3$, $R_4$ et $R_5$ ont les valeurs indiquées ci-dessus pour (I) dans la revendication 1, et ses dérivés fonctionnels.

**10.** Un composé selon la revendication 1 de formule :

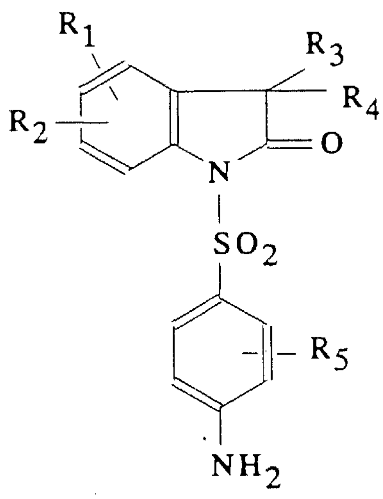

(X)

dans laquelle $R_1$, $R_2$, $R_3$, $R_4$ et $R_5$ ont les valeurs indiquées ci-dessus pour (I) dans la revendication 1, et ses sels éventuels.

**11.** Un composé selon la revendication 1 de formule :

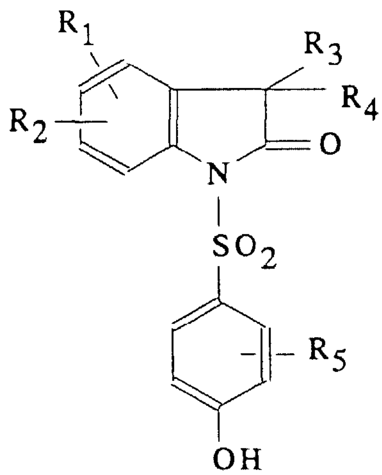

(XI)

dans laquelle $R_1$, $R_2$, $R_3$, $R_4$ et $R_5$ ont les valeurs indiquées ci-dessus pour (I) dans la revendication 1.

**12.** Un composé selon la revendication 1 de formule :

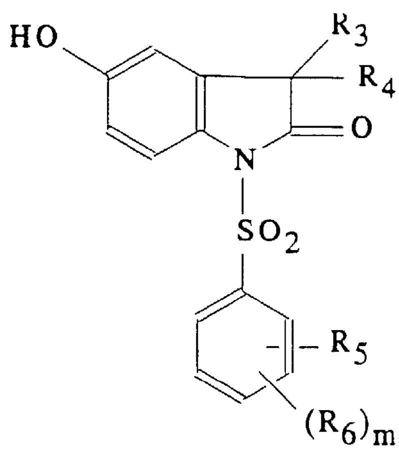

(XII)

dans laquelle $R_3$, $R_4$, $R_5$, $R_6$ et m ont les valeurs indiquées ci-dessus pour (I) dans la revendication 1.

**13.** Un composé selon la revendication 1 de formule :

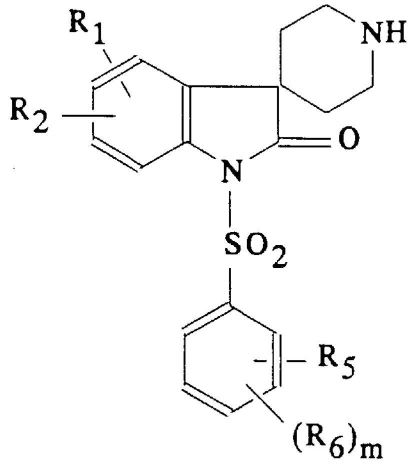

(XIII)

dans laquelle $R_1$, $R_2$, $R_5$, $R_6$ et m ont les valeurs indiquées ci-dessus pour (I) dans la revendication 1.

**14.** Un procédé de préparation d'un composé (I) selon la revendication 1 caractérisé en ce que :
on fait agir sur un oxo-2 indole disubstitué en position 3, de formule :

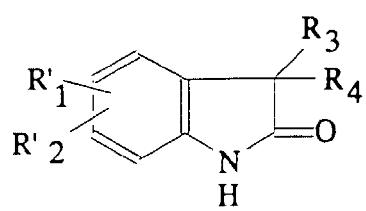

(II)

dans laquelle $R'_1$ et $R'_2$ représentent, respectivement, soit $R_1$, $R_2$ tels que définis pour (I), soit des groupes précurseurs de $R_1$, $R_2$ , et $R_3$, $R_4$ sont tels que définis ci-dessus pour (I), un halogénure de benzènesulfonyle de formule :

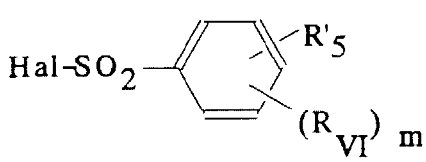

(III)

dans laquelle $R'_5$ et $R_{VI}$ représentent, respectivement, soit $R_5$ et $R_6$ tels que définis ci-dessus pour (I), soit des groupes précurseurs de $R_5$ et $R_6$ ; et,

- soit, lorsque $R'_1 = R_1$ ; $R'_2 = R_2$, $R'_5 = R_5$ et $R_{VI} = R_6$, on isole le composé de formule (I) ainsi obtenu ;
- soit, lorsque l'un quelconque des groupes $R'_1$, $R'_2$, $R'_5$ et $R_{VI}$ présente respectivement un groupement précurseur de $R_1$, $R_2$, $R_5$ et/ou $R_6$, on soumet le composé obtenu à un traitement ultérieur pour préparer le composé de formule (I) par transformation de l'un quelconque des groupes $R'_1$, $R'_2$, $R'_5$ et $R_{VI}$ en, respectivement, $R_1$, $R_2$, $R_5$ et $R_6$.

**15.** Un composé de formule :

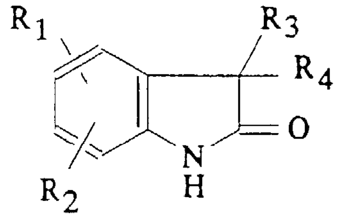

(II)'

dans laquelle :

- $R_1$ et $R_2$ représentent chacun indépendamment un hydrogène, un hydroxyle, un ω-halogénoalcoxy en $C_1$-$C_4$, un halogène, un alkyle en $C_1$-$C_4$, un trifluorométhyle, un alcoxy en $C_1$-$C_7$, un polyhalogénoalcoxy en $C_1$-$C_4$, un ω-hydroxyalcoxy en $C_2$-$C_4$, un ω-méthoxyalcoxy dans lequel l'alkyle est en $C_2$-$C_4$, un ω-aminoalcoxy en $C_2$-$C_4$ libre ou substitué par un ou deux alkyles en $C_1$-$C_4$ ; un cycloalkyloxy en $C_3$-$C_7$ ; un cycloalkylméthoxy dans lequel le cycloalkyle est en $C_3$-$C_7$ ; un phénoxy ; un benzyloxy ; un alkylthio en $C_1$-$C_4$ ; un phénylthio ; un nitro ; un amino libre ou substitué par un ou deux alkyles en $C_1$-$C_4$ ; un cyano ; un acyle en $C_1$-$C_4$ ; un acyloxy en $C_1$-$C_4$ ; un alkylsulfonamido en $C_1$-$C_4$ ; un phénylsulfonamido ; un alkylamido en $C_1$-$C_4$ ; un alcoxycarbonylamino en $C_1$-$C_4$ ; un ureido non substitué ou substitué par un phényle ou par un ou deux alkyles en $C_1$-$C_4$ ;
- $R_3$ et $R_4$ ensemble avec le carbone auquel ils sont liés constituent

  . un adamantane,
  . un indane ou un hexahydroindane non substitués ou substitués par un ou plusieurs groupes alkyles en $C_1$-$C_7$,
  . un tricyclo [5.2.1.0.2,6]décane ou un tricyclo [5.2.1.0.2,6]déc-8-ène non substitués ou substitués par un ou plusieurs groupes alkyles en $C_1$-$C_7$,

  ou

  un cycle hydrocarboné en $C_4$-$C_8$ substitué par un ou plusieurs groupes alkyles en $C_1$-$C_7$ ou par un spirocycloalkyle en $C_3$-$C_5$,

  ou bien $R_3$ et $R_4$ ensemble constituent un groupe $-(CH_2)_p-X(CH_2)_q-$ dans lequel p et q sont des nombres entiers dont la somme peut varier de 3 à 6 et X représente l'oxygène, le soufre ou un groupe $NR_{13}$, $R_{13}$ étant un phényle, un benzyle, un acyle en $C_1$-$C_4$, un alcoxycarbonyle en $C_1$-$C_4$, un carbamoyle non substitué ou substitué par un ou deux alkyles en $C_1$-$C_4$,

  avec la limitation que lorsque $CR_3R_4$ représente l'adamantane, $R_1$ et $R_2$ sont différents de l'hydrogène.

**16.** Un composé de formule :

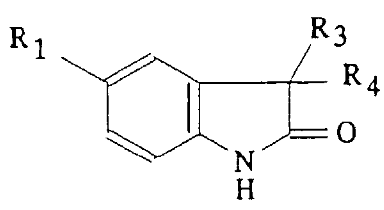

(II)"

dans laquelle

- $R_1$ représente un hydroxyle, un ω-halogénoalcoxy en $C_1$-$C_4$, un halogène, un alkyle en $C_1$-$C_4$, un trifluorométhyle, un alcoxy en $C_1$-$C_7$, un polyhalogénoalcoxy en $C_1$-$C_4$, un ω-hydroxyalcoxy en $C_2$-$C_4$, un ω-méthoxyalcoxy dans lequel l'alkyle est en $C_2$-$C_4$, un-ω-aminoalcoxy en $C_2$-$C_4$ libre ou substitué par un ou deux alkyles en $C_1$-$C_4$ ; un cycloalkyloxy en $C_3$-$C_7$ ; un cycloalkylméthoxy dans lequel le cycloalkyle est en $C_3$-$C_7$; un phénoxy ; un benzyloxy ; un alkylthio en $C_1$-$C_4$ ; un phénylthio ; un nitro ; un amino libre ou substitué par un

ou deux alkyles en $C_1$-$C_4$ ; un cyano ; un acyle en $C_1$-$C_4$ ; un acyloxy en $C_1$-$C_4$ ; un alkylsulfonamido en $C_1$-$C_4$ ; un phénylsulfonamido ; un alkylamido en $C_1$-$C_4$ ; un alcoxycarbonylamino en $C_1$-$C_4$ ; un uréido non substitué ou substitué par un phényle ou par un ou deux alkyles en $C_1$-$C_4$ ;

- $R_3$ et $R_4$ ensemble constituent un groupe $-(CH_2)_pX(CH_2)_q-$ ;
  ou
- $R_3$ et $R_4$ ensemble avec le carbone auquel ils sont liés constituent un cycle hydrocarboné en $C_3$-$C_{10}$, saturé ou insaturé, éventuellement condensé, non substitué ou substitué par un ou plusieurs groupes alkyles en $C_1$-$C_7$ ou par un spirocycloalkyle en $C_3$-$C_5$ ;
- p et q représentent chacun un nombre entier, leur somme peut varier de 3 à 6;
- X représente l'oxygène, le soufre ou un groupe $NR_{13}$ ;
- $R_{13}$ représente l'hydrogène, un alkyle en $C_1$-$C_4$, un phényle, un benzyle, un acyle en $C_1$-$C_4$, un alcoxycarbonyle en $C_1$-$C_4$, un carbamoyle non substitué ou substitué par un ou 2 alkyles en $C_1$-$C_4$ ;

sous réserve que

- lorsque $R_1$ est méthoxy, $CR_3R_4$ est différent d'une pyrrolidine-3, non substituée ou N-substituée par un alkyle en $C_1$-$C_4$,
- lorsque $R_1$ représente un halogène ou un nitro, $R_3$ et $R_4$ ensemble avec l'atome de carbone auquel ils sont liés soient différents d'un cycle hydrocarboné en $C_3$-$C_{10}$, saturé ou insaturé, et
- lorsque $R_1$ représente un groupe propionyle, $R_3$ et $R_4$ ensemble avec l'atome de carbone auquel ils sont liés ne constituent pas un cyclopropane.

**17.** Un composé selon la revendication 16 dans lequel $R_1$ est éthoxy.

**18.** Un composé de formule

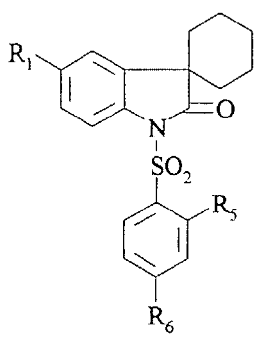

dans laquelle

- $R_1$ est un atome de chlore
- $R_5$ est un groupe méthoxy, et
- $R_6$ est un groupe

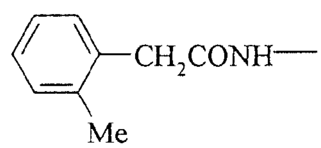

**19.** Un composé de formule

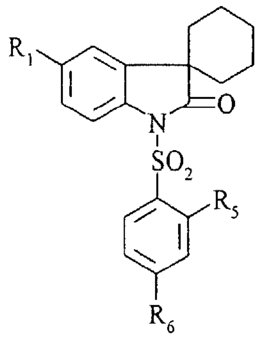

dans laquelle

- $R_1$ est un groupe méthoxy,
- $R_5$ est un groupe méthoxy, et
- $R_6$ est un groupe $MeOCH_2CONH$-.

**20.** Un composé de formule

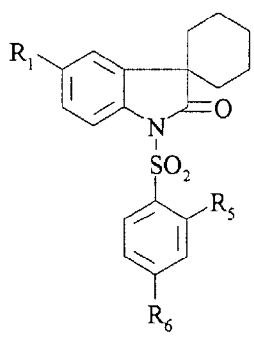

dans laquelle

- $R_1$ est un groupe éthoxy,
- $R_5$ est un groupe méthoxy, et
- $R_6$ est un groupe

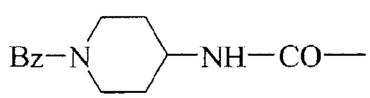

**21.** Composition pharmaceutique contenant à titre de principe actif un composé selon l'une quelconque des revendications 1 à 8 et 18 à 20.

**22.** Composition pharmaceutique contenant un composé selon l'une quelconque des revendications 1 à 8 et 18 à 20 en association avec un autre principe actif.

**23.** Composition pharmaceutique selon la revendication 22 contenant un composé antagoniste des récepteurs V1 en association avec un antagoniste de l'angiotensine II.

**Patentansprüche**

1. Verbindung der Formel:

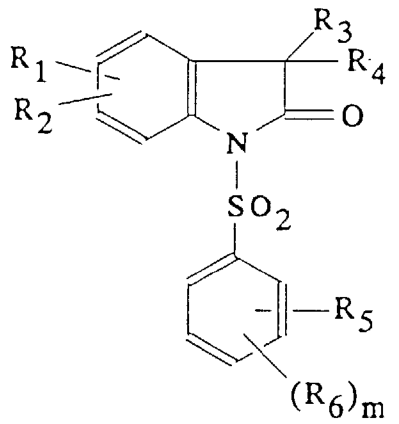

(I),

worin

- $R_1$ und $R_2$ jeweils unabhängig einen Wasserstoff, ein Hydroxyl, ein $C_1$-$C_4$-ω-Halogenalkoxy, ein Halogen, ein $C_1$-$C_4$-Alkyl, ein Trifluormethyl, ein $C_1$-$C_7$-Alkoxy, ein $C_1$-$C_4$-Polyhalogenalkoxy, ein $C_2$-$C_4$-ω-Hydroxyalkoxy, ein ω-Methoxyalkoxy, wobei das Alkyl $C_2$-$C_4$ ist, ein $C_2$-$C_4$-ω-Aminoalkoxy, frei oder substituiert durch ein oder zwei $C_1$-$C_4$-Alkyle; ein $C_3$-$C_7$-Cycloalkyloxy; ein Cycloalkylmethoxy, wobei das Cycloalkyl $C_3$-$C_7$ ist; ein Phenoxy; ein Benzyloxy; ein $C_1$-$C_4$-Alkylthio; ein Phenylthio; ein Nitro; ein Amino, frei oder substituiert durch ein oder zwei $C_1$-$C_4$-Alkyle; ein Cyano; ein $C_1$-$C_4$-Acyl; ein $C_1$-$C_4$-Acyloxy; ein $C_1$-$C_4$-Alkylsulfonamido; ein Phenylsulfonamido; ein $C_1$-$C_4$-Alkylamido; ein $C_1$-$C_4$-Alkoxycarbonylamino; ein Ureido, unsubstituiert oder substituiert durch ein Phenyl oder durch ein oder zwei $C_1$-$C_4$-Alkyle, bedeuten;
- $R_3$ und $R_4$ jeweils unabhängig ein $C_1$-$C_6$-Alkyl, ein $C_3$-$C_7$-Cycloalkyl, ein Phenyl, ein Benzyl, ein Cycloalkylmethyl, wobei das Cycloalkyl $C_3$-$C_7$ ist, darstellen;
  oder
- $R_3$ und $R_4$ gemeinsam eine Gruppe $-(CH_2)_pX(CH_2)_q-$ sind;
  oder
- $R_3$ und $R_4$ gemeinsam mit dem Kohlenstoff, an den sie gebunden sind, einen gesättigten oder ungesättigten, gegebenenfalls kondensierten $C_3$-$C_{10}$-Kohlenwasserstoff-Cyclus, unsubstituiert oder substituiert durch eine oder mehrere $C_1$-$C_7$-Alkyl-Gruppen oder durch ein $C_3$-$C_5$-Spirocycloalkyl, bilden;
  oder auch
- $R_1$ und $R_4$ jeweils einen der vorstehend angegebenen Werte darstellen, und $R_2$ in Stellung 4 des Indols vorliegt und mit $R_3$ eine Gruppe $(CH_2)_3$ bildet;
- $R_5$ und $R_6$ jeweils unabhängig einen Wasserstoff, ein Halogen, ein $C_1$-$C_7$-Alkyl, ein Trifluormethyl, ein Cyano, ein Nitro, ein Amino, frei oder substituiert durch ein oder zwei $C_1$-$C_7$-Alkyle; ein Hydroxylamino; ein Hydroxy; ein Carboxy; eine Gruppe $OR_7$; eine Gruppe $SR_7$; ein $C_1$-$C_7$-Acyl; ein $C_1$-$C_7$-Acyloxycarbonyl; ein Phenoxycarbonyl; ein Benzyloxycarbonyl; ein Carbamoyl, substituiert durch Gruppen $R'_6$ und $R''_6$; ein Thiocarbamoyl, frei oder substituiert durch ein oder zwei $C_1$-$C_7$-Alkyle; ein Sulfamoyl; ein Alkylsulfamoyl oder Dialkylsulfamoyl, wobei das Alkyl $C_1$-$C_7$ ist; eine Gruppe $SO_2R'_7$; ein Alkylsulfonamido, wobei das Alkyl $C_1$-$C_7$ ist; eine Gruppe $COR'_7$; eine Gruppe $NR_8R_9$; eine Gruppe $CO-NH-CH(R_{10})-COR_{12}$; wobei gegebenenfalls die den Substituenten $R_5$ und/oder $R_6$ bildende Phenyl-Gruppe unsubstituiert oder einfach oder mehrfach substituiert sein kann durch ein $C_1$-$C_7$-Alkyl, ein Trifluormethyl, ein Methoxy, ein Halogen, ein Sulfamoyl, ein Alkylsulfamoyl, wobei das Alkyl $C_1$-$C_7$ ist, ein Carboxy, ein Alkoxycarbonyl, wobei das Alkyl $C_1$-$C_7$ ist, ein $C_1$-$C_7$-Acyloxy, ein Imidazolyl, bedeuten;
- $R'_6$ und $R''_6$ jeweils unabhängig Wasserstoff, ein $C_1$-$C_7$-Alkyl, unsubstituiert oder substituiert durch $R'''_6$, ein Phenyl, ein Pyridyl, ein Methylpyridyl, ein Piperidin-4-yl, ein Methylpiperidin-4-yl darstellen;
- $R'''_6$ ein Hydroxyl, ein Cyano, ein Carboxy, frei oder verestert durch ein $C_1$-$C_7$-Alkyl oder durch ein Benzyl; ein Phenyl; ein Pyridyl; ein Methylpyridyl; ein Amino, frei oder substituiert durch ein oder zwei $C_1$-$C_7$-Alkyle, ist;
- $R_7$ ein $C_1$-$C_7$-Alkyl, ein Phenyl, ein Benzyl, ein $C_3$-$C_7$-Cycloalkyl, ein $C_2$-$C_4$-Acenyl, ein $C_1$-$C_7$-ω-Halogenalkyl, ein $C_1$-$C_7$-Polyhalogenalkyl, ein $C_1$-$C_7$-Acyl, ein $C_1$-$C_7$-ω-Carboxyalkyl, frei oder verestert durch ein $C_1$-$C_4$-

Alkyl oder durch ein Benzyl, ein $C_2$-$C_7$-ω-Aminoalkyl, wobei die Amino-Gruppe frei, durch ein oder zwei $C_1$-$C_4$-Alkyle substituiert oder in Ammoniumionen-Form vorliegt, bedeutet;

- $R'_7$ eine Gruppe Piperazin-1-yl, unsubstituiert oder substituiert in 4 durch eine Gruppe $R''_7$, eine Piperidino-Gruppe, unsubstituiert oder substituiert in 4 durch eine Gruppe $R'''_7$, eine Gruppe Azetidin-1-yl, unsubstituiert oder substituiert in 3 durch eine Gruppe $R'''_7$, eine Pyridyl-Gruppe, unsubstituiert oder substituiert durch ein Methyl, darstellt;
- $R''_7$ ein $C_1$-$C_4$-Alkyl, ein Phenyl, ein Benzyl, ein $C_1$-$C_4$-Acyl ist;
- $R'''_7$ $R''_7$ oder ein Amino, frei oder eine Schutzgruppe tragend, bedeutet;
- $R_8$ und $R_9$ jeweils unabhängig einen Wasserstoff, ein $C_1$-$C_7$-Alkyl, ein Phenyl, ein Benzyl darstellen, wobei $R_9$ außerdem ein $C_1$-$C_7$-Acyl, $C_1$-$C_7$-Thioacyl, ein Cycloalkylcarbonyl, worin das Cycloalkyl $C_3$-$C_7$ ist, ein Cycloalkylthiocarbonyl, worin das Cycloalkyl $C_3$-$C_7$ ist, ein $C_1$-$C_4$-ω-Aminoacyl, ein $C_1$-$C_4$-ω-Hydroxyacyl, ein $C_1$-$C_4$-ω-Benzyloxyacyl, ein Phenoxycarbonyl, ein Thienocarbonyl, ein Pyridylcarbonyl, ein Methylpyridylcarbonyl, ein $C_1$-$C_4$-Alkoxycarbonyl, ein Benzoyl, eine Gruppe CO-CH($R_{10}$)-$NR_{11}R'_{11}$; eine Gruppe CH($R_{10}$) $CO_2R_{11}$; eine Gruppe $(CH_2)_tCOR_{12}$; eine Gruppe $CO(CH_2)_tCOR_{12}$, ein Carbamoyl, unsubstituiert oder substituiert durch ein Phenyl oder durch ein oder zwei $C_1$-$C_4$-Alkyle, sein kann;
- m 1 ist, oder, wenn $R_6$ ein Halogen, ein $C_1$-$C_7$-Alkyl oder ein $C_1$-$C_7$-Alkoxy bedeutet, m auch 2, 3 oder 4 sein kann, oder auch $(R_6)_m$ m Substituenten mit verschiedenen Werten, ausgewält aus Halogen, $C_1$-$C_7$-Alkyl oder $C_1$-$C_7$-Alkoxy, bedeuten kann;
- p und q jeweils eine ganze Zahl sind, wobei ihre Summe von 3 bis 6 variieren kann;
- t eine ganze Zahl ist, die von 1 bis 5 variieren kann;
- X Sauerstoff, Schwefel oder eine Gruppe $NR_{13}$ darstellt;
- $R_{10}$ Wasserstoff, ein $C_1$-$C_4$-Alkyl oder ein Benzyl ist;
- $R_{11}$ und $R'_{11}$ jeweils unabhängig Wasserstoff oder ein $C_1$-$C_4$-Alkyl bedeuten;
- $R_{12}$ ein Hydroxy, ein $C_1$-$C_4$-Alkoxy, ein Amino, unsubstituiert oder substituiert durch ein oder zwei $C_1$-$C_4$-Alkyle, darstellt;
- $R_{13}$ Wasserstoff ein $C_1$-$C_4$-Alkyl, ein Phenyl, ein Benzyl, ein $C_1$-$C_4$-Acyl, ein $C_1$-$C_4$-Alkoxycarbonyl, ein Carbamoyl, unsubstituiert oder substituiert durch ein oder zwei $C_1$-$C_4$-Alkyle, ist;

mit der Maßgabe, daß, wenn $R_3$ ein Methyl bedeutet, $R_4$ ein Methyl oder ein Ethyl darstellt, $R_5$ Wasserstoff ist, m 1 ist, und $R_6$ ein Methyl in Stellung 4 bedeutet, $R_1$ und $R_2$ nicht gleichzeitig Wasserstoff darstellen;
und ihre gegebenenfalls vorliegenden Salze.

2. Verbindung nach Anspruch 1 der Formel (I), worin $R_1$ ein Chloratom oder eine Ethoxy-Gruppe in Stellung 5 des Indols bedeutet, und $R_2$ Wasserstoff darstellt.

3. Verbindung nach Anspruch 1 der Formel (I), worin $R_3$ und $R_4$ gemeinsam mit dem Kohlenstoff, an den sie gebunden sind, einen $C_3$-$C_{10}$-Kohlenwasserstoff-Cyclus bilden.

4. Verbindung nach Anspruch 1 der Formel (I), worin $R_3$ und $R_4$ gemeinsam mit dem Kohlenstoff, an den sie gebunden sind, ein Cyclohexan, unsubstituiert oder substituiert durch eine oder zwei $C_1$-$C_7$-Alkyl-Gruppen oder durch ein $C_3$-$C_5$-Spirocycloalkyl, ein Cycloheptan, ein Adamantan oder ein Tricyclo[5.2.1.0.2,6]dec-8-en, bilden.

5. Verbindung der Formel (I), worin $R_3$ und $R_4$ gemeinsam mit dem Kohlenstoff, an den sie gebunden sind, einen 4-Piperidin- oder N-4-Methylpiperidin-Cyclus bilden.

6. Verbindung nach Anspruch 1 der Formel (I), worin $R_5$ und $R_6$ jeweils ein Methoxy bedeuten.

7. Verbindung nach Anspruch 1 der Formel (I), worin $R_5$ in Stellung 2 ein Methoxy bedeutet, und $R_6$ in Stellung 4 ein $C_1$-$C_7$-Acylamino, ein $C_1$-$C_4$-Dialkylureido, ein Alkoxycarbonylalkylcarbamoyl, wobei die Alkyl-Gruppen $C_1$-$C_7$ sind, darstellt.

8. Verbindung nach Anspruch 1 der Formel (I), worin $R_1$ in Stellung 5 vorliegt, und $R_2$ Wasserstoff bedeutet.

9. Verbindung nach Anspruch 1 der Formel:

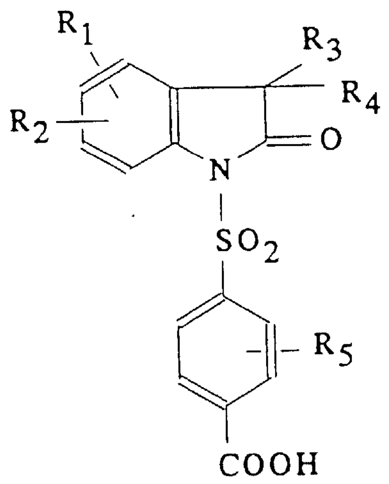

(IX),

worin

$R_1$, $R_2$, $R_3$, $R_4$ und $R_5$ die oben für (I) in Anspruch 1 angegebenen Werte haben,

und ihre funktionellen Derivate.

**10.** Verbindung nach Anspruch 1 der Formel:

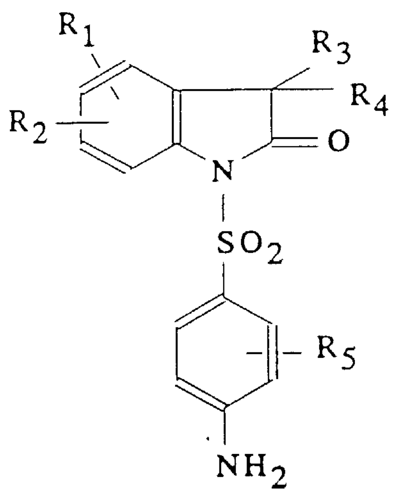

(X),

worin

$R_1$, $R_2$, $R_3$, $R_4$ und $R_5$ die oben für (I) in Anspruch 1 angegebenen Werte haben,

und ihre gegebenenfalls vorliegenden Salze.

**11.** Verbindung nach Anspruch 1 der Formel:

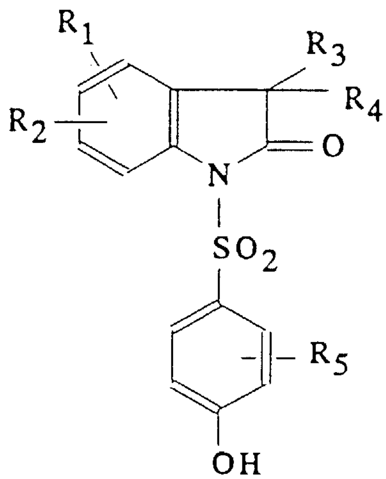

(XI),

worin

$R_1$, $R_2$, $R_3$, $R_4$ und $R_5$ die oben für (I) in Anspruch 1 angegebenen Werte haben.

**12.** Verbindung nach Anspruch 1 der Formel:

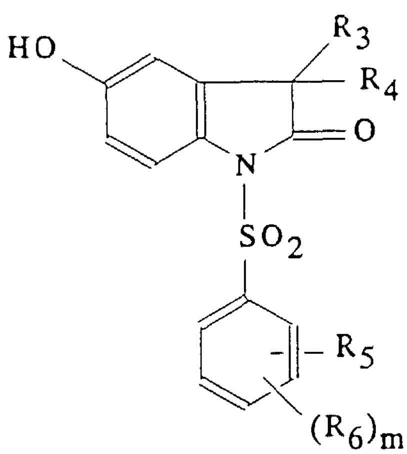

(XII),

worin

$R_3$, $R_4$, $R_5$, $R_6$ und m die oben für (I) in Anspruch 1 angegebenen Werte haben.

**13.** Verbindung nach Anspruch 1 der Formel:

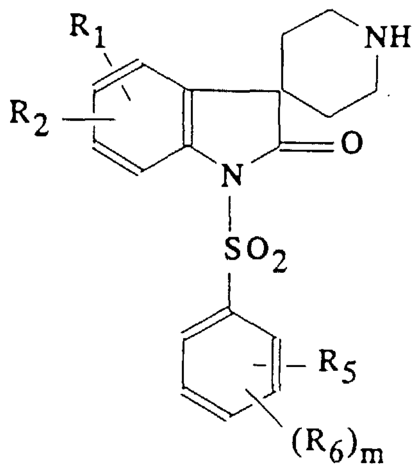

(XIII),

worin

$R_1$, $R_2$, $R_5$, $R_6$ und m die oben für (I) in Anspruch 1 angegebenen Werte haben.

**14.** Verfahren zur Herstellung einer Verbindung (I) nach Anspruch 1, dadurch gekennzeichnet, daß:
auf ein 2-Oxoindol, disubstituiert in Stellung 3, der Formel:

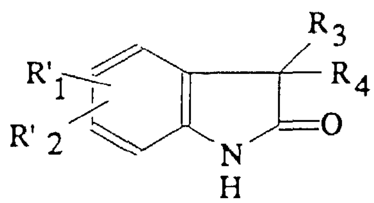

(II),

worin $R'_1$ und $R'_2$ jeweils entweder $R_1$, $R_2$ wie für (I) definiert, oder Vorläufer-Gruppen von $R_1$, $R_2$ sind, und $R_3$, $R_4$ wie für (I) oben definiert sind, ein Benzolsulfonylhalogenid der Formel:

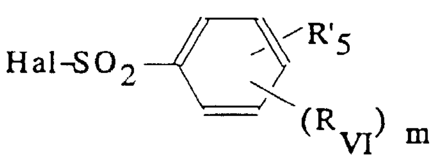

(III),

worin $R'_5$ und $R_{VI}$ jeweils entweder $R_5$ und $R_6$ wie oben für (I) definiert, oder Vorläufer-Gruppen von $R_5$ und $R_6$ sind; einwirken gelassen wird; und

- entweder, wenn $R'_1 = R_1$, $R'_2 = R_2$, $R'_5 = R_5$ und $R_{VI} = R_6$, die so erhaltene Verbindung der Formel (I) isoliert wird;
- oder, wenn irgendeine der Gruppen $R'_1$, $R'_2$, $R'_5$ und $R_{VI}$ jeweils eine Vorläufer-Gruppe von $R_1$, $R_2$, $R_5$ und/oder $R_6$ bedeutet, die erhaltene Verbindung einer abschließenden Behandlung zur Herstellung der Verbindung der Formel (I) durch Transformation irgendeiner der Gruppen $R'_1$, $R'_2$, $R'_5$ und $R_{VI}$ in jeweils $R_1$, $R_2$, $R_5$ und $R_6$ unterworfen wird.

**15.** Verbindung der Formel:

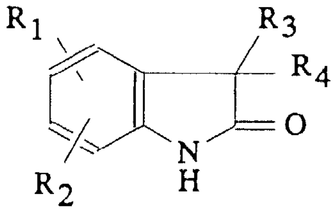

(II)',

worin:

- $R_1$ und $R_2$ jeweils unabhängig einen Wasserstoff, ein Hydroxyl, ein $C_1$-$C_4$-ω-Halogenalkoxy, ein Halogen, ein $C_1$-$C_4$-Alkyl, ein Trifluormethyl, ein $C_1$-$C_7$-Alkoxy, ein $C_1$-$C_4$-Polyhalogenalkoxy, ein $C_2$-$C_4$-ω-Hydroxyalkoxy, ein ω-Methoxyalkoxy, wobei das Alkyl $C_2$-$C_4$ ist, ein $C_2$-$C_4$-ω-Aminoalkoxy, frei oder substituiert durch ein oder zwei $C_1$-$C_4$-Alkyle; ein $C_3$-$C_7$-Cycloalkyloxy; ein Cycloalkylmethoxy, wobei das Cycloalkyl $C_3$-$C_7$ ist; ein Phenoxy; ein Benzyloxy; ein $C_1$-$C_4$-Alkylthio; ein Phenylthio; ein Nitro; ein Amino, frei oder substituiert durch ein oder zwei $C_1$-$C_4$-Alkyle; ein Cyano; ein $C_1$-$C_4$-Acyl; ein $C_1$-$C_4$-Acyloxy; ein $C_1$-$C_4$-Alkylsulfonamido; ein Phenylsulfonamido; ein $C_1$-$C_4$-Alkylamido; ein $C_1$-$C_4$-Alkoxycarbonylamino; ein Ureido, unsubstituiert oder substituiert durch ein Phenyl oder durch ein oder zwei $C_1$-$C_4$-Alkyle, bedeuten;
- $R_3$ und $R_4$ gemeinsam mit dem Kohlenstoff, an den sie gebunden sind,

  . ein Adamantan,
  . ein Indan oder Hexahydroindan, unsubstituiert oder substituiert durch eine oder mehrere $C_1$-$C_7$-Alkyl-Gruppen,
  . ein Tricyclo[5.2.1.0.2,6]decan oder ein Tricyclo[5.2.1.0.2,6]dec-8-en, unsubstituiert oder substituiert durch eine oder mehrere $C_1$-$C_7$-Alkyl-Gruppen,
  oder

  einen $C_4$-$C_8$-Kohlenwasserstoff-Cyclus, substituiert durch eine oder mehrere $C_1$-$C_7$-Alkyl-Gruppen oder durch ein $C_3$-$C_5$-Spirocycloalkyl, bilden,

  oder auch

  $R_3$ und $R_4$ gemeinsam eine Gruppe $-(CH_2)_pX(CH_2)_q-$ bilden, wobei p und q ganze Zahlen sind, deren Summe von 3 bis 6 variieren kann, und X Sauerstoff, Schwefel oder eine Gruppe $NR_{13}$ darstellt, wobei $R_{13}$ ein Phenyl, ein Benzyl, ein $C_1$-$C_4$-Acyl, ein $C_1$-$C_4$-Alkoxycarbonyl, ein Carbamoyl, unsubstituiert oder substituiert durch ein oder zwei $C_1$-$C_4$-Alkyle, ist;

  mit der Maßgabe, daß, wenn $CR_3R_4$ Adamantan bedeutet, $R_1$ und $R_2$ von Wasserstoff verschieden sind.

**16.** Verbindung der Formel:

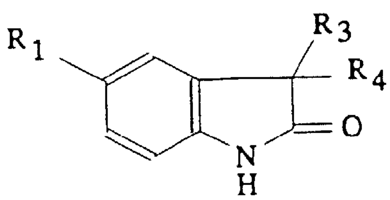

(II)",

worin:

- $R_1$ ein Hydroxyl, ein $C_1$-$C_4$-ω-Halogenalkoxy, ein Halogen, ein $C_1$-$C_4$-Alkyl, ein Trifluormethyl, ein $C_1$-$C_7$-Alkoxy, ein $C_1$-$C_4$-Polyhalogenalkoxy, ein $C_2$-$C_4$-ω-Hydroxyalkoxy, ein ω-Methoxyalkoxy, wobei das Alkyl $C_2$-$C_4$ ist, ein $C_2$-$C_4$-ω-Aminoalkoxy, frei oder substituiert durch ein oder zwei $C_1$-$C_4$-Alkyle; ein $C_3$-$C_7$-Cycloalkyloxy; ein Cycloalkylmethoxy, wobei das Cycloalkyl $C_3$-$C_7$ ist; ein Phenoxy; ein Benzyloxy; ein $C_1$-$C_4$-Alkylthio; ein Phenylthio; ein Nitro; ein Amino, frei oder substituiert durch ein oder zwei $C_1$-$C_4$-Alkyle; ein Cyano; ein $C_1$-$C_4$-Acyl; ein $C_1$-$C_4$-Acyloxy; ein $C_1$-C4-Alkylsulfonamido; ein Phenylsulfonamido; ein $C_1$-$C_4$-Alkylamido; ein $C_1$-$C_4$-Alkoxycarbonylamino; ein Ureido, unsubstituiert oder substituiert durch ein Phenyl oder durch ein oder

zwei $C_1$-$C_4$-Alkyle, bedeutet;

- $R_3$ und $R_4$ gemeinsam eine Gruppe $-(CH_2)_pX(CH_2)_q-$ bilden;
  oder
- $R_3$ und $R_4$ gemeinsam mit dem Kohlenstoff, an den sie gebunden sind, einen gesättigten oder ungesättigten, gegebenenfalls kondensierten $C_3$-$C_{10}$-Kohlenwasserstoff-Cyclus, unsubstituiert oder substituiert durch eine oder mehrere $C_1$-$C_7$-Alkyl-Gruppen oder durch ein $C_3$-$C_5$-Spirocycloalkyl, bilden;
- p und q jeweils eine ganze Zahl sind, wobei ihre Summe von 3 bis 6 variieren kann;
- X Sauerstoff, Schwefel oder eine Gruppe $NR_{13}$ darstellt;
- $R_{13}$ Wasserstoff, ein $C_1$-$C_4$-Alkyl, ein Phenyl, ein Benzyl, ein $C_1$-$C_4$-Acyl, ein $C_1$-$C_4$-Alkoxycarbonyl, ein Carbamoyl, unsubstituiert oder substituiert durch ein oder zwei $C_1$-$C_4$-Alkyle, ist;

mit der Maßgabe, daß,

- wenn $R_1$ Methoxy bedeutet, $CR_3R_4$ von einem 3-Pyrrolidin, unsubstituiert oder N-substituiert durch ein $C_1$-$C_4$-Alkyl, verschieden ist,
- wenn $R_1$ ein Halogen oder ein Nitro bedeutet, $R_3$ und $R_4$ gemeinsam mit dem Kohlenstoffatom, an das sie gebunden sind, von einem gesättigten oder ungesättigten $C_3$-$C_{10}$-Kohlenwasserstoff-Cyclus verschieden sind, und
- wenn $R_1$ eine Propionyl-Gruppe bedeutet, $R_3$ und $R_4$ gemeinsam mit dem Kohlenstoffatom, an das sie gebunden sind, kein Cyclopropan bilden.

**17.** Verbindung nach Anspruch 16, worin $R_1$ Ethoxy darstellt.

**18.** Verbindung der Formel

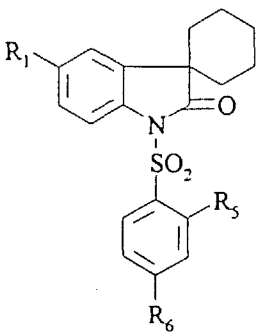

,

worin

- $R_1$ ein Chloratom bedeutet;
- $R_5$ eine Methoxy-Gruppe darstellt; und
- $R_6$ eine Gruppe

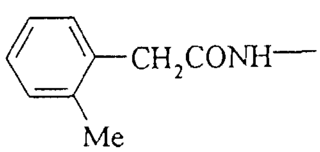

ist.

**19.** Verbindung der Formel

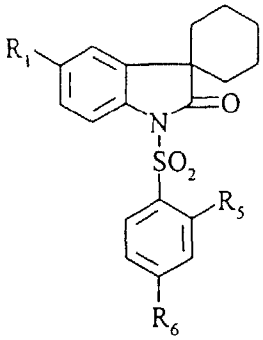

,

worin

- $R_1$ eine Methoxy-Gruppe bedeutet;
- $R_5$ eine Methoxy-Gruppe darstellt; und
- $R_6$ eine Gruppe $MeOCH_2CONH$- ist.

**20.** Verbindung der Formel

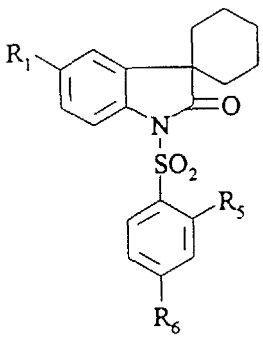

,

worin

- $R_1$ eine Ethoxy-Gruppe bedeutet;
- $R_5$ eine Methoxy-Gruppe darstellt; und
- $R_6$ eine Gruppe

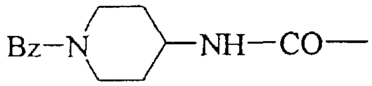

ist.

**21.** Pharmazeutische Zusammensetzung, welche als aktiven Bestandteil eine Verbindung nach einem der Ansprüche 1 bis 8 und 18 bis 20 enthält.

**22.** Pharmazeutische Zusammensetzung, welche eine Verbindung nach einem der Ansprüche 1 bis 8 und 18 bis 20 in Assoziation mit einem anderen aktiven Bestandteil enthält.

**23.** Pharmazeutische Zusammensetzung nach Anspruch 22, welche eine Antagonisten-Verbindung von V1-Rezeptoren in Assoziation mit einem Angiotensin II-Antagonisten enthält.

**Claims**

1. A compound of formula

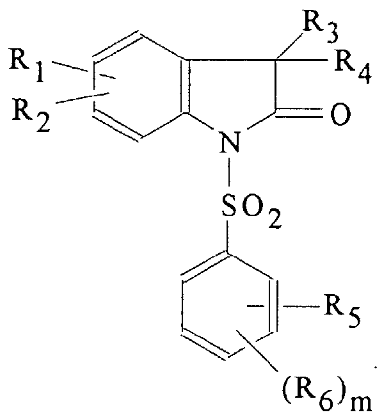

(I)

in which

- $R_1$ and $R_2$ are each independently a hydrogen, a hydroxyl, a $C_1$-$C_4$ ω-halogenoalkoxy, a halogen, a $C_1$-$C_4$ alkyl, a trifluoromethyl, a $C_1$-$C_7$ alkoxy, a $C_1$-$C_4$ polyhalogenoalkoxy, a $C_2$-$C_4$ ω-hydroxyalkoxy, a ω-methoxyalkoxy in which the alkyl is a $C_2$-$C_4$ alkyl, a $C_2$-$C_4$ ω-aminoalkoxy which is free or substituted by one or two $C_1$-$C_4$ alkyls; a $C_3$-$C_7$ cycloalkyloxy; a cycloalkylmethoxy in which the cycloalkyl is a $C_3$-$C_7$ cycloalkyl; a phenoxy; a benzyloxy; a $C_1$-$C_4$ alkylthio; a phenylthio; a nitro; an amino which is free or substituted by one or two $C_1$-$C_4$ alkyls; a cyano; a $C_1$-$C_4$ acyl; a $C_1$-$C_4$ acyloxy; a $C_1$-$C_4$ alkylsulfonamido; a phenylsulfonamido; a $C_1$-$C_4$ alkylamido; a $C_1$-$C_4$ alkoxycarbonylamino; an ureido which is unsubstituted or substituted by a phenyl or by one or two $C_1$-$C_4$ alkyls;
- $R_3$ and $R_4$ are each independently a $C_1$-$C_6$ alkyl, a $C_3$-$C_7$ cycloalkyl, a phenyl, a benzyl, a cycloalkylmethyl in which the cycloalkyl is a $C_3$-$C_7$ cycloalkyl;
  or
- $R_3$ and $R_4$ together form a $-(CH_2)_pX(CH_2)_q-$ group;
  or
- $R_3$ and $R_4$ together with the carbon atom to which they are bonded form a saturated or unsaturated $C_3$-$C_{10}$ hydrocarbon ring, which is optionally condensed, unsubstituted or substituted by one or several $C_1$-$C_7$ alkyl groups or by a $C_3$-$C_5$ spirocycloalkyl;
  or else
- $R_1$ and $R_4$ have each one of the above meanings and $R_2$ is located in the 4-position of the indole ring and form with $R_3$ a $(CH_2)_3$ group;
- $R_5$ and $R_6$ are each independently hydrogen, a halogen, a $C_1$-$C_7$ alkyl, a trifluoromethyl, a cyano, a nitro, an amino which is free or substituted by one or two $C_1$-$C_7$ alkyls; a hydroxylamino; a hydroxyl; a carboxyl; an $OR_7$ group; a $SR_7$ group; a $C_1$-$C_7$ acyl; a $C_1$-$C_7$ alkoxycarbonyl; a phenoxycarbonyl; a benzyloxycarbonyl; a carbamoyl which is substituted by $R'_6$ and $R''_6$ groups; a thiocarbamoyl which is free or substituted by one or two $C_1$-$C_7$ alkyls; a sulfamoyl; an alkylsulfamoyl or a dialkylsulfamoyl in which the alkyl is a $C_1$-$C_7$ alkyl; a $SO_2R'_7$ group; an alkylsulfonamido in which the alkyl is a $C_1$-$C_7$ alkyl; a $COR'_7$ group; a $NR_8R_9$ group; a $CO$-$NH$-$CH(R_{10})$-$COR_{12}$ group; optionally, the phenyl group which is constitutive of the $R_5$ and/or $R_6$ substitutent may be unsubstituted or substituted one or several times by a $C_1$-$C_7$ alkyl, a trifluoromethyl, a methoxy, a halogen, a sulfamoyl, an alkylsulfamoyl in which the alkyl is a $C_1$-$C_7$ alkyl, a carboxyl, an alkoxycarbonyl in which the alkyl is a $C_1$-$C_7$ alkyl, a $C_1$-$C_7$ acyloxy, an imidazolyl;
- $R'_6$ and $R''_6$ are each independently hydrogen, a $C_1$-$C_7$ alkyl which is unsubstituted or substituted by $R'''_6$, a phenyl, a pyridyl, a methylpyridyl, a piperidin-4-yl, a methylpiperidin-4-yl;
- $R'''_6$ is a hydroxyl, a cyano, a carboxyl which is free or esterified by a $C_1$-$C_7$ alkyl or by a benzyl; a phenyl; a pyridyl; a methylpyridyl; an amino which is free or substituted by one or two $C_1$-$C_7$ alkyls;
- $R_7$ is a $C_1$-$C_7$ alkyl, a phenyl, a benzyl, a $C_3$-$C_7$ cycloalkyl, a $C_2$-$C_4$ alkenyl, a $C_1$-$C_7$ ω-halogenoalkyl, a $C_1$-$C_7$ polyhalogenoalkyl, a $C_1$-$C_7$ acyl, a $C_1$-$C_7$ ω-carboxyalkyl which is free or esterified by a $C_1$-$C_4$ alkyl or by a benzyl, a $C_2$-$C_7$ ω-aminoalkyl in which the amino group is free or substituted by one or two $C_1$-$C_4$ alkyls or

in the form of an ammonium ion;

- $R'_7$ is a piperazin-1-yl group which is unsubstituted or substituted in position 4 by a $R''_7$ group, a piperidino group which is unsubstituted or substituted in position 4 by a $R'''_7$ group, an azetidin-1-yl group which is unsubstituted or substituted in position 3 by a $R'''_7$ group, a pyridyl group which is unsubstituted or substituted by a methyl;
- $R''_7$ is a $C_1$-$C_4$ alkyl, a phenyl, a benzyl, a $C_1$-$C_4$ acyl;
- $R'''_7$ is $R''_7$ or an amino which is free or bears a protecting group;
- $R_8$ and $R_9$ are each indepedently hydrogen, a $C_1$-$C_7$ alkyl, a phenyl, a benzyl, $R_9$ may also be a $C_1$-$C_7$ acyl, a $C_1$-$C_7$ thioalkyl, a cycloalkylcarbonyl in which the cycloalkyl is a $C_3$-$C_7$ cycloalkyl, a cycloalkylthiocarbonyl in which the cycloalkyl is a $C_3$-$C_7$ cycloalkyl, a $C_1$-$C_4$ ω-aminoacyl, a $C_1$-$C_4$ ω-hydroxyacyl, a $C_1$-$C_4$ ω-benzyloxyacyl, a phenoxycarbonyl, a thienocarbonyl, a pyridylcarbonyl, a methylpyridylcarbonyl, a $C_1$-$C_4$ alkoxycarbonyl, a benzoyl, a $CO$-$CH(R_{10})$-$NR_{11}R'_{11}$ group, a $CH(R_{10})CO_2R_{11}$ group, a $(CH_2)_tCOR_{12}$ group, a $CO(CH_2)_tCOR_{12}$ group, a carbamoyl which is unsubstituted or substituted by a phenyl or by one or two $C_1$-$C_4$ alkyls;
- m is 1 or, when $R_6$ is a halogen, a $C_1$-$C_7$ alkyl or a $C_1$-$C_7$ alkoxy, m can also be 2, 3 or 4 or else $(R_6)_m$ can represent m substituents having different meanings selected from halogen, a $C_1$-$C_7$ alkyl or a $C_1$-$C_7$ alkoxy;
- p and q are each integers, their sum varying from 3 to 6;
- t is an integer varying from 1 to 5;
- X is oxygen, sulfur or a $NR_{13}$ group;
- $R_{10}$ is hydrogen, a $C_1$-$C_4$ alkyl or a benzyl;
- $R_{11}$ and $R'_{11}$ are each independently hydrogen or a $C_1$-$C_4$ alkyl;
- $R_{12}$ is a hydroxyl, a $C_1$-$C_4$ alkoxy or an amino which is unsubstituted or substituted by one or two $C_1$-$C_4$ alkyls;
- $R_{13}$ is hydrogen, a $C_1$-$C_4$ alkyl, a phenyl, a benzyl, a $C_1$-$C_4$ acyl, a $C_1$-$C_4$ alkoxycarbonyl or a carbamoyl which is unsubstituted or substituted by one or two $C_1$-$C_4$ alkyls;

provided that when $R_3$ is a methyl, $R_4$ is a methyl or an ethyl, $R_5$ is hydrogen, m is 1 and $R_6$ is a methyl in the 4-position, then $R_1$ and $R_2$ are not simultaneously hydrogen ;
as well as its possible salts.

**2.** A compound of formula (I) according to claim 1 in which $R_1$ is chlorine or an ethoxy group in the position 5 of the indole ring and $R_2$ is hydrogen.

**3.** A compound of formula (I) according to claim 1 in which $R_3$ and $R_4$ together with the carbon atom to which they are bonded constitute a $C_3$-$C_{10}$ hydrocarbon cycle.

**4.** A compound of formula (I) according to claim 1 in which $R_3$ and $R_4$ together with the carbon atom to which they are bonded constitute a cyclohexane which is unsubstituted or substituted by one or two $C_1$-$C_7$ alkyl groups or by a $C_3$-$C_5$ spirocycloalkyl, a cycloheptane, an adamantane or a tricyclo[5.2.1.0.2,6]dec-8-ene.

**5.** A compound of formula (I) according to claim 1 in which $R_3$ and $R_4$ together with the carbon atom to which they are bonded constitute a piperidine-4 or a N-methylpiperidine-4.

**6.** A compound of formula (I) according to claim 1 in which $R_5$ and $R_6$ each represent a methoxy.

**7.** A compound of formula (I) according to claim 1 in which $R_5$ in position 2 is methoxy and $R_6$ in position 4 is a $C_1$-$C_7$ acylamino, a $C_1$-$C_4$ dialkylureido, an alkoxycarbonylalkylcarbamoyl in which the alkyl groups are $C_1$-$C_7$ alkyls.

**8.** A compound of formula (I) according to claim 1 in which $R_1$ is in position 5 and $R_2$ is hydrogen.

**9.** A compound according to claim 1 of formula:

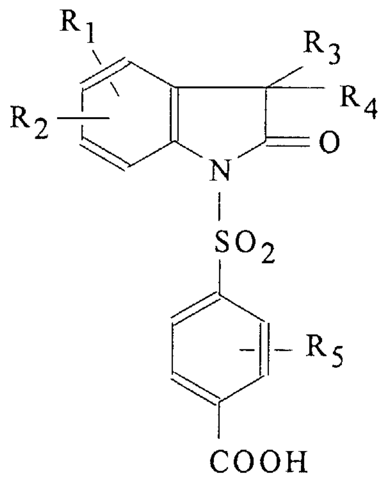

(IX)

in which $R_1$, $R_2$, $R_3$, $R_4$ and $R_5$ have the meanings indicated above for (I) in claim 1, and its functional derivatives.

**10.** A compound according to claim 1 of formula:

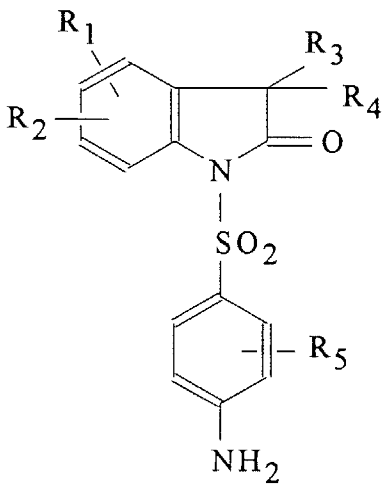

(X)

in which $R_1$, $R_2$, $R_3$, $R_4$ and $R_5$ have the meanings indicated above for (I) in claim 1, and its possible salts.

**11.** A compound according to claim 1 of formula:

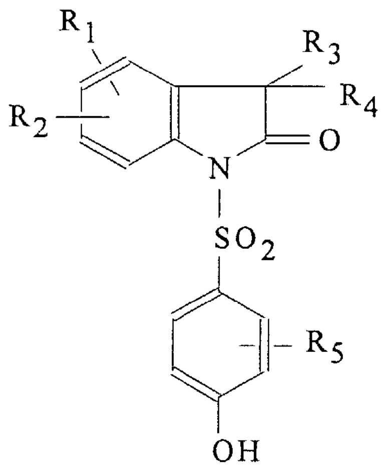

(XI)

in which $R_1$, $R_2$, $R_3$, $R_4$, and $R_5$ have the meanings indicated above for (I) in claim 1.

**12.** A compound according to claim 1 of formula:

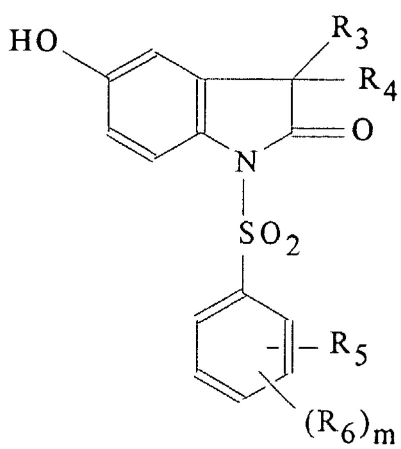

(XII)

in which $R_3$, $R_4$, $R_5$, $R_6$ and m have the meanings indicated above for (I) in claim 1.

**13.** A compound according to claim 1 of formula:

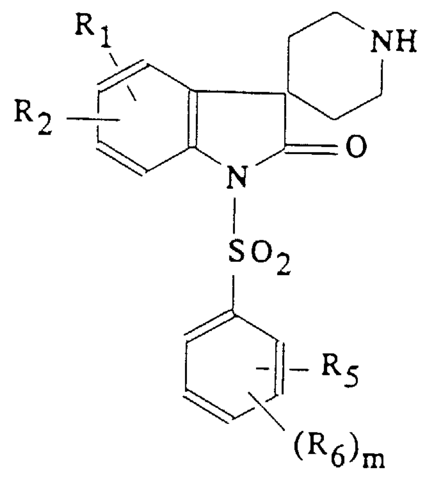

(XIII)

in which $R_1$, $R_2$, $R_5$, $R_6$ and m have the meanings indicated above for (I) in claim 1.

**14.** A process for the preparation of a compound (I) according to claim 1, characterized in that : a 2-oxoindole which is disubstituted in the 3-position, of formula :

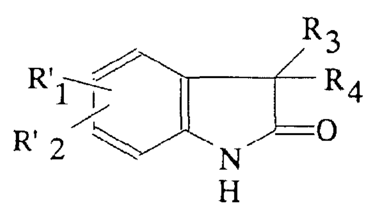

(II)

in which $R'_1$ and $R'_2$ are respectively either $R_1$ and $R_2$ as defined for (I), or precursor groups of $R_1$ and $R_2$, and $R_3$ and $R_4$ as defined above for (I), is reacted with a benzenesulfonyl halide of formula :

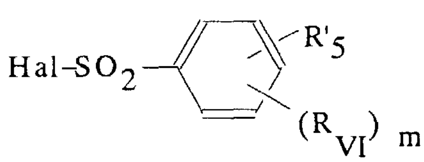

(III)

in which $R'_5$ and $R_{VI}$ are respectively either $R_5$ and $R_6$ as defined above for (I), or precursor groups of $R_5$ and $R_6$ ; and,

- either, when $R'_1=R_1$; $R'_2=R_2$, $R'_5=R_5$ and $R_{VI}=R_6$, the compound of formula (I) so obtained is isolated,
- or, when any one of the $R'_1$, $R'_2$, $R'_5$ and $R_{VI}$ groups is respectively a precursor group of $R_1$, $R_2$, $R_5$ and/or $R_6$, the compound obtained is subjected to a further treatment to prepare a compound of formula (I) by converting any one of the $R'_1$, $R'_2$, $R'_5$ and $R_{VI}$ groups into, respectively, $R_1$, $R_2$, $R_5$ and $R_6$.

**15.** A compound of formula:

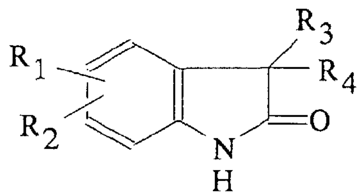

(II)'

in which

- $R_1$ and $R_2$ are each independently a hydrogen, a hydroxyl,a $C_1$-$C_4$ ω-halogenoalkoxy, a halogen, a $C_1$-$C_4$ alkyl, a trifluoromethyl, a $C_1$-$C_7$ alkoxy, a $C_1$-$C_4$ polyhalogenoalkoxy, a $C_2$-$C_4$ ω-hydroxyalkoxy, a ω-methoxyalkoxy in which the alkyl is a $C_2$-$C_4$ alkyl, a $C_2$-$C_4$ ω-aminoalkoxy which is free or substituted by one or 2 $C_1$-$C_4$ alkyls; a $C_3$-$C_7$ cycloalkoxy; a cycloalkylmethoxy in which the cycloalkyl is a $C_3$-$C_7$ cycloalkyl; a phenoxy; a benzyloxy; a $C_1$-$C_4$ alkylthio; a phenylthio; a nitro; an amino which is free or substituted by one or two $C_1$-$C_4$ alkyls; a cyano; a $C_1$-$C_4$ acyl; a $C_1$-$C_4$ acyloxy; a $C_1$-$C_4$ alkylsulfonamido; a phenylsulfonamido; a $C_1$-$C_4$ alkylamido; a $C_1$-$C_4$ alkoxycarbonylamino; an ureido which is unsubstituted or substituted by a phenyl or by one or two $C_1$-$C_4$ alkyls ;
- $R_3$ and $R_4$ together with the carbon to which they are bonded constitute
  - . an adamantane,
  - . an indane or a hexahydroindane unsubstituted or substituted by one or several $C_1$-$C_7$ alkyl groups,
  - . a tricyclo[5.2.1.0.2,6]decane or a tricyclo[5.2.1.0.2,6]dec-8-ene unsubstituted or substituted by one or several $C_1$-$C_7$ alkyl groups,

    or
- a $C_4$-$C_8$ hydrocarbon cycle unsubstituted or substituted by one or several $C_1$-$C_7$ alkyl groups or by a $C_3$-$C_5$ spirocycloalkyl,

  or else
- $R_3$ and $R_4$ together constitute a $-(CH_2)_p-X(CH_2)q$-group in which p and q each represent an integer, and their sum can vary from 3 to 6 and X is oxygen, sulfur or a $NR_{13}$ group, $R_{13}$ being a phenyl, a benzyl, a $C_1$-$C_4$ acyl, a $C_1$-$C_4$ alkoxycarbonyl, a carbamoyl which is unsubstituted or substituted by one or 2 $C_1$-$C_4$ alkyls;

provided that when $CR_3R_4$ is an adamantane, $R_1$ and $R_2$ are different from hydrogen.

**16.** A compound of formula:

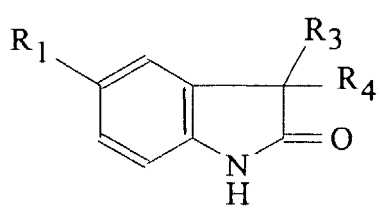

(II)"

in which

- $R_1$ is a hydroxyl, a $C_1$-$C_4$ ω-halogenoalkoxy, a halogen, a $C_1$-$C_4$ alkyl, a trifluoromethyl, a $C_1$-$C_7$ alkoxy, a $C_1$-$C_4$ polyhalogenoalkoxy, a $C_2$-$C_4$ ω-hydroxyalkoxy, a ω-methoxyalkoxy in which the alkyl is a $C_2$-$C_4$ alkyl, a $C_2$-$C_4$ ω-aminoalkoxy which is free or substituted by one or two $C_1$-$C_4$ alkyls ; a $C_3$-$C_7$ cycloalkyloxy ; a cycloalkylmethoxy in which the cycloalkyl is a $C_3$-$C_7$ cycloalkyl ; a phenoxy ; a benzyloxy ; a $C_1$-$C_4$ alkylthio ; a phenylthio ; a nitro ; an amino which is free or substituted by one or two $C_1$-$C_4$ alkyls ; a cyano ; a $C_1$-$C_4$ acyl ; a $C_1$-$C_4$ acyloxy ; a $C_1$-$C_4$ alkylsulfonamido ; a phenylsulfonamido ; a $C_1$-$C_4$ alkylamido ; a $C_1$-$C_4$ alkoxycarbonylamino ; an ureido which is unsubstituted or substituted by a phenyl or by one or two $C_1$-$C_4$ alkyls ;

- $R_3$ and $R_4$ together form a $-(CH_2)_pX(CH_2)q-$ group ; or
- $R_3$ and $R_4$ together with the carbon to which they are bonded constitute a saturated or unsaturated $C_3$-$C_{10}$ hydrocarbon cycle, optionally condensed, which is unsubstituted or substituted by one or several $C_1$-$C_7$ alkyl groups or by a $C_3$-$C_5$ spirocycloalkyl ;
- p and q each represent an integer, and their sum can vary from 3 to 6 ;
- X is oxygen, sulfur or a $NR_{13}$ group ;
- $R_{13}$ is hydrogen, a $C_1$-$C_4$ alkyl, a phenyl, a benzyl, a $C_1$-$C_4$ acyl, a $C_1$-$C_4$ alkoxycarbonyl, a carbamoyl which is unsubstituted or substituted by one or 2 $C_1$-$C_4$ alkyls ;

provided that :

- when $R_1$ is methoxy, $CR_3R_4$ is different from 3-pyrrolidine which is unsubstituted or N-substituted by a $C_1$-$C_4$ alkyl,
- when $R_1$ is a halogen or a nitro, $R_3$ and $R_4$ together with the carbon atom to which they are bonded are different from a $C_3$-$C_{10}$ hydrocarbon cycle which is saturated or unsaturated, and
- when $R_1$ is a propionyl group, $R_3$ and $R_4$ together with the carbon atom to which they are bonded do not form a cyclopropane.

**17.** A compound according to claim 16 in which $R_1$ is ethoxy.

**18.** A compound of formula

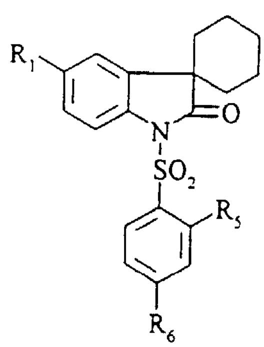

in which

- $R_1$ is a chlorine atom
- $R_5$ is a methoxy group, and
- $R_6$ is a

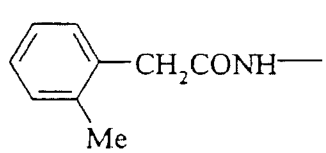

group.

**19.** A compound of formula

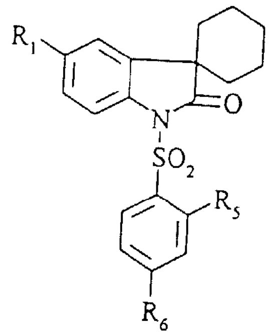

in which

- $R_1$ is a methoxy group,
- $R_5$ is a methoxy group, and
- $R_6$ is a $MeOCH_2CONH$- group.

**20.** A compound of formula

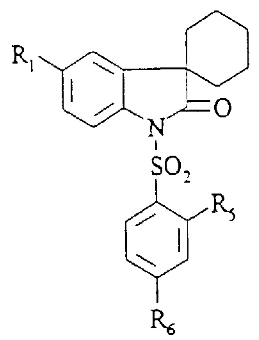

in which

- $R_1$ is an ethoxy group,
- $R_5$ is a methoxy group, and
- $R_6$ is a

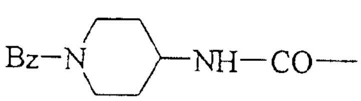

group.

**21.** A pharmaceutical composition containing as active principle a compound according to any one of claims 1 to 8 and 18 to 20.

**22.** A pharmaceutical composition containing a compound according to any one of claims 1 to 8 and 18 to 20 in association with another active principle.

**23.** A pharmaceutical composition according to claim 22 containing an antagonist compound of the V1 receptors in association with an angiotensin II antagonist.